Europäisches Patentamt

European Patent Office

Office européen des brevets

(11) Publication number: **0 434 909 A2**

## EUROPEAN PATENT APPLICATION

(21) Application number: **90117879.8**

(22) Date of filing: **17.09.90**

(51) Int. Cl.5: **C07K 15/08**, C12N 9/64,
C12N 15/57, C12P 21/08,
A61K 39/00

(30) Priority: **18.09.89 US 408339**
**01.03.90 US 487181**

(43) Date of publication of application:
**03.07.91 Bulletin 91/27**

(84) Designated Contracting States:
**AT BE CH DE DK ES FR GB GR IT LI LU NL SE**

(71) Applicant: **SYNERGEN, INC.**
**1885, 33rd Street**
**Boulder Colorado 80301(US)**

(72) Inventor: **Cox, George N.**

678 West Willow Street
**Louisville, Colorado 80027(US)**
Inventor: **Milhausen, Michael**
**2292 Holyoke Drive**
**Boulder, Colorado 80303(US)**
Inventor: **Hageman, Robert**
**3784 Moffitt Court**
**Boulder, Colorado 803304(US)**

(74) Representative: **Patentanwälte Grünecker,**
**Kinkeldey, Stockmair & Partner**
**Maximilianstrasse 58**
**W-8000 München 22(DE)**

(54) **Anticoagulant and antihelminthic proteins and methods for the production and use of same.**

(57) The present invention provides proteins from nematodes or derivatives of such proteins. According to certain embodiments, those proteins can be used in vaccinations against nematodes. The present invention also provides improved methods of purifying those proteins from a natural source.

FIG. 1

## ANTICOAGULANT AND ANTIHELMINTHIC PROTEINS AND METHODS FOR THE PRODUCTION AND USE OF SAME

BACKGROUND OF THE INVENTION

This is a continuation-in-part application of U.S. Patent Application Serial No. 07/408,339, filed September 18, 1989.

Field of the Invention

The present invention relates to proteins and their use as vaccines against helminthes in mammals, particularly sheep. The invention also relates to methods for producing these proteins in substantially pure form.

Haemonchus contortus is a blood feeding parasite commonly found in sheep, cattle, goats and wild ruminants of many species. These parasites feed on the blood of the host, and inject hemolytic proteins into the host's system, thereby resulting in anemia, emaciation, edema and intestinal disturbances. In cases of heavy infection, the host usually dies.

Infection occurs when the third-stage juvenile, still wearing the remains of a second-stage cuticle, is eaten. Exsheathment takes place in the forestomach, at which point the parasite is referred to as an XL3 larvae (XL3s). Upon entering the abomasum, the worm molts within 48 hours, becoming an L4 larvae (L4s), or forth stage juvenile, which feeds on the blood of its host. In about three days, the worm molts for a final time, and egg production begins about 15 days later.

There have been many attempts to develop a vaccine against these parasites. However, the immune mechanisms responsible for protection have not been elucidated, nor have specific protein antigens been identified. Studies have suggested that development of the worm through the XL3 and L4 stages within the host is sufficient to confer protection against reinfection by larvae. (Adams, D.B. Int. J. Parasitology 12: 439-443, 1982) Ozerol et al., J. Parasitology 56: 1199-1205 (1970) and Neilson, Exp. Parasitology 25: 131-141 (1969). In agreement with this, it has been shown that the number of XL3 and L4 larvae recovered from the abomasa of experimentally reinfected immune animals is greatly reduced compared to naive control sheep, suggesting that the host's immune response is directed against these two developmental states. (Bitakaramire, P.K., Parasitology 56: 619-622, 1966). Most research to date has focused on identifying protective antigens in fluids obtained by cultivating H. contortus in vitro to the XL3 and L4 stages. (Ozerol et al., J. Parasitology 55:79-87 (1969); vaccination studies in sheep using such concentrated fluids demonstrated partial protection in lambs 6 months or older, but demonstrated marginal protection in lambs less than 6 months of age. (Ozerol et al., Parasitology 56, supra; Neilson, Int. J. Parasitology 5: 427-430 (1975) and Bioisvenue et al. Am. J . Vet. Res. 48: 1236-1238 (1987)).

It is also known that certain proteins present on the cuticular surface of parasitic nematodes may induce the formation of antibodies by the host. (Mackenzie et al., Eur. J. of Immunology 10: 594-601 (1980) and Maizels et al. Immunology 38: 107-121 (1983). These proteins are also known to undergo profound antigenic changes between developmental stages, and in some cases, during a single developmental stage. (Phillip et al., Nature 287: 538-540 (1980) and Maizels et al, Immunology 38: 107-121 (1983)). In vitro studies have shown that antibodies to surface antigens of certain parasitic nematodes promote adherence and killing by granulocycles and macrophages. Kazura et al., Nature 274: 588-589 (1978); Mackenzie et al., Eur. J. Immunology: 594-601 (1980) and Subahmanyam et al., Nature 260: 529-530 (1984). The ability of antibodies and effector cells (eosinophils and macrophages) to kill worms In vitro, coupled with the finding that parasitic nematodes change surface antigens as they develop within the host, has led to the notion that surface proteins may prove effective as subunit vaccines. However, testing this hypothesis has been hampered by the inability to purify adequate quantities of surface proteins free of cellular proteins.

It has been shown that a monoclonal antibody to a surface component of Trichinella spiralis newborn larvae confers partial protection in vitro in passive transfer experiments. (Ortega-Pierres et al., Parasite Immunol. 6: 275-284 (1984)). It is also known that membrane associated proteins present in the microvilli in the intestines of Haemonchus may be used as a vaccine to partially protect sheep from Haemonchus. (Parasitology 94: 385-397, 1987).

Although control of helminthes including Haemonchus has been achieved to some extent by the administration of antihelmintic drugs, this method is not satisfactory as it requires repeated dosing in order to achieve any degree of control.

Surprisingly, despite the inadequacies of prior methods, the present inventors have found a method to purify adequate quantities of cuticular surface proteins, free of cellular proteins, identified a collagen peptide and a method of producing an anticoagulant protein extract, all of which are useful as vaccines against infection by helminthes in sheep.

## Summary of the Invention

It is an object of the present invention to provide proteins useful as antihelmintic sheep vaccines and a method for production of the same which overcomes the disadvantages of previously known proteins and processes.

Additional objects and advantages of the invention will be set forth in the description which follows, and in part will be apparent from the description, or may be learned by practice of the invention. The objects and advantages of the invention may be realized and obtained by means of the instrumentalities and combinations particularly pointed out in the appended claims.

To achieve the foregoing objects, and in accordance with the purposes of the invention as embodied and broadly described herein, there is provided various proteins useful in the prevention of infection by helminthes in sheep.

In addition, a recombinant-DNA method for the manufacture of anticoagulant and anthelmintic proteins, and a method for the purification of cuticular proteins is provided.

It is understood that the foregoing description and the following detailed description are exemplary and explanatory only, and are not restrictive of the invention as claimed.

## BRIEF DESCRIPTION OF THE DRAWINGS

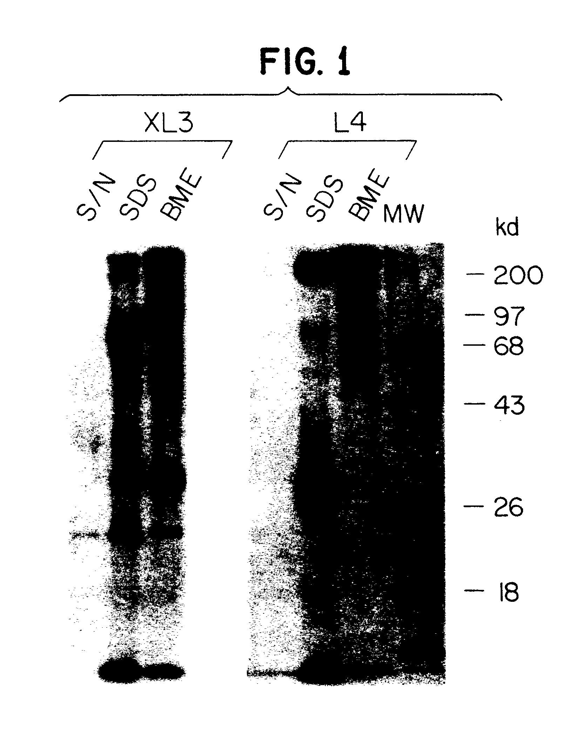

Figure 1. Patterns of proteins obtained by $^{125}$I-labeling of live XL3s and L4s. Live worms were labeled with $^{125}$I and chloramine T. After labeling, the worms were sonicated and particulate worm fragments collected by centrifugation. Labeled proteins remaining in the supernatant are shown in lanes labeled S/N. Labeled proteins solubilized from the worm fragment pellet by sequential extractions with SDS and SDS + BME are shown in lanes labeled SDS and BME, respectively. Aliquots of each fraction (10,000 cpm from the SDS and BME lanes) were diluted into SDS sample buffer and electrophoresed on a 12% SDS-polyacrylamide gel. The gel was dried and exposed to X-ray film at -70. Molecular weights (in kilodaltons) of protein standards are shown on the right. The major 27 and 29 kDa SDS-soluble proteins of L4s appear as a single fat band in this autoradiogram.

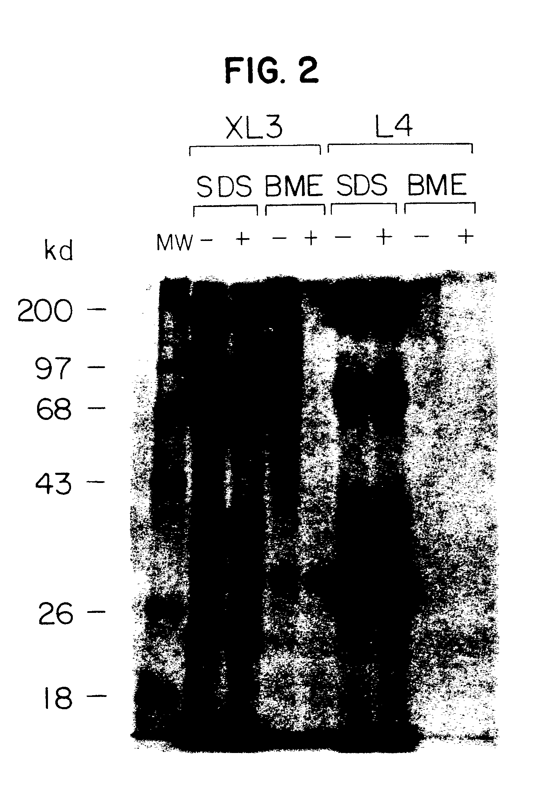

Figure 2. Autoradiogram of XL3 and L4 surface proteins treated with bacterial collagenase. $^{125}$I-labeled proteins solubilized from worms with SDS (SDS lanes) or SDS + BME (BME lanes) were mixed with unlabeled adult cuticle proteins and incubated overnight with bacterial collagenase (+ lanes) or buffer (- lanes). Reactions were terminated by dilution into SDS sample buffer, followed by electrophoresis of the samples in a 12% SDS-polyacrylamide gel.

The gel was stained with Coomassie blue to monitor digestion of adult cuticle collagens in the (+) lanes and to confirm the absence of nonspecific proteolysis (stained gel not shown). The gel was dried and exposed to X-ray film at -70. Molecular weights of protein standards are given to the left.

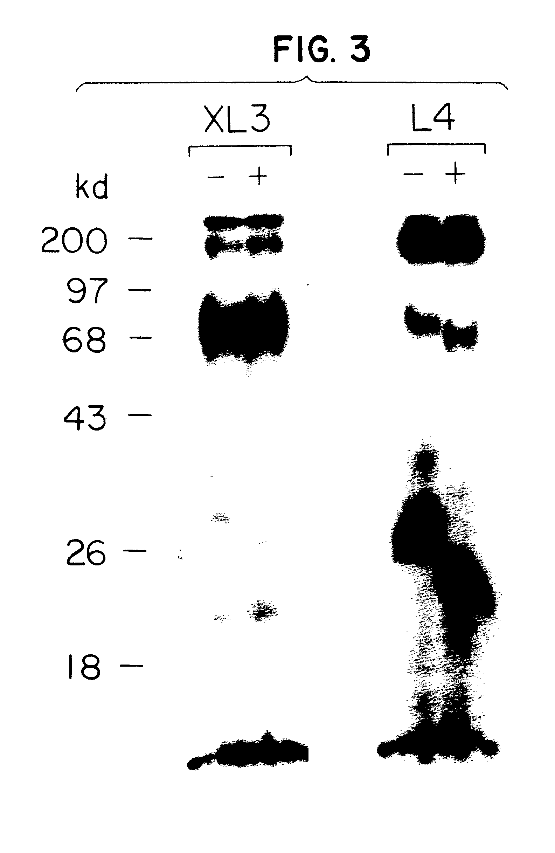

Figure 3. Autoradiogram of XL3 and L4 surface proteins after treatment with Endoglycosidase F. $^{125}$I-labeled, SDS-soluble surface proteins of XL3s and L4s were incubated overnight with Endoglycosidase F (+ lanes) or with buffer (- lanes). The reaction mixes were diluted into SDS sample buffer and electrophoresed on a 12% SDS-polyacrylamide gel. The positions and molecular weight (in kilodaltons) of protein standards are shown on the left.

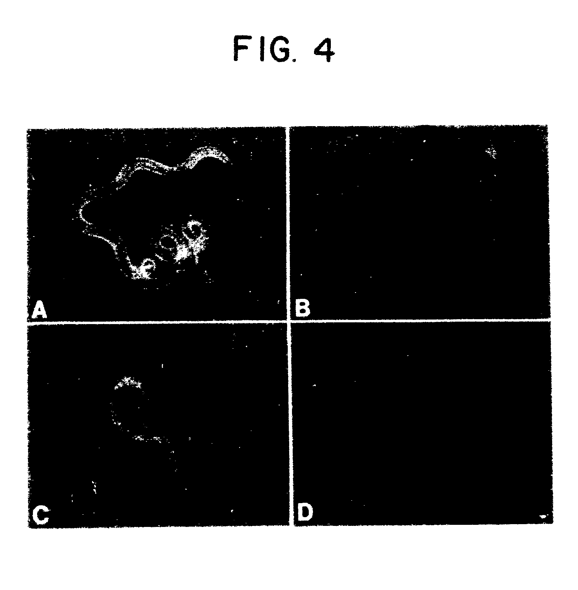

Figure 4. Fluorescence photomicrographs of live XL3s and L4s with various anti-cuticle sera. Reactions of live XL3s and L4s with a rabbit anti-native XL3/L4 cuticle serum (Rb8061) is shown in (A) and (C), respectively. Reactions of live XL3s with a rabbit anti-SDS-treated XL3 cuticle serum (Rb-6791) is shown in (B). Reactions of live L4s with a rabbit anti-SDS-treated L4 cuticle serum (Rb-7539) is shown in (D). After incubation with primary antisera the worms were washed and incubated with an FITC-conjugated goat anti-rabbit IgG serum. After further washing, the worms were observed in a light microscope equipped with fluorescence optics. Reactions of pre-bleed sera with XL3s and L4s were comparable to the reactions shown in (B) and (D). Reactions of Rb-6791 serum with L4s, and Rb-7539 serum with L3s, also were comparable to the reactions shown in (B) and (D). All photomicrographs are comparable exposures. The magnification of photomicrographs (A) and (B) is approximately twice that of (C) and (D).

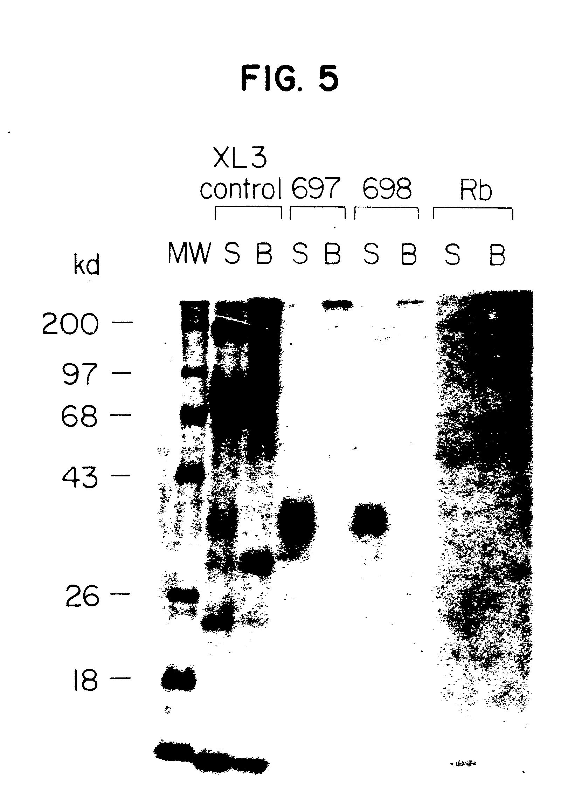

Figure 5 shows an immunoprecipitation of $^{125}$I-labeled XL3 surface proteins with immune sheep sera. S

3

and B refer to SDS or SDS + BME extracts of XL3s.

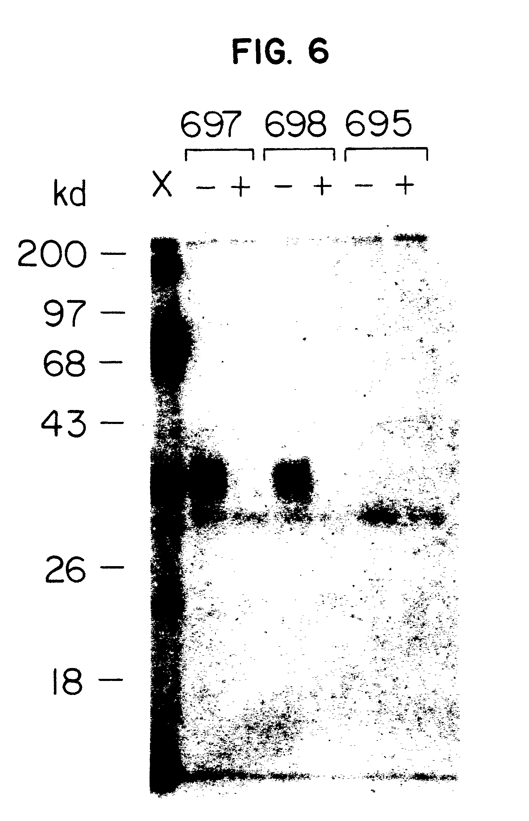

Figure 6 shows an immunoprecipitation of $^{125}$I-labeled XL3 surface proteins with immune sheep sera. XL3 protein samples were reduced with BME (+ lanes) or not reduced with BME (- lanes).

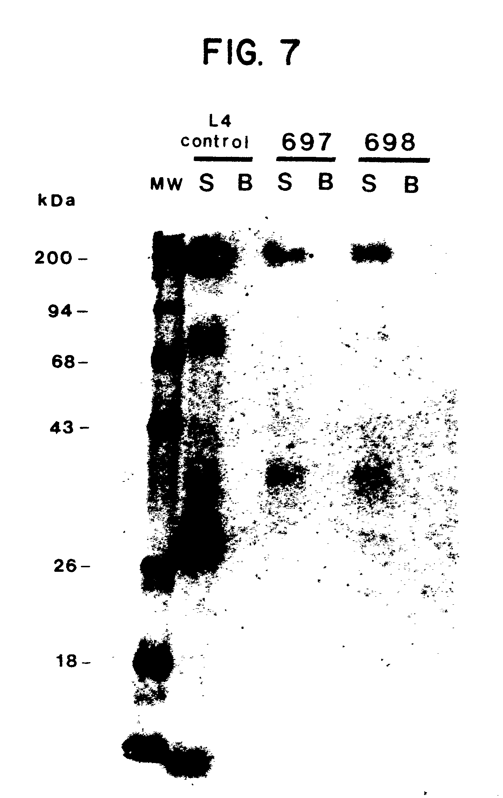

Figure 7 shows an immunoprecipitation of $^{125}$I-labeled L4 surface proteins with immune sheep sera. S and B refer to SDS or SDS + BME extracts of L4s.

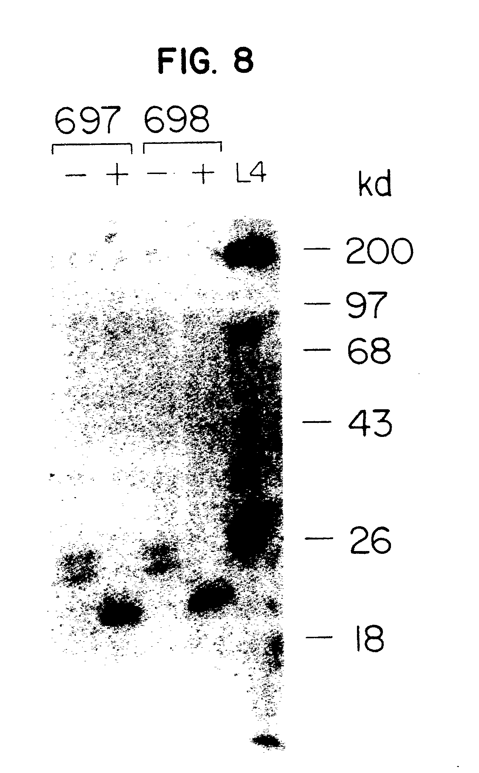

Figure 8 shows an immunoprecipitation of $^{125}$I-labeled L4 surface proteins before (- lanes) or after (+ lanes) treatment with Endoglycosidase F.

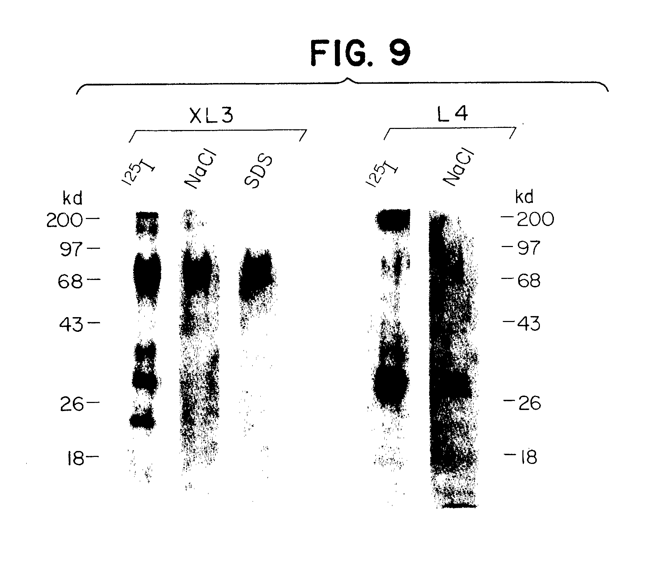

Figure 9. Comparison of Haemonchus contortus XL3 and L4 surface proteins identified by $^{125}$I surface labeling and purified by various extraction procedures. Surface proteins labeled by $^{125}$I and chloramine T ($^{125}$I lanes) are compared to Coomassie blue stained gel patterns of proteins extracted by the SDS (SDS lane) or NaCl (NaCl lanes) procedures. Protein samples were electrophoresed in 12% SDS-polyacrylaminde gels. Each of the stained gel lanes contain 10 micrograms of proteins. Molecular weights of proteins standards, in kilodaltons, are indicated.

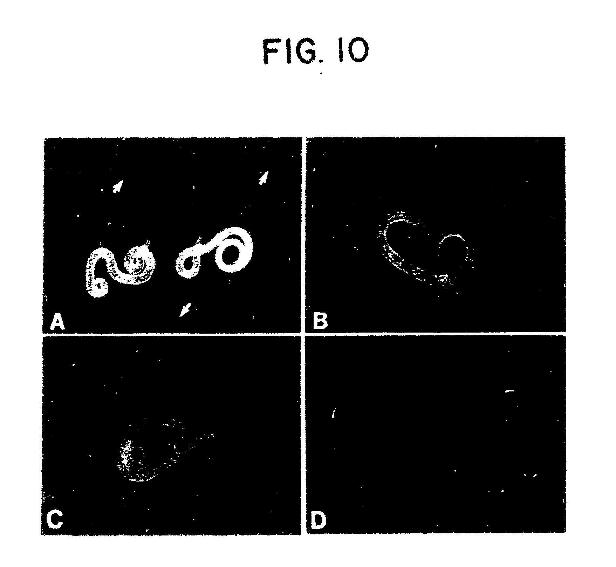

Figure 10. Fluorescence photomicrographs of live Haemonchus contortus XL3s and L4s incubated with rabbit antisera prepared against XL3 and L4 surface protein extracts. The reaction of anti-XL3 surface protein serum (Rb-9446) with a mixture of XL3s and L4s is shown in (A). Two XL3s in the center of the field fluoresce brightly, while L4s are negative. Arrows indicate positions of several of the L4s. The positive reactions of anti-L4 surface protein serum (Rb-153) with live XL3s and L4s are shown in (B) and (C), respectively. The negative reaction of Rb-153 pre-bleed serum with L4s is shown in (D). Reaction of Rb-153 pre-bleed serum with XL3s was comparable to that seen with L4s. Reactions of immune Rb-154 serum (anti-SDS denatured L4 surface protein serum) with XL3s and L4s were comparable to the reactions shown in (B) and (C).

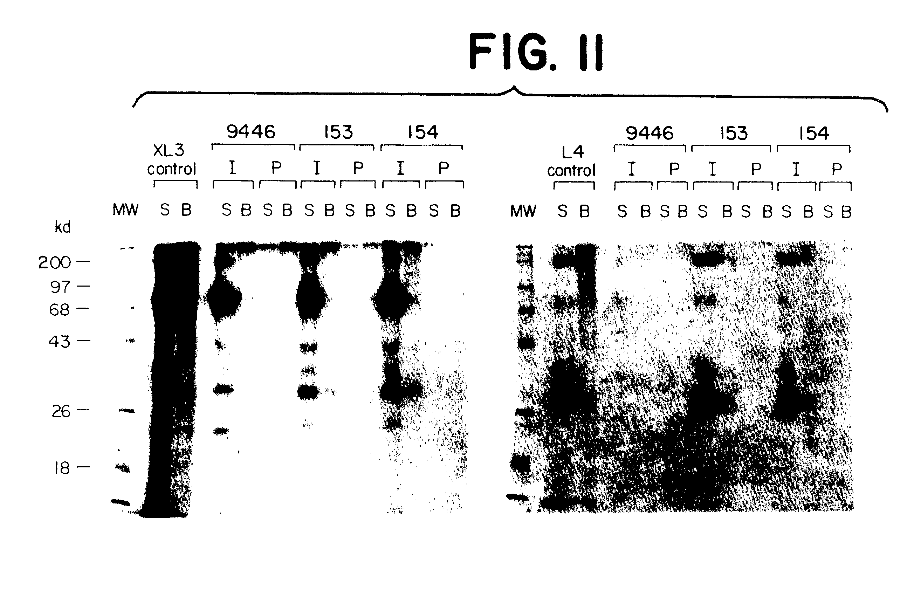

Figure 11. Immune-precipitation reactions of $^{125}$I-labeled XL3 (left panel) and L4 (right panel) surface proteins with rabbit antisera prepared against surface proteins. The XL3 and L4 control lanes show the patterns of $^{125}$I-labeled XL3 and L4 proteins in the samples used for the immune-precipitation reactions. Aliquots of $^{125}$I-labeled surface proteins were incubated overnight with pre-bleed (P) or immune (I) anti-XL3 (Rb-9446) or anti-L4 (Rb-153 and RB-154) rabbit sera. SDS-soluble (S) and SDS + BME-soluble (B) surface-labeled proteins were analyzed separately. Immune precipitates were analyzed on 12% SDS-polyacrylamide gels. The gel was dried and autoradiographed. Molecular weights of protein standards (MW), in kilodaltons, are shown to the left.

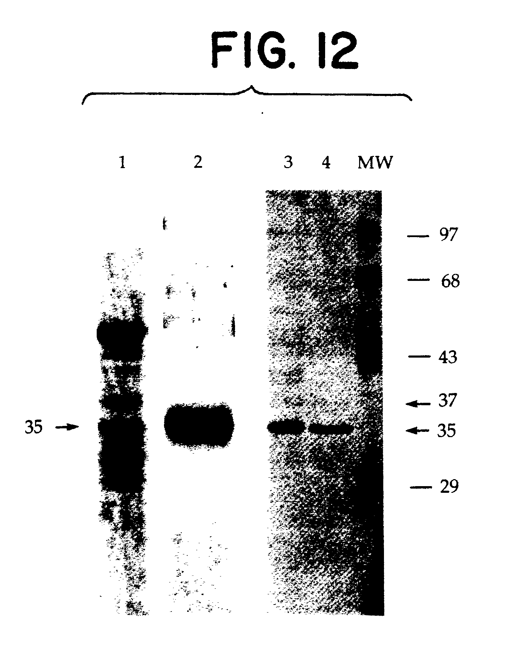

Figure 12. Gel purification and characterization of rabbit antisera prepared against the 35 kDa protease. Proteins present in the active fractions eluting in the void volume of a Sepharose CL-4B column are shown in Lane (1). The arrow indicates the position of the 35 kDa thiol protease. The 35 kDa protein obtained by electroelution from preparative SDS gels is shown in Lane (2). The eluted 35 kDa band was used to immunize rabbit #10285. The reaction of this immune sera with total adult worm proteins and with anticoagulant extracts obtained by FPLC Mono Q column chromatography are shown in Lanes (3) and (4), respectively. The 37 kDa protein that reacts weakly with the antiserum is indicated with an arrow. Molecular weights of protein standards are shown to the right.

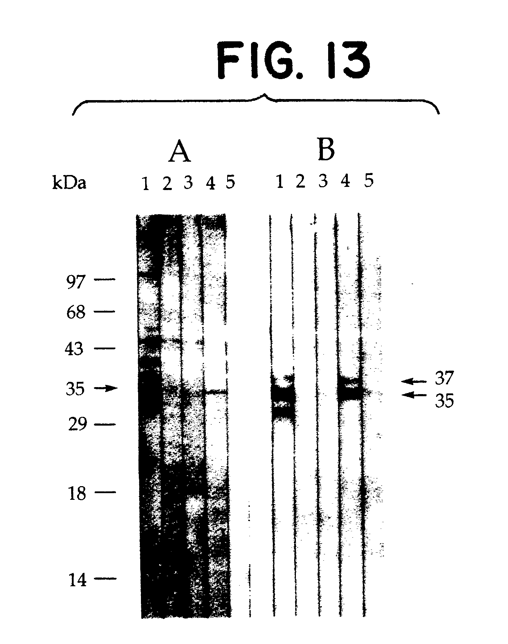

Figure 13. Western blot analyses of antibodies selected by recombinant phage clones. Phage clones isolated by screening the Ggt11:H. contortus adult cDNA expression library were plated on agar plates, overlain with nitrocellulose filters, grown overnight, and the filters incubated with Rb-10285 antiserum and washed. Bound antibodies were eluted with low pH glycine buffer, neutralized and used to probe Western blots of total adult worm proteins (Panel A) or Mono Q purified anticoagulant extracts (Panel B). Lane 1 shows the reaction of Rb-10285 serum with these antigens. The reactions of antibodies selected by phages λgt11, 2A, 2B and 4A are shown in Lanes 2, 3, 4 and 5, respectively. Only phage 2B selected antibodies that react with the 35 kDa protein (indicated by an arrow) in both Panels A and B. Antibodies selected by this phage clone also react weakly with a 37 kDa protein (arrow). Molecular weights of protein standards are indicated to the left.

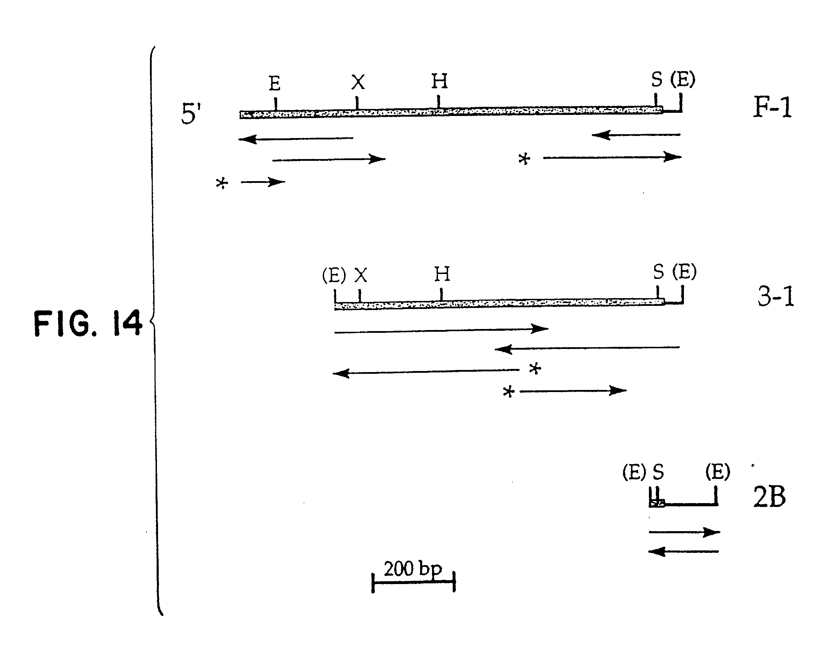

Figure 14. Relationship of cDNAs 2B, 3-1 and F-1. The relative sizes and restriction maps of cDNAs 2B, 3-1 and F-1 are shown. The thick horizontal lines denote coding regions; the thin horizontal lines represent 3' untranslated sequences. Regions of the cDNAs that were sequenced are indicated by arrows. Asterisks indicate sequences that were determined using synthetic oligonucleotide primers. Restriction enzyme sites shown are EcoRI (E); HindIII (H); SaII (S); and, XhoI (X). The EcoRI sites present at the 5' and 3' ends of the cDNAs, which were added during the cloning procedure, are indicated in parentheses. cDNA F-1 has a defective EcoRI site at its 5' end. Note that the lengths of the 3' untranslated regions differ in 2B versus 3-1 and F-1.

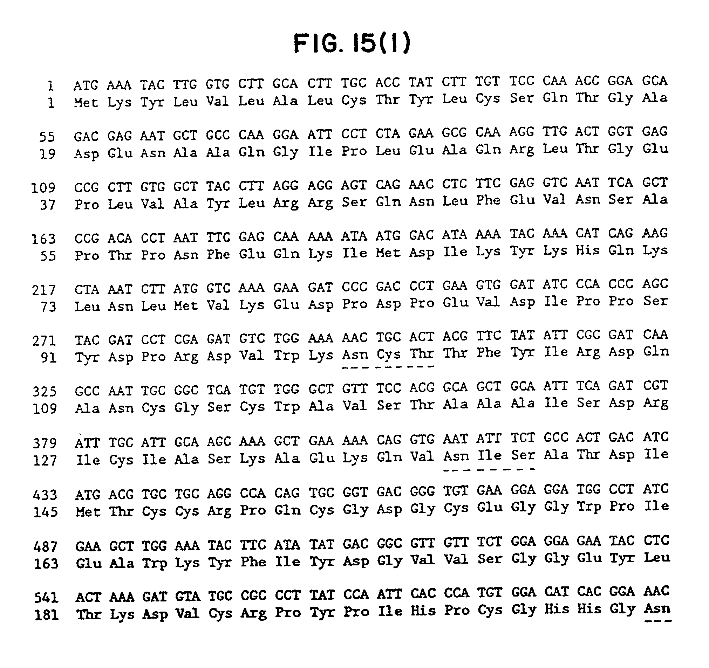

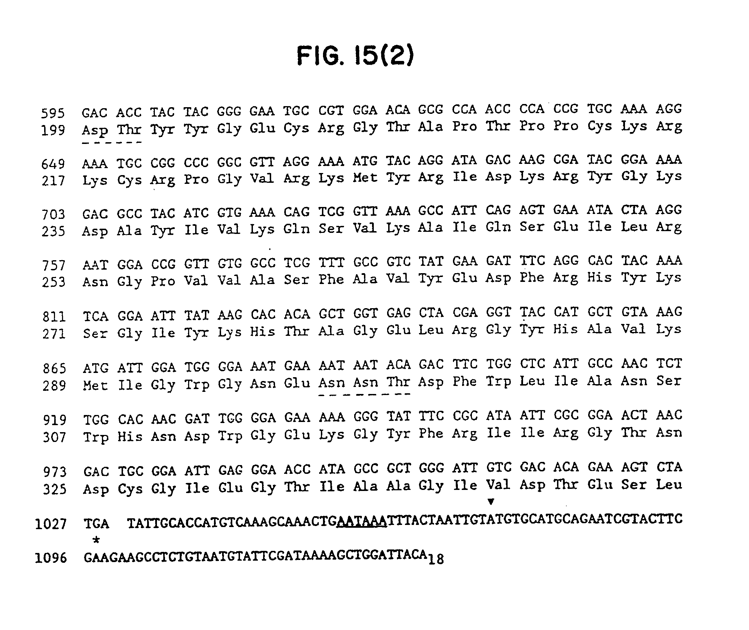

Figure 15. Nucleotide and predicted amino acid sequence of AC-1. The sequence shown is a composite of sequences obtained from various regions of cDNAs 2B, 3-1 and F-1. The AT of the initiator ATG shown is not present in the cDNAs and was inferred from the sequence of the AC-2 gene isolated from an H. contortus:λEMBL-3 phage library. The EcoRI linkers added during the cloning process are not shown. Potential N-linked glycosylation sites are underlined with dashes. The asterisk denotes the termination codon. The position of a potential poly(A) additional signal, AATAAA, is underlined. The solid triangle at nucleotide 1073 indicates the location of the poly(A) tail in cDNAs F-1 and 3-1.

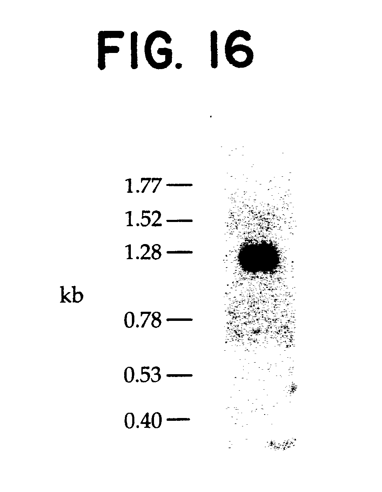

Figure 16. Northern blot analysis of AC-1 mRNA transcripts. 1.5 μg of adult worm poly(A)$^+$ mRNA was electrophoresed on a 1.5% denaturing formaldehyde agarose gel, blotted onto a nitrocellulose filter and hybridized with a $^{32}$P- labeled pBR325 plasmid containing the ~1.0 kb EcoRI fragment of cDNA F-1. The size of the hybridizing mRNA is about 1.25 kb. Positions of RNA size markers are indicated on the left.

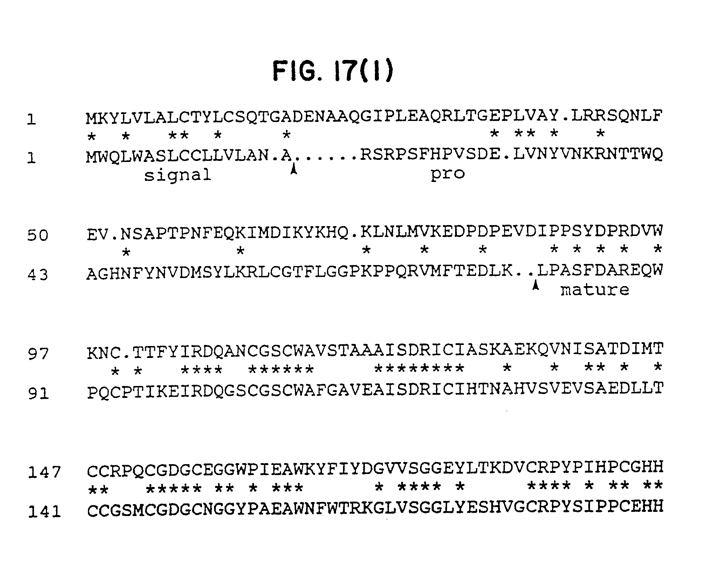

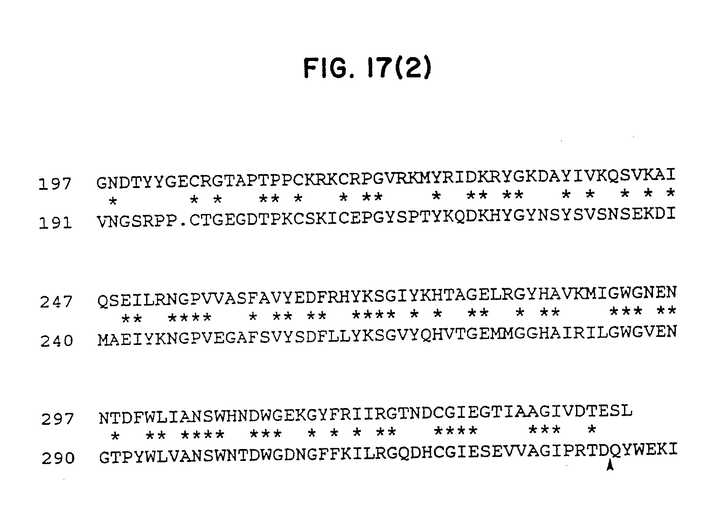

Figure 17. Comparison of the predicted amino acid sequence of AC-1 with human cathepsin B. The upper sequence is AC-1, the lower sequence is human cathepsin B, which is taken from [17]. Amino acid positions are indicated to the left. Dots indicate gaps that were introduced to increase similarities between the proteins. Identical amino acids in the proteins are indicated by an asterisk. Arrowheads denote positions of cleavages that occur during maturation of cathepsin B. The location of the signal sequence, "pro" sequence and mature enzyme sequence of cathepsin B are shown and blocked by the arrowheads. The final six amino acids of cathepsin B are not present in the mature enzyme (cleavage indicated by an arrowhead).

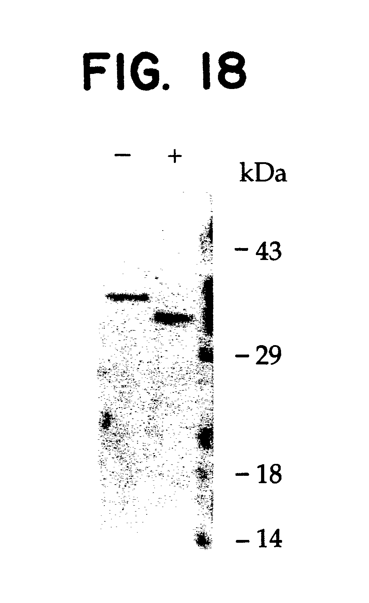

Figure 18. Deglycosylation of AC-1 with Endoglycosidase F. Mono Q-purified anticoagulant proteins (~2μg) were denatured by boiling in 1% SDS/5% -mercaptoethanol and incubated overnight with buffer (- Lane) or buffer + 1.5 units of Endoglycosidase F ( + Lane). The next day the samples were electrophoresed on a 12% SDS gel, blotted to a nitrocellulose filter and reacted with Rb-10285 antiserum. Molecular weights or protein standards are shown to the right.

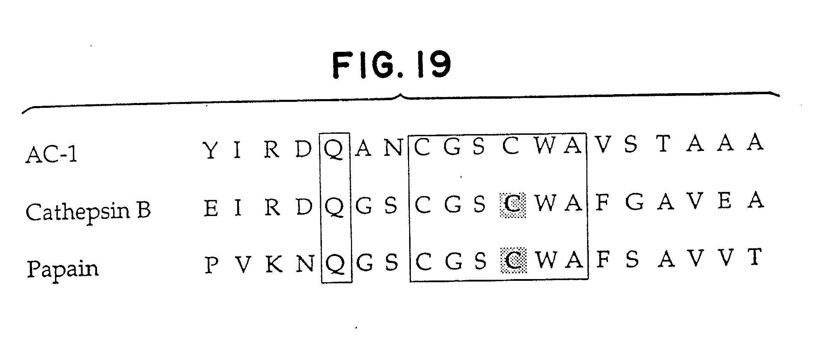

Figure 19. Comparison of the amino acid sequences surrounding the active site cysteines of cathepsin B, papain and AC-1. Amino acid residues 97-117 cathepsin B [17], 14-34 of the mature form of papain [18], and 102-122 AC-1 are compared. Amino acids that are identical in all three proteases are boxed. The active site cysteine residues of cathepsin B and papain are shaded. The corresponding cysteine residue of AC-1 is presumed to be the active site cysteine of this protease.

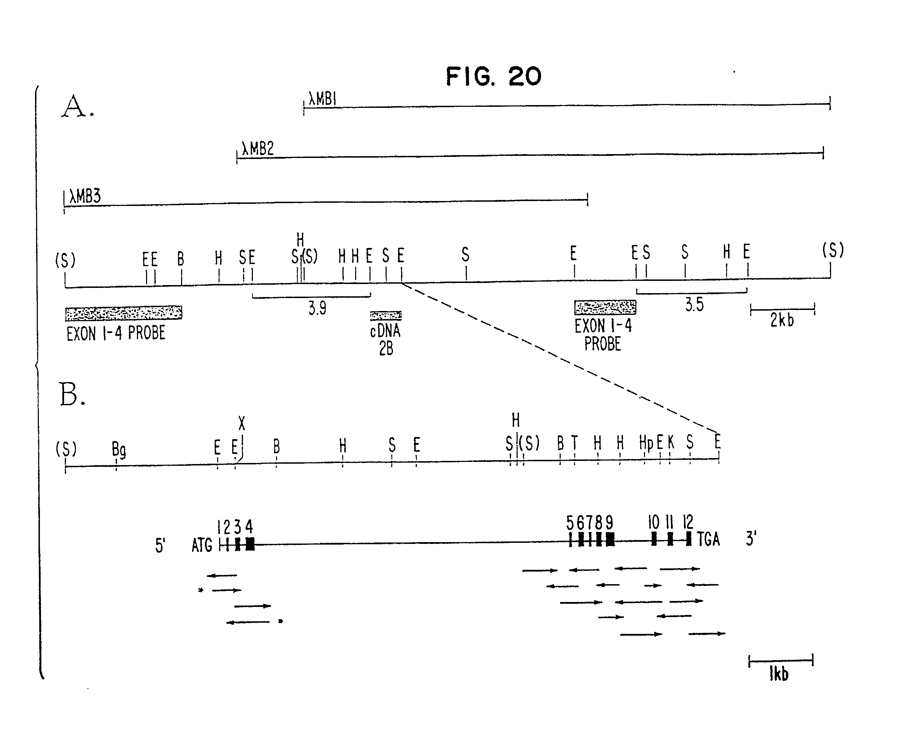

Figure 20. Restriction enzyme map and exon/intron organization of the Haemonchus contortus AC-2 gene. A composite restriction map of the AC-2 gene and flanking regions is shown in (A). The limits of recombinant λEMBL-3 phages λMB-1, λMB-2 and λMB-3 are shown above the map. Restriction enzyme sites shown are: E, EcoRI; S, SalI and H, HindIII. The SalI sites in parentheses occur in the EMBL-3 polylinker sequences and are not present in H. contortus DNA. They are shown because they were used to generate restriction fragments for DNA sequencing. The 3.9 kb and 3.5 kb EcoRI fragments of λMB2 that were used to doublescreen the λEMBL-3 library to isolate λMB3 are indicated by brackets. The 1.0 kb EcoRI fragment that hydridizes to the cDNA 2B is marked. Regions of the EMBL-3 phages that were sequenced are indicated by arrows in (B), which is an expanded version of the pertinent region of (A). Asterisks indicate sequences that were generated using synthetic oligonucleotide primers. Additional restriction enzyme sites shown are: B, BamHI, Bg, BglII; Hp, HpaI; K, KpnI, T, SacI; X, XbaI. In some cases the EMBL-3 phages contain additional sites for these restriction enzymes that are not shown. The exon/intron organization of the AC-2 gene also is shown in (B). Black boxes indicate exons. The horizontal length of the box approximates the length of the exon, except for exon 1 which consists only of the initiator ATG codon.

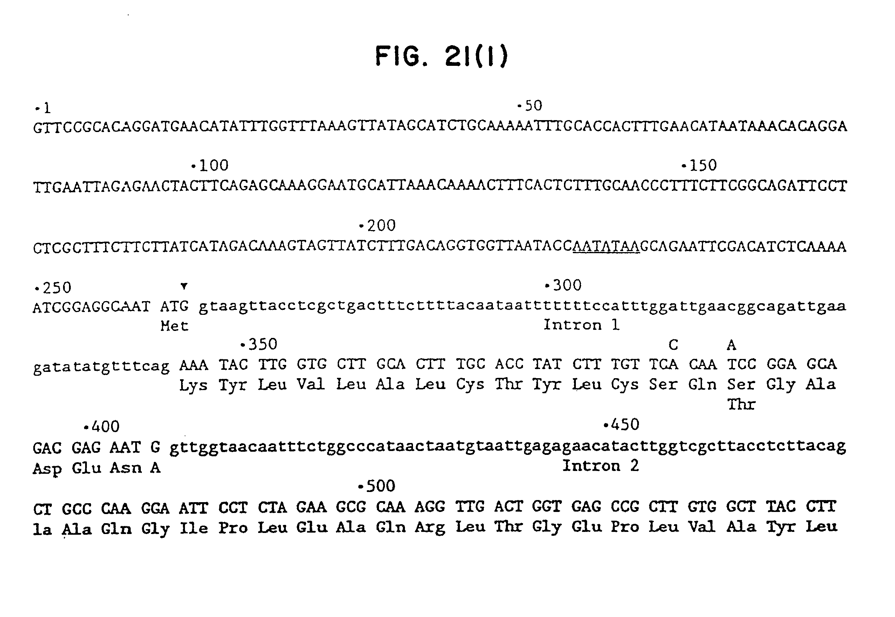

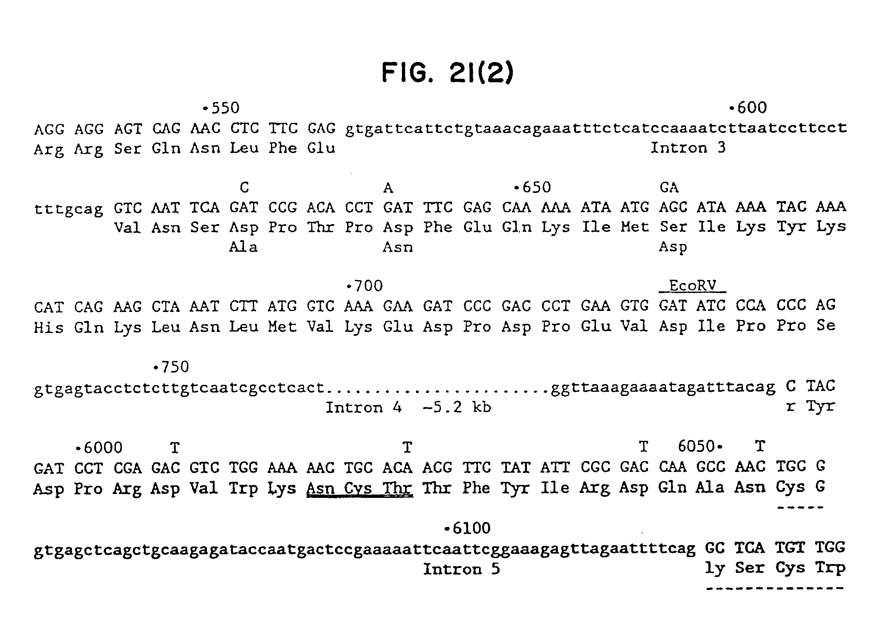

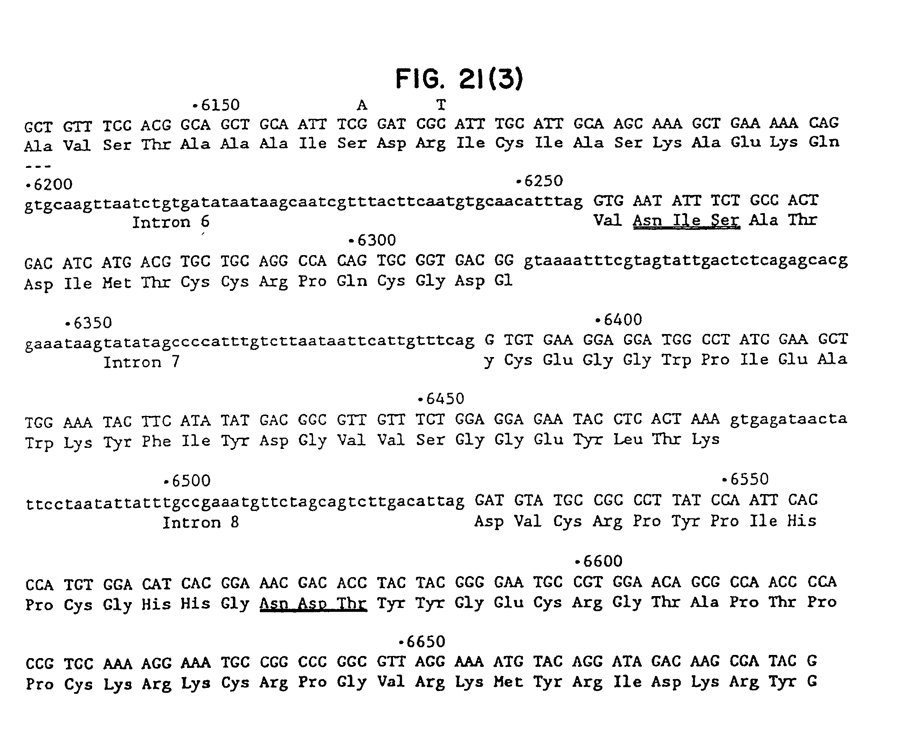

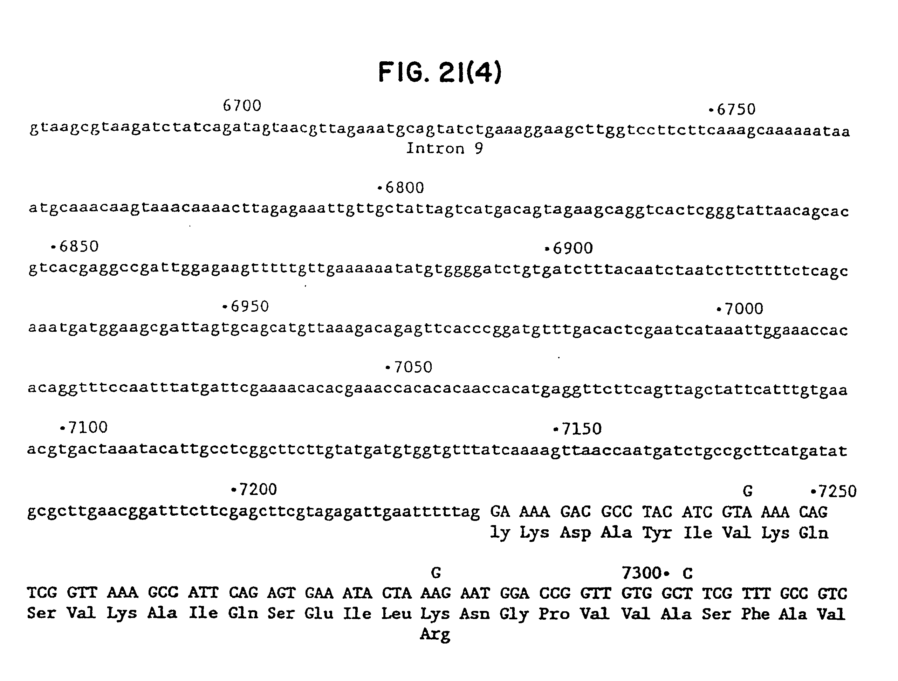

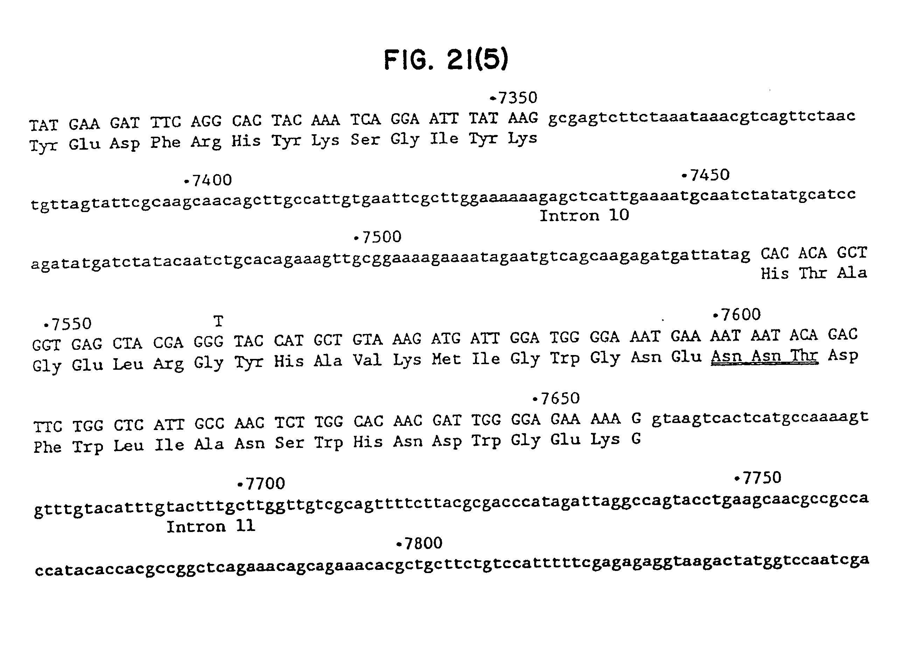

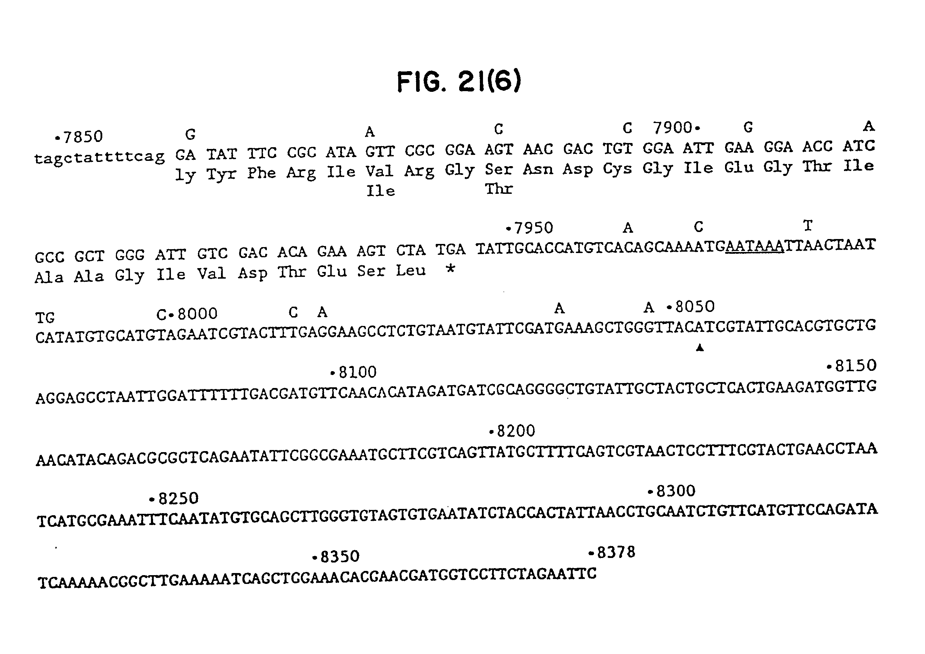

Figure 21. Nucleotide and deduced amino acid sequence of the Haemonchus contortus AC-2 gene. Lower case letters indicate introns. Nucleotides are numbered consecutively until intron 4, which is approximately 5.2 kb in length and was not sequenced in its entirety. Nucleotide numbers after intron 4 are approximations. Nucleotides and amino acids that are different in the AC-1 cDNAs F-1 and 2B are shown above and below the AC-2 sequences. Nucleotides corresponding to the beginning and end of the cDNAs are marked with solid triangles. Potential N-linked glycosylated sites are marked with double underlines. The six amino acids that are present in the active site and conserved in AC-1, AC-2, cathepsin B and papain are underlined with dashes. The arrow marks the EcoRV cleavage site present in AC-1 and AC-2 that was used to create the AC-1:β-galactosidase gene fusion. The termination codon is marked with an asterisk. Sequences similar to tha eukaryotic TATA promoter element and AATAAA polyadenylation signal are underlined.

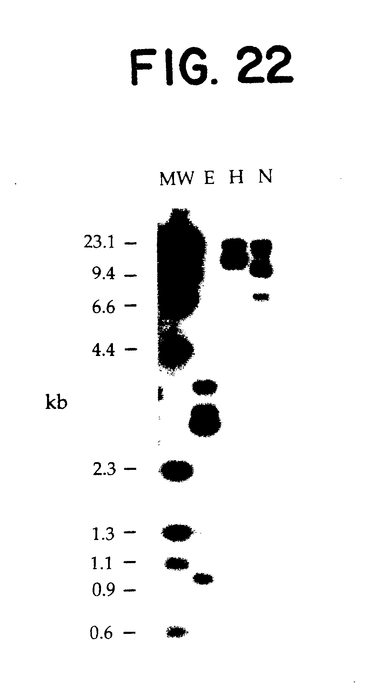

Figure 22. Southern blot analysis of Haemochus contortus AC protease genes. H. contortus genomic DNA (2μg) was digested with EcoRI (E); HindIII (H) or NheI (N), size-fractionated on a 0.8% agarose gel, blotted to a nitrocellulose filter and hybridized with the $^{32}$P-labeled AC-1 cDNA 2B (180 bp long) under low stringency conditions. Sizes of marker DNA fragments (MW) are indicated on the left in kilobase pairs.

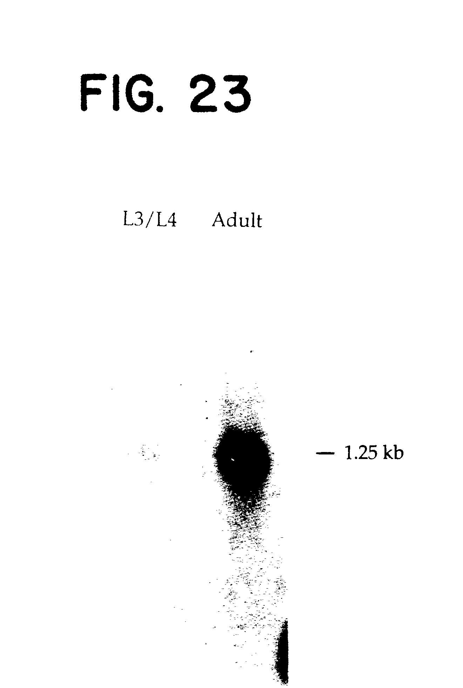

Figure 23. Northern blot analysis of Haemonchus contortus AC protease mRNAs. Poly (A)[+] mRNAs isolated from adult worms or from a mixed population of XL3s and young L4s were size-fractionated on denaturing formaldehyde gels, blotted to a nitrocellulose filter and hybridized under low stringency conditions with a $^{32}$P-labeled pBR325 plasmid containing the ~1 kb EcoRI fragment of AC-1 cDNA F-1. The size of the hybridizing mRNA is 1.25 kb.

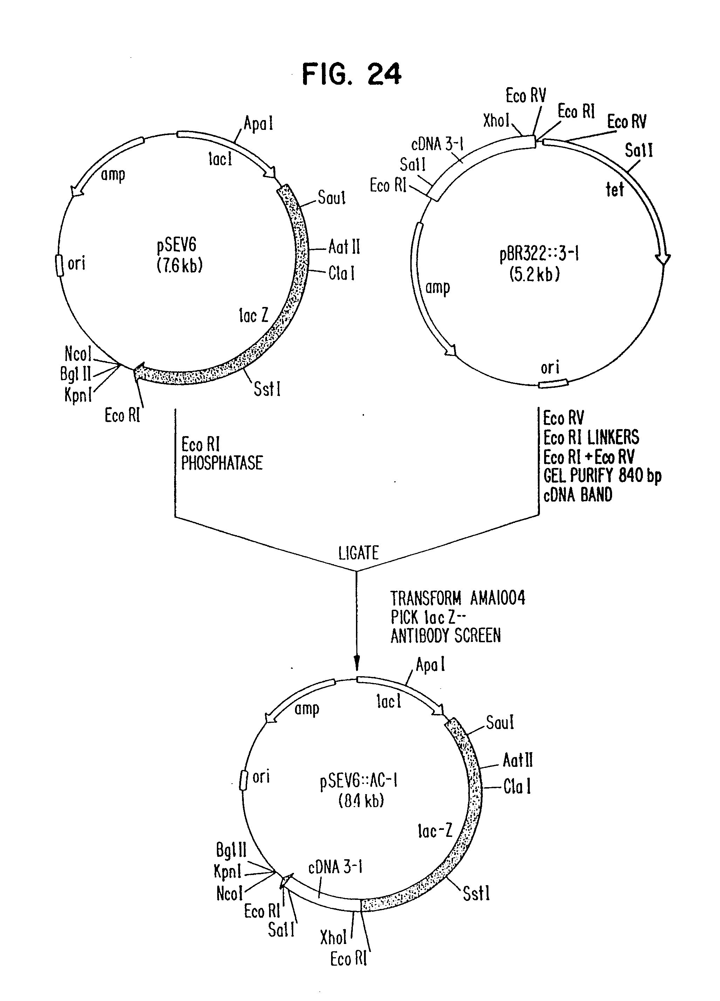

Figure 24. Construction of plasmid pSEV6::AC-1. The β-galactosidase expression vector pSEV6 was constructed from pSEV4 (U.S. Serial No. 023,113). The relative positions of the β-galactosidase gene (lacZ), lac I[Q] repressor gene (lacI) and unique EcoRI, SstI, KpnI, BglII and NcoI sites are indicated. To construct pSEV6::AC-1, plasmid pBR322::3-1, which contains the AC-1 cDNA 3-1 inserted into the EcoRI site of pBR322, was digested with EcoRV (this restriction site also present in AC-2 and is marked in Figure 2), ligated to synthetic EcoRI linkers, digested with excess EcoRI and EcoRV, and the 840 bp DNA fragment purified by agarose gel electrophoresis. This fragment was inserted into the EcoRI site present in the β-galactosidase gene of pSEV6. Plasmids containing the cDNA inserted in the correct orientation for expression were identified by screening lacZ-bacterial colonies with Rb-10285 antiserum and by mapping plasmid DNAs with XhoI.

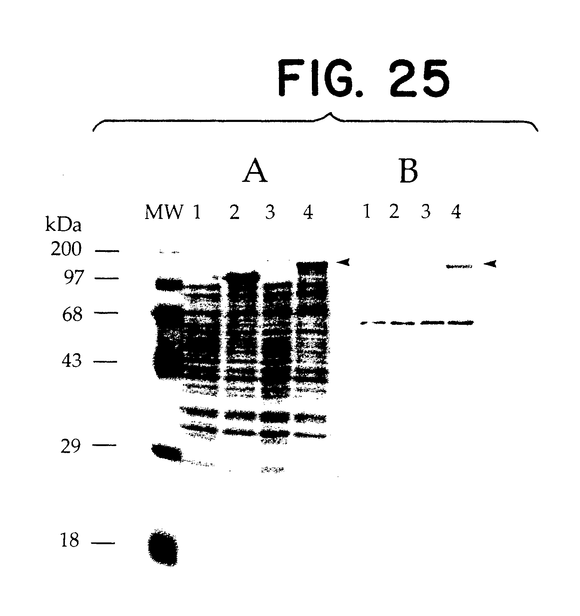

Figure 25. Expression of recombinant AC-1:β-galactosidase fusion protein in E. coli. E. coli cells harboring plasmid pSEV6 or pSEV6::AC-1 were grown in the presence (induced) or absence (uninduced) of IPTG. Equal volume aliquots of the cultures were boiled in SDS sample buffer and electrophoresed on 12% SDS gels. Panel (A) shows the Coomassie blue strained gel of the E. coli proteins. Panel (B) shows a Western blot of the E. coli proteins probed with rabbit antisera (Rb-10285) prepared against the 35 kDa protein purified from H. contortus adults (11). Lanes shown are: (1) pSEV6, uninduced; (2) pSEV6, induced; (3) pSEV6::AC-1, uninduced; and (4) pSEV6::AC-1, induced. The AC-1 fusion protein is indicated with an arrowhead. Positions of molecular weight markers are indicated on the left.

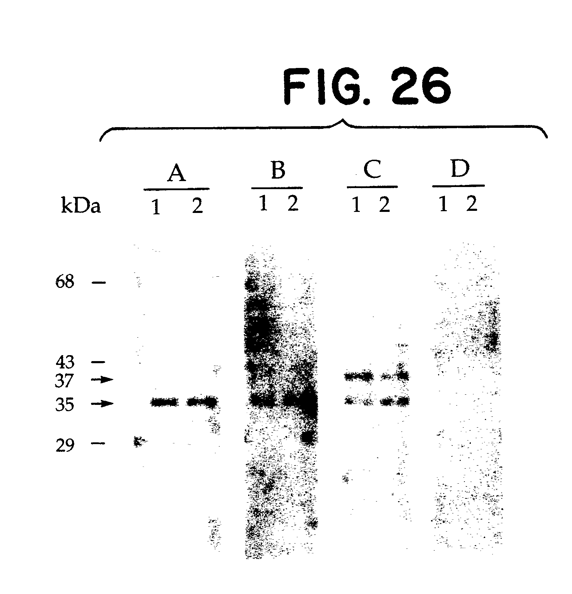

Figure 26. Western blot analyses of adult worm proteins with rabbit antisera raised against the recombinant AC-1 protease. Aliquots of total adult worm proteins (lane 1) or Mono Q column-purified anticoagulant proteins (lane 2) were electrophoresed on 12% SDS gels, blotted to a nitrocellulose filter and reacted with rabbit antisera raised against the H. contortus protease. Panel (A) shows the reaction of Rb-10285 antiserum, which was raised against the 35 KDa protease purified from adult worms. Panels (B) and (C) show the reactions of Rb-9190 and Rb-8552 antisera, respectively, which were raised against the recombinant AC-1:β-galactosidase fusion protein. Panel (D) shows the reaction of Rb-9190 pre-bleed serum. All of the immune sera react with the 35 and 37 kDa forms of the protease in adult worms and in purified anticoagulant extracts. Arrows point to the positions of the 35 and 37 kDa forms of the protease. The reaction of Rb-8552 serum with the 37 KDa protein is consistently much stronger than that of the other immune sera. Positions of molecular weight markers are indicated to the left.

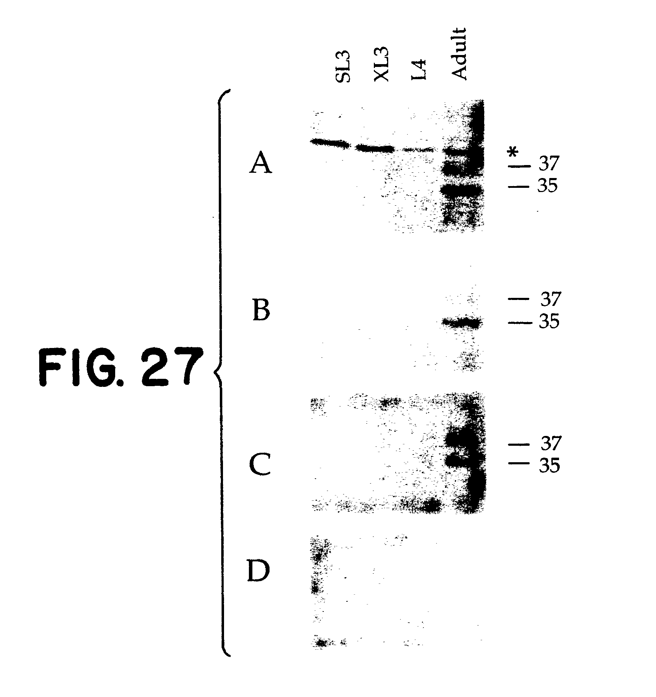

Figure 27. Developmental expression of the Haemonchus contortus AC-1 (AC-2) protease. Aliquots of SL3, XL3, L4 and adult worms containing equivalent amounts of proteins were separated on 12% SDS gels, blotted onto nitrocellulose filters and reacted with various rabbit antisera raised against the H. contortus protease. Panel (A) shows the reaction of RB-10285 serum, which was raised against the 35 kDa protease purified from Haemonchus contortus adult worms. This antiserum reacts with other proteins besides the protease. The 35 kDa and 37 kDa forms of the protease are marked with arrows. The band marked by an asterisk is not the protease and probably is tropomyosin. Panels (B) and (c) show the reactions of rabbit antisera Rb-9190 and Rb-8552, respectively, which were raised against the recombinant AC-1:β-galactosidase fusion protein. Panel (D) shows the reaction of pre-bleed serum from Rb-9190.

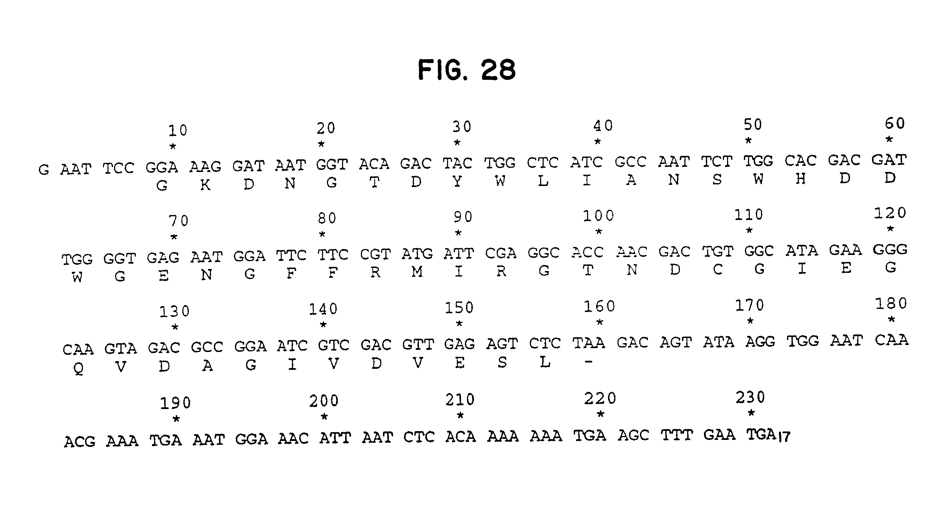

Figure 28 shows the nucleotide and predicted amino acid sequence of cDNA haemV24.

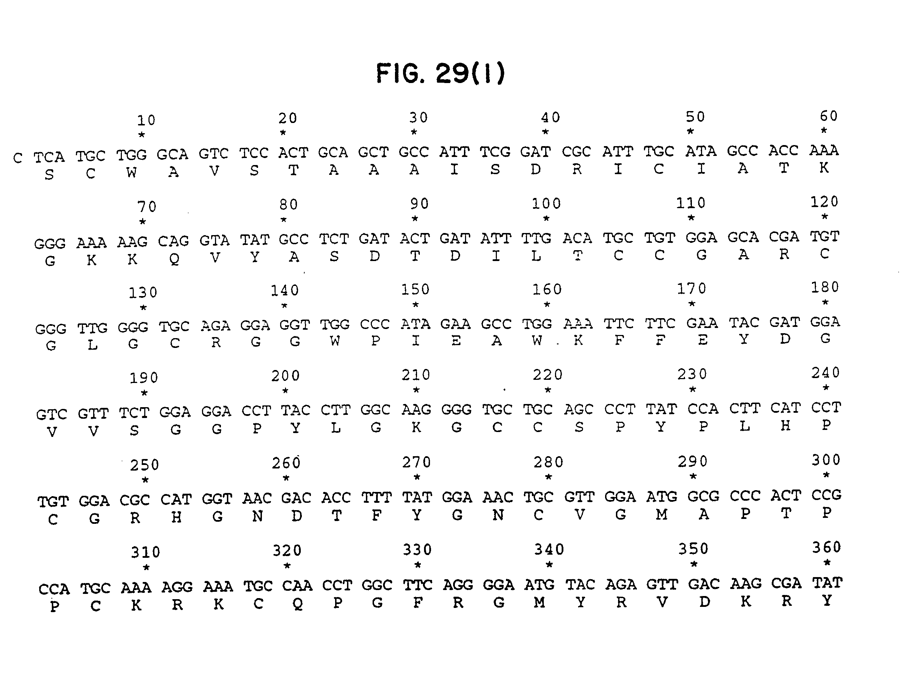

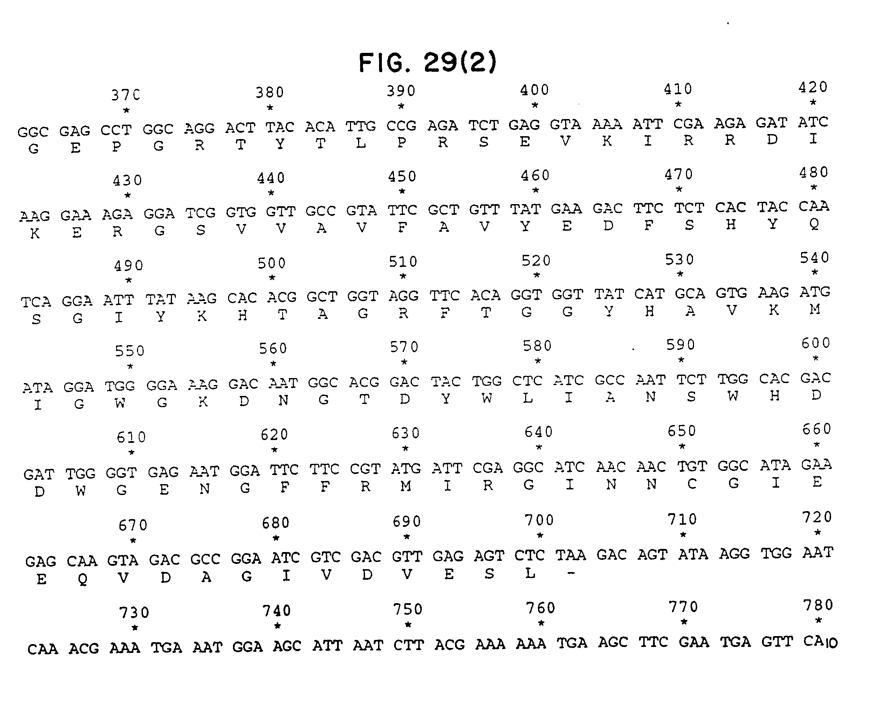

Figure 29 shows the nucleotide and predicted anino acid sequence of cDNA haemV22.

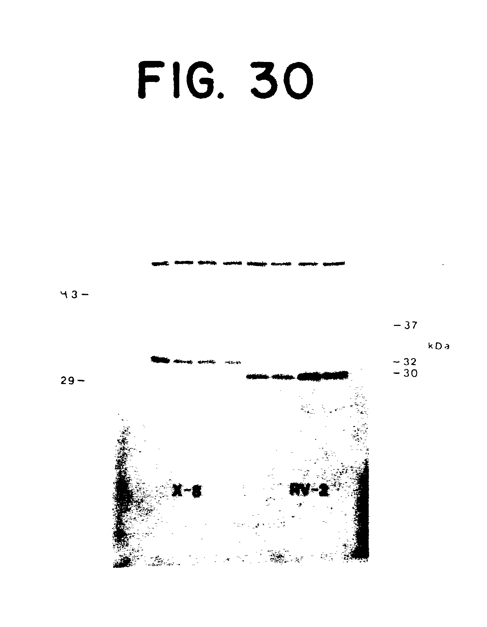

Figure 30 is a Coomassie stained gel and Western blot of AC-1 expressed in E. coli.

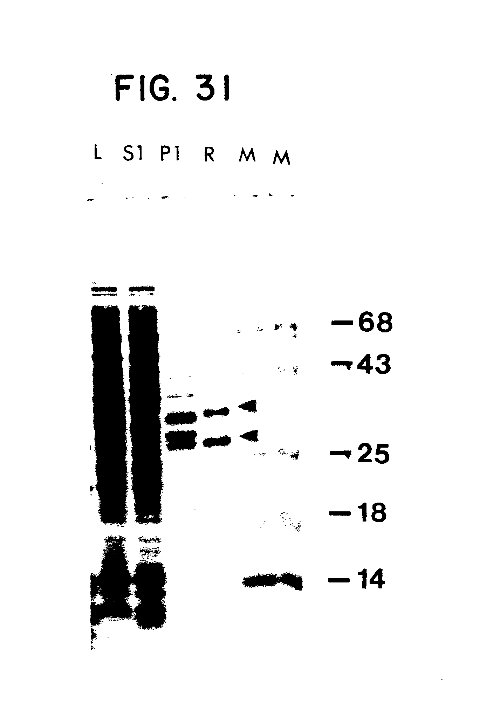

Figure 31. Coomassie stained SDS-PAGE of fractions from the isolation of 35 kDa recombinant protein. A 12% SDS-PAGE gel with a lane of molecular weight markers (M) indicates the stained pattern of the final product of the isolation of the recombinant 35 kDa protein. The arrows indicate the 37 and 32 kDa polypeptides. Also included as protein profiles from fractions obtained during the procedure; lysate, L; supernatant to the first centrifugation, S1 and resolubilized pellet, P1.

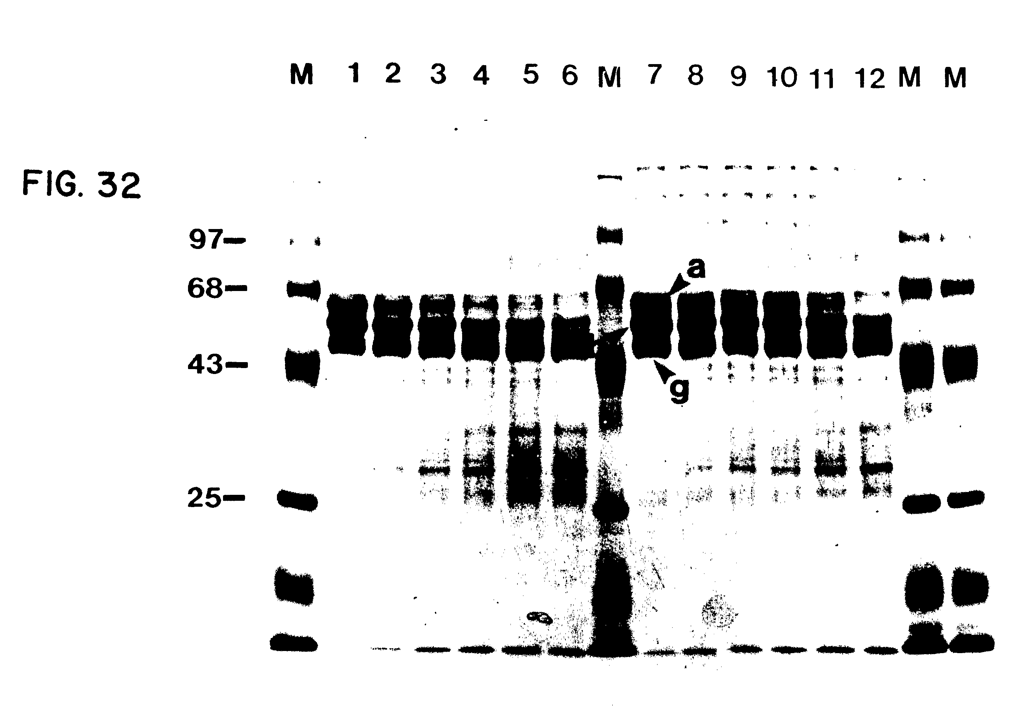

Figure 32. Stained SDS-PAGE of anti-coagulant assay. Anti-coagulant was assayed at increasing amounts; lanes 2 and 8, 0.1 ul; lanes 3 and 9; 0.2 ul; lanes 4 and 10, 0.4 ul; lanes 5 and 11, 0.6 ul; and lanes 6 and 12, 1 ul. Lanes 1 and 7 are fibrinogen incubated in the absence of anti-coagulant. Lanes 2 to 6 are enzyme from preparation E and lanes 8 to 12 preparation G. M indicates molecular weight lanes shown in Kd. Arrows indicate the fibrinogen polypeptides; a, alpha; b, beta; and g, gamma.

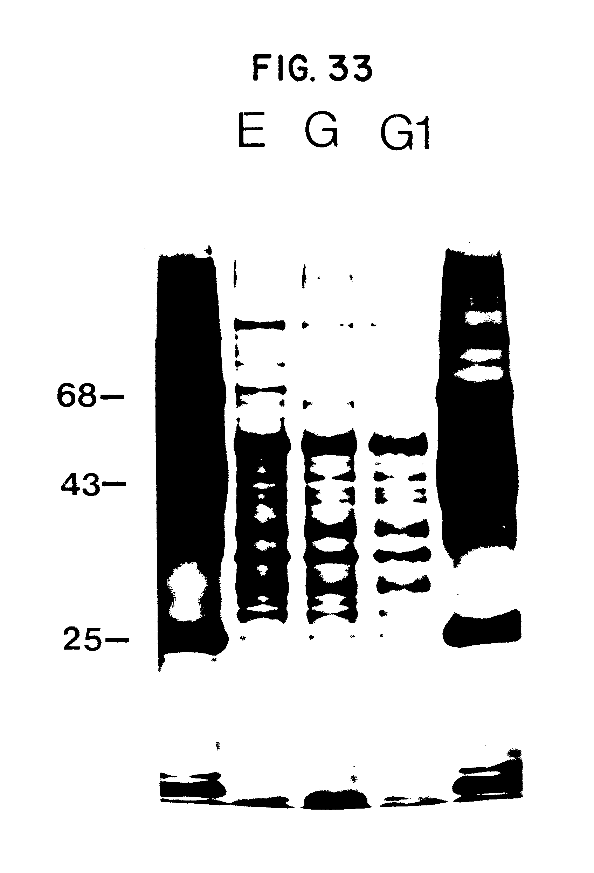

Figure 33. Silver-stained SDS-PAGE of anti-coagulant. The separate enzyme preparations E and G are indicated. G1 is a sample from the G preparation that was allowed to undergo several cycles of freezing and thawing. Molecular weight markers are in Kd.

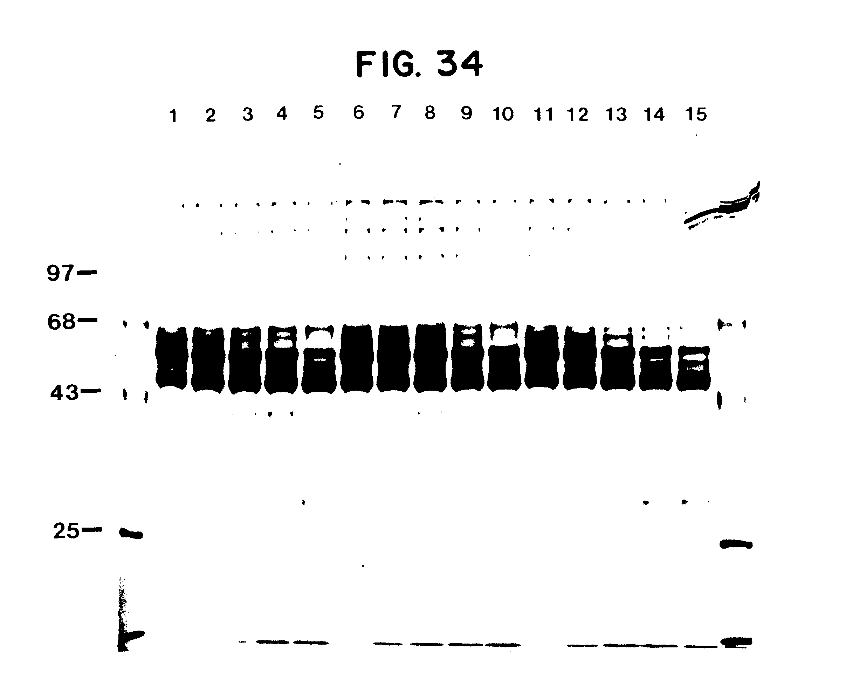

Figure 34. Anti-coagulant inhibition assay using antiserum to the 35 kDa polypeptide. Lanes 1 to 5, 6 to 10 and 11 to 15 represent increasing amounts of preparation E; 0, 0.1, 0.4, 0.6 and 1 ul, respectively. Lanes 1 to 5 represent enzyme pre-incubated with pre-immune serum (Rb-10285) to 35 kDa, lanes 6 to 10 represent enzyme pre-incubated with antiserum (Rb-10285) to 35 kDa and lanes 11 to 15 enzyme alone.

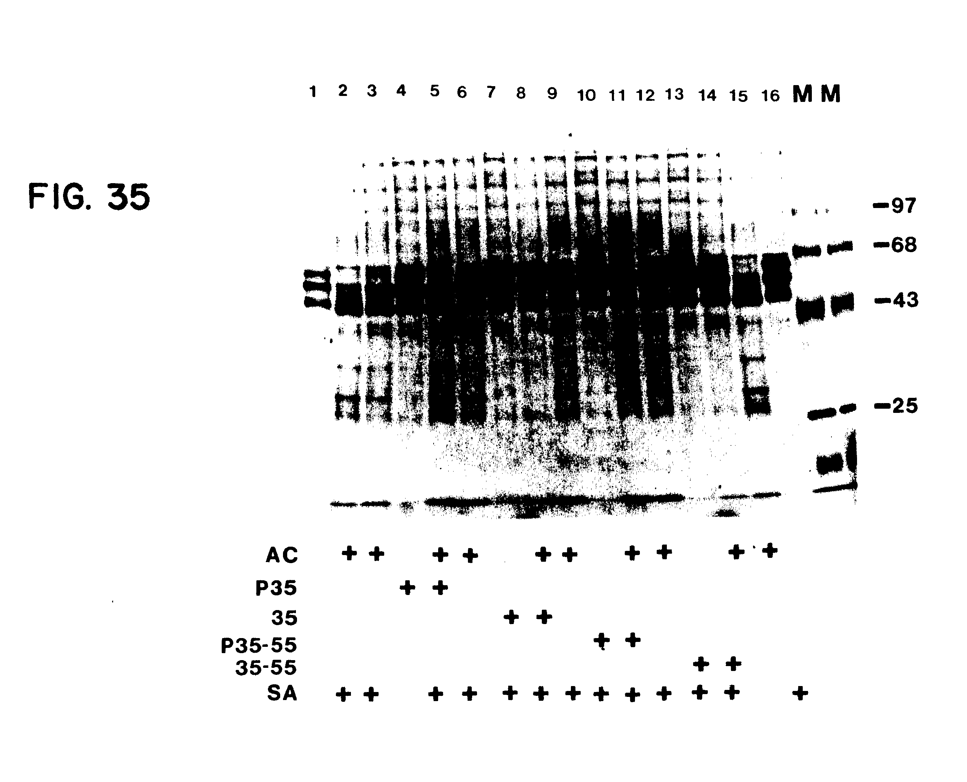

Figure 35. Gel analysis of 'immune complexing' experiment. Anti-coagulant is incubated with various IgG antisera fractions and then the complexes are sequestered from the solution using Staph A cells. The supernatants were then used in a standard fibrinogen assay. Underneath the lanes the + signs indicate the presence of E, enzyme; P-35, pre-immune serum (Rb-10285) to 35 kDa; 35, immune serum (Rb-10295) to 35 kDa; P-35/55, pre-immune serum (Rb-10286) to 55/35 kDa; 35/55, immune serum (Rb-10286) to 35/55 kDa and SA, Staph A cell treatment. M indicates the molecular weight lane shown in kDa.

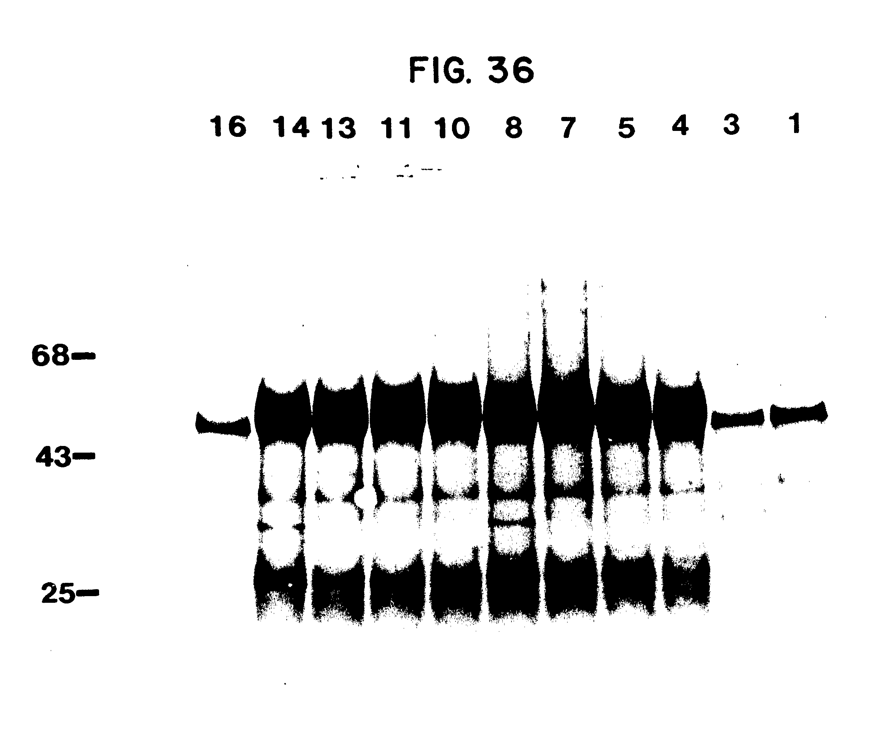

Figure 36. Western analysis of the 'immune complexed' components from the experiment described in Figure 35. The Staph A cells from Figure 35 were boiled in SDS sample buffer and the resultant supernatant electrophoresed, blotted and probed with antiserum reactive with the 35 and 55 kDa polypeptides. The second antibody was goat anti-rabbit HRP and reacts with the excess rabbit heavy chain. The abbreviations and the lanes indicated are as in Figure 35.

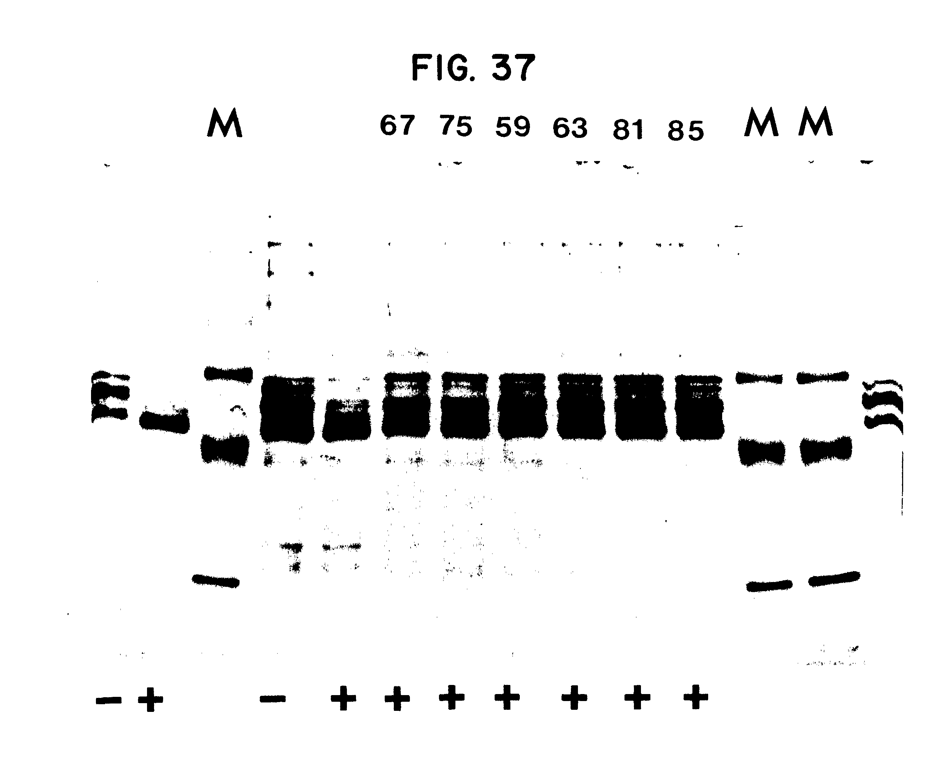

Figure 37. Gel analysis of enzyme inhibition with immune sheep sera. The IgG antiserum used are indicated above each lane; 67 and 75 are control sheep, while 59, 63, 81 and 85 are immune sheep. Underneath each lane the + and - signs indicate the presence and absence of enzyme in the assays.

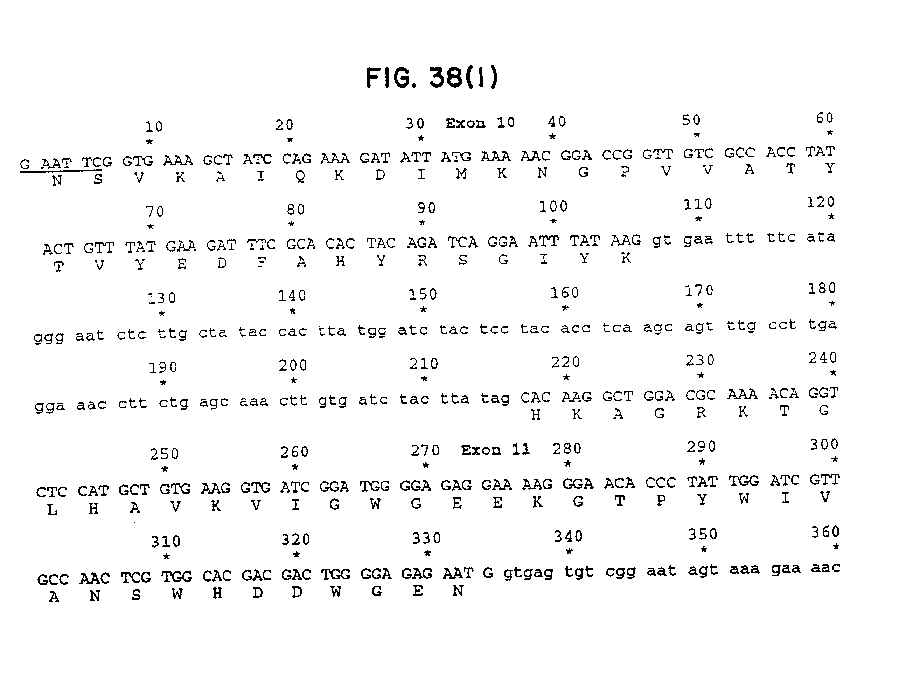

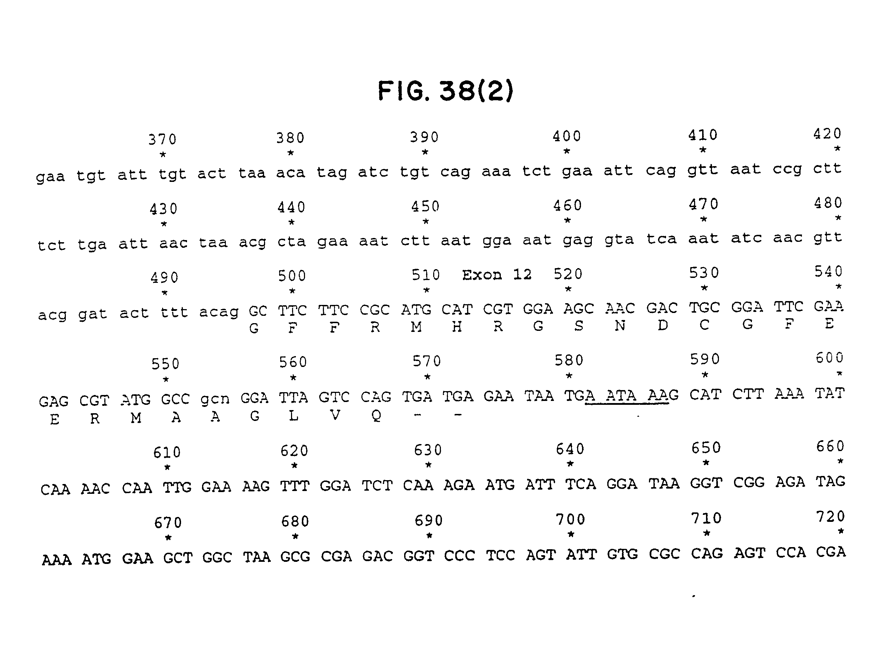

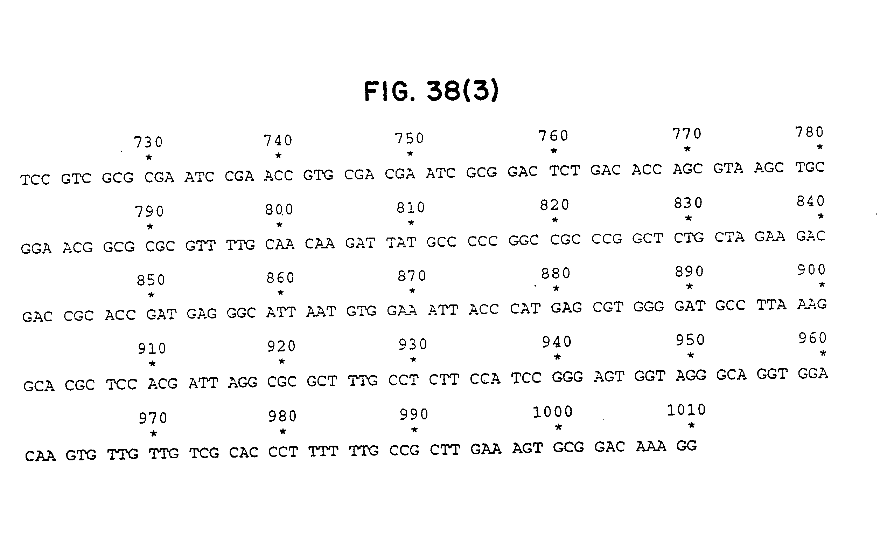

Figure 38 is the nucleotide and deduced amino acid sequence of the cysteine protease in λ002.

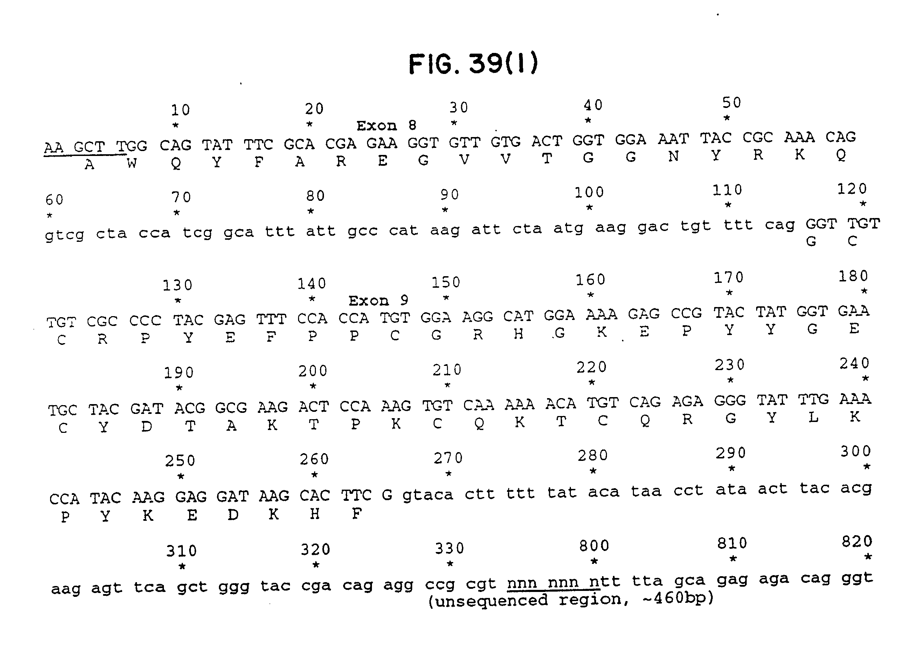

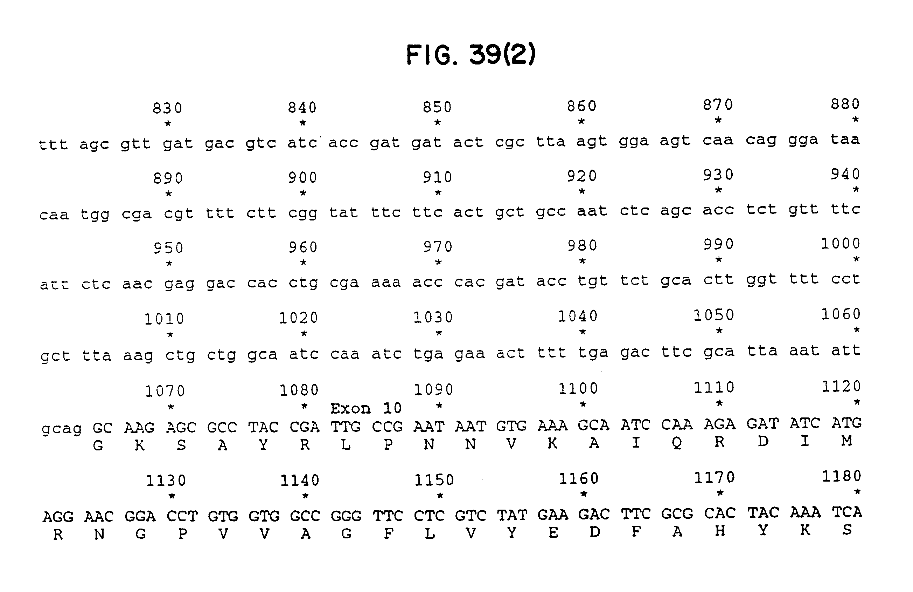

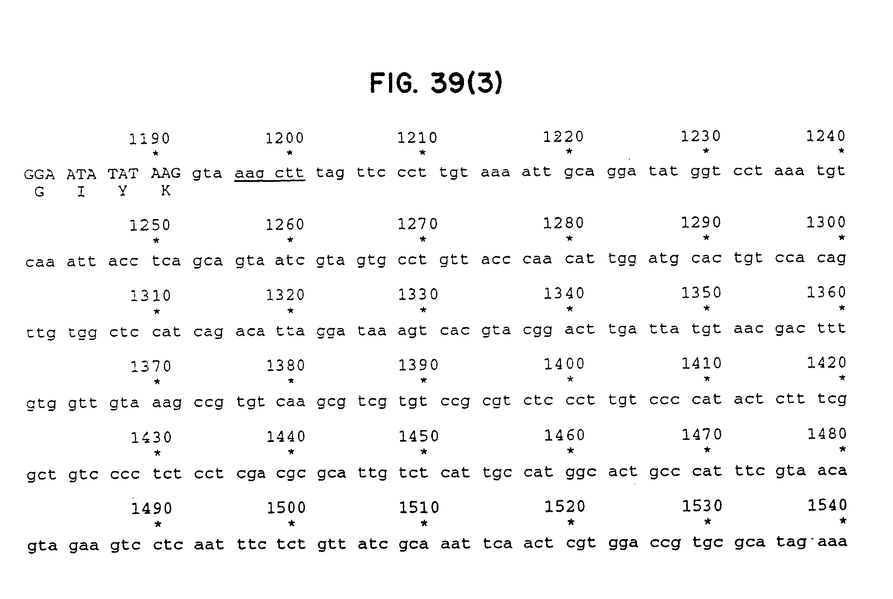

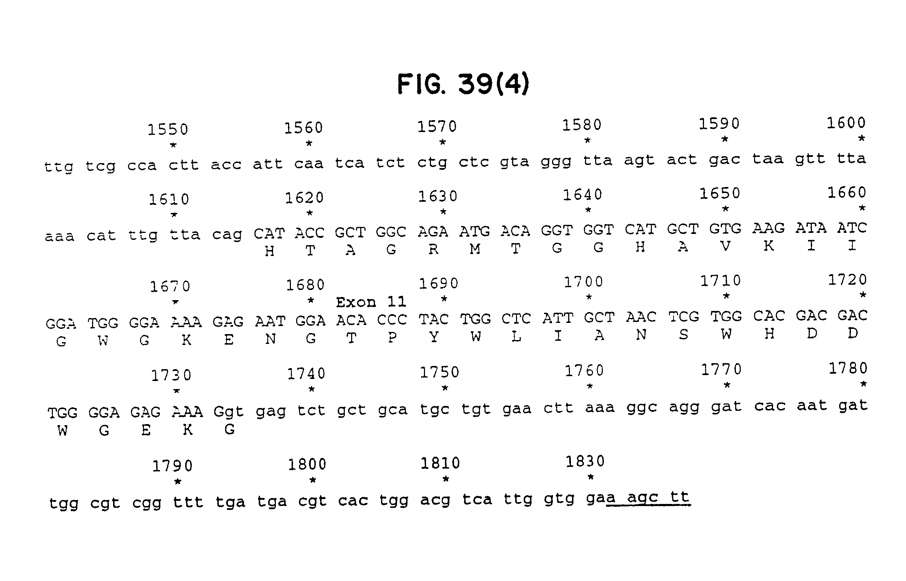

Figure 39 is the nucleotide and deduced amino acid sequence of the cysteine protease in λ003.

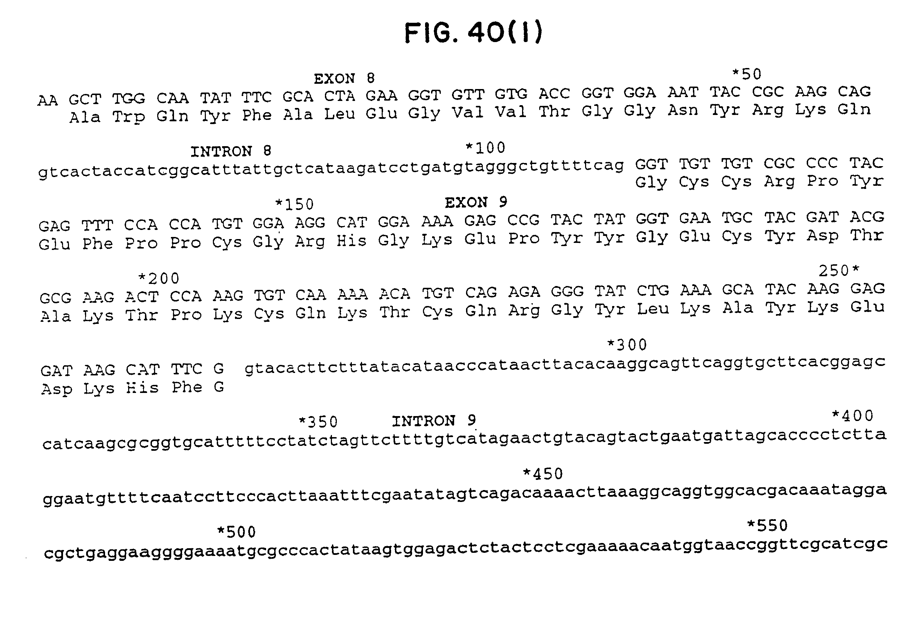

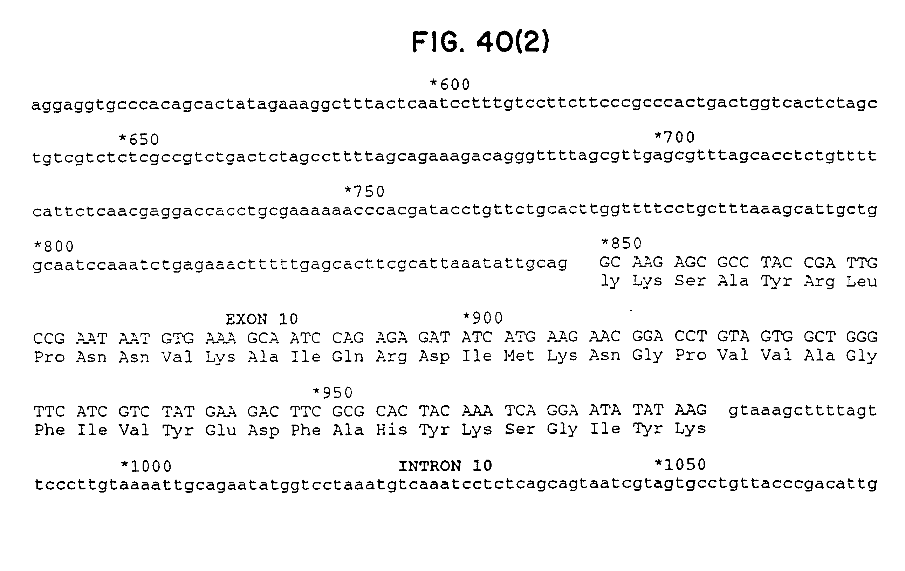

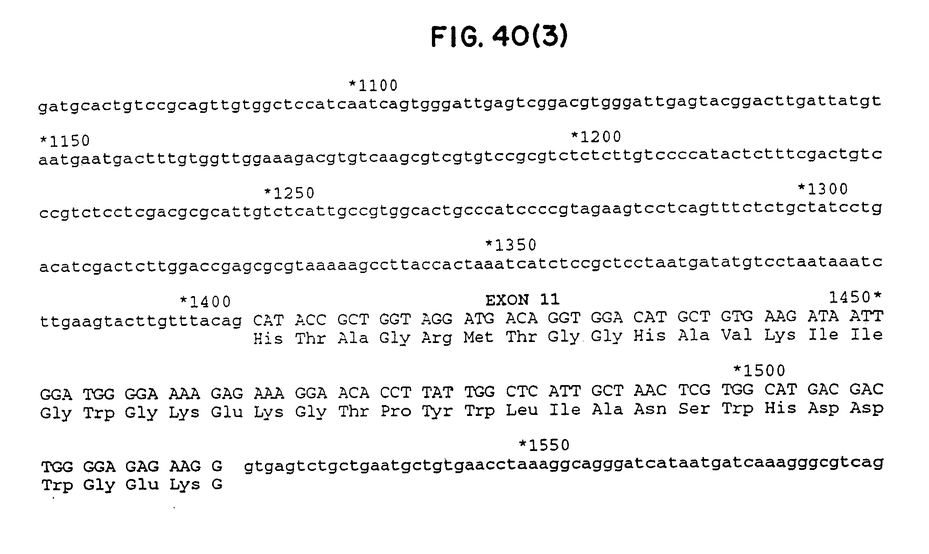

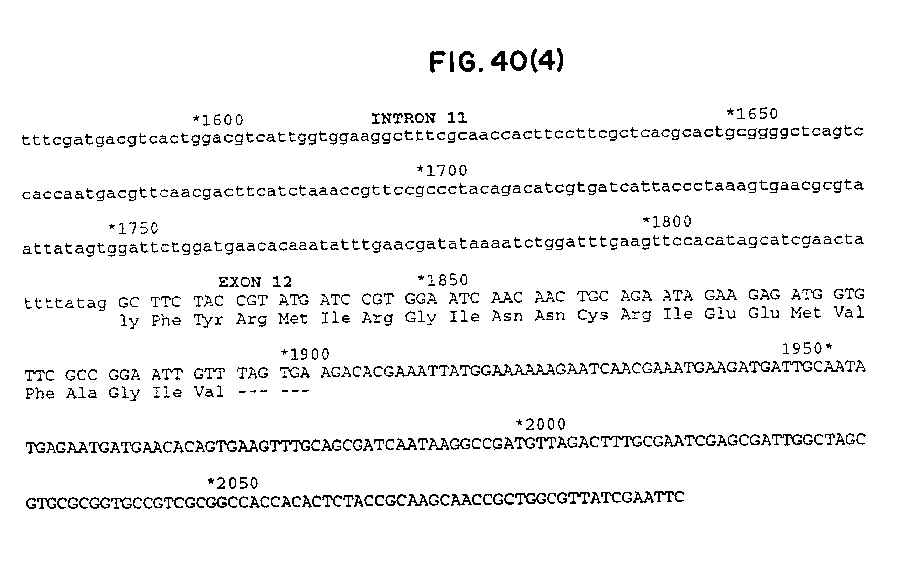

Figure 40 is nucleotide and deduced amino acid sequence of the cysteine protease in λ004.

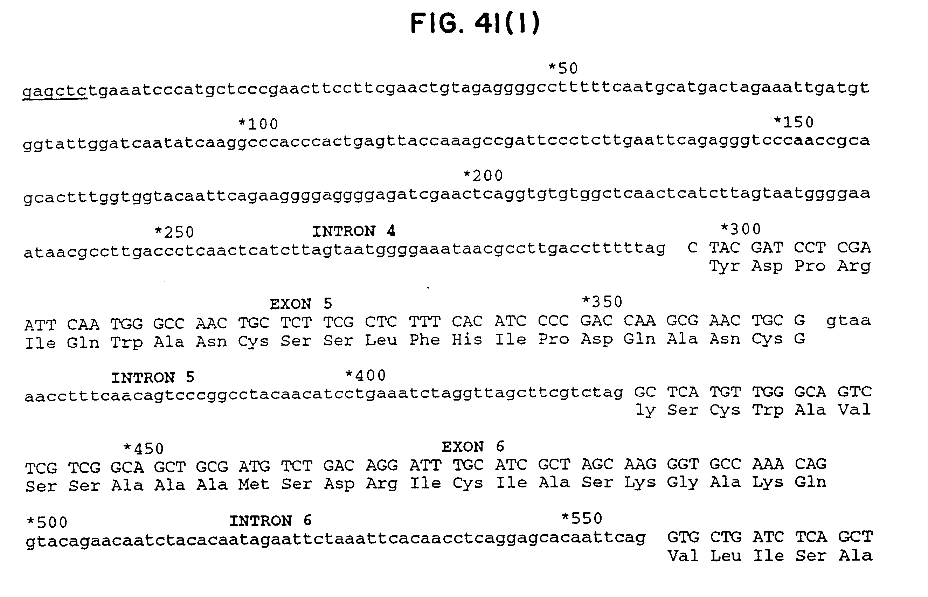

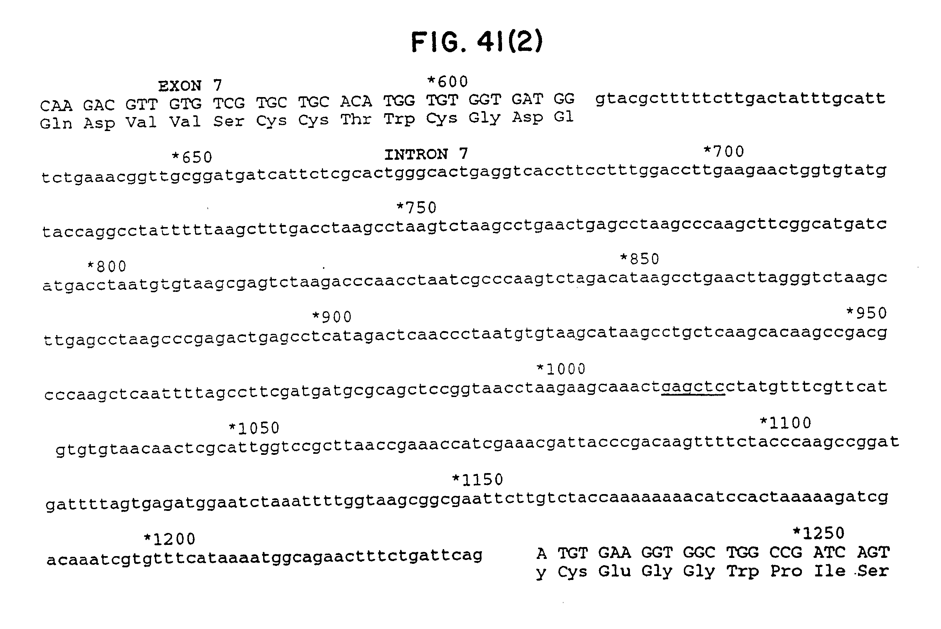

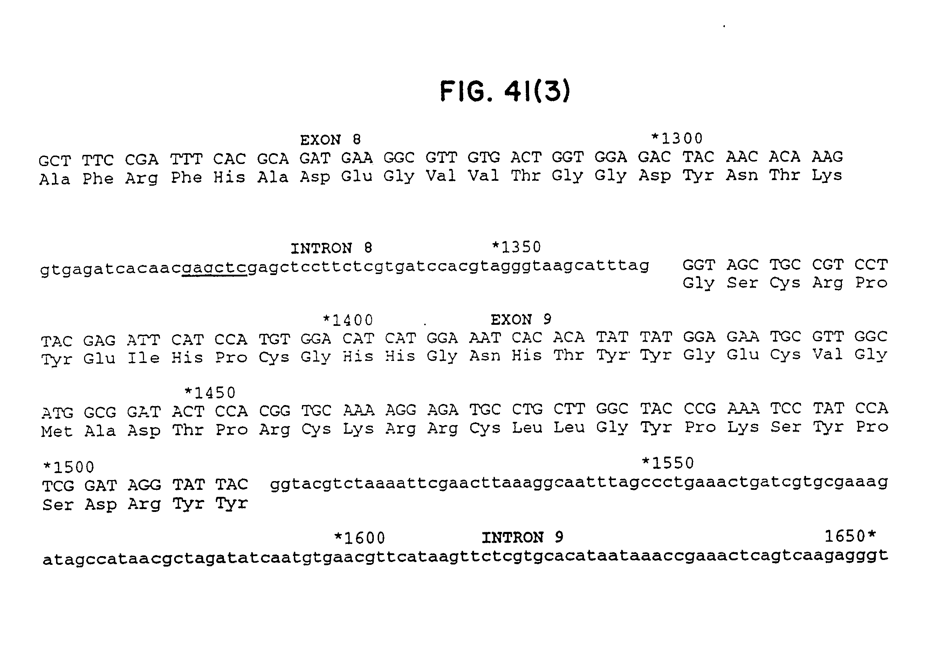

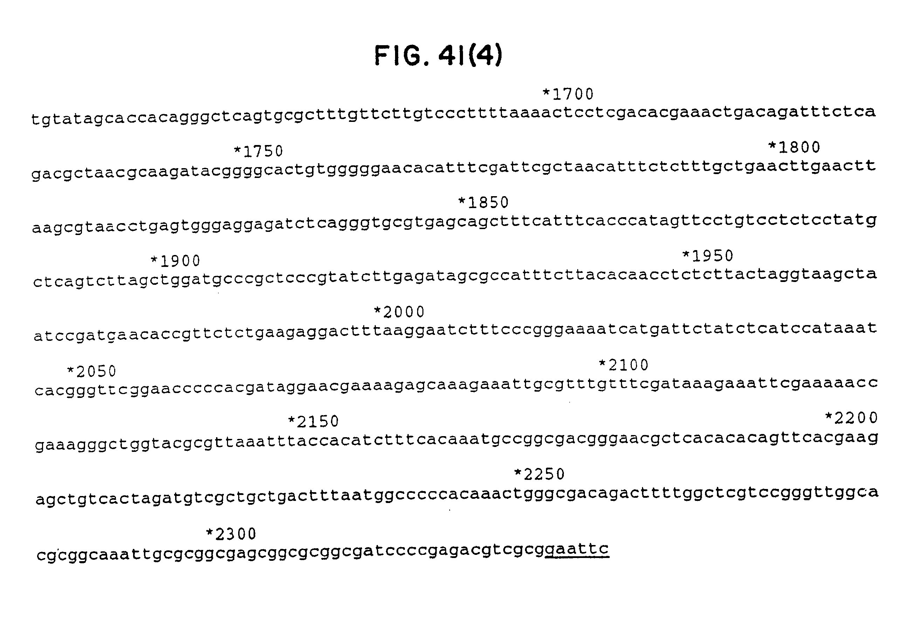

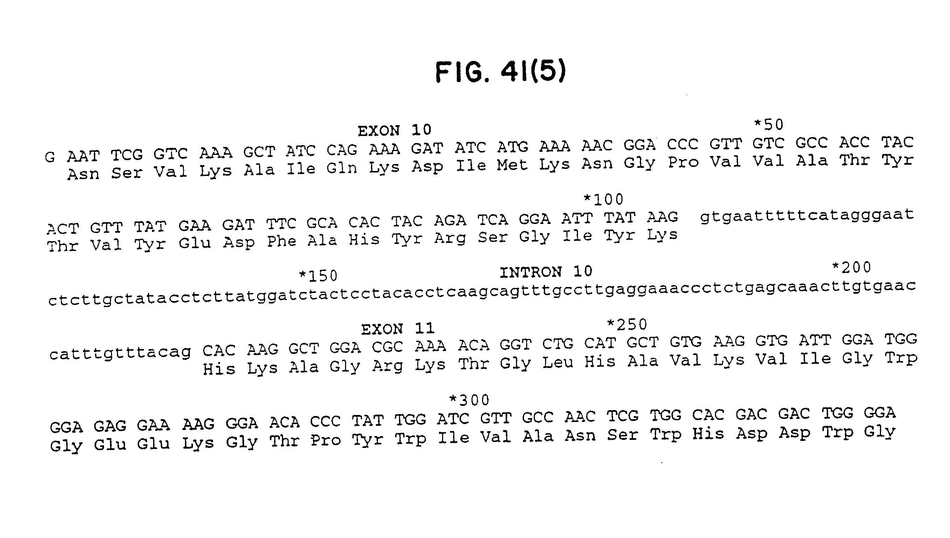

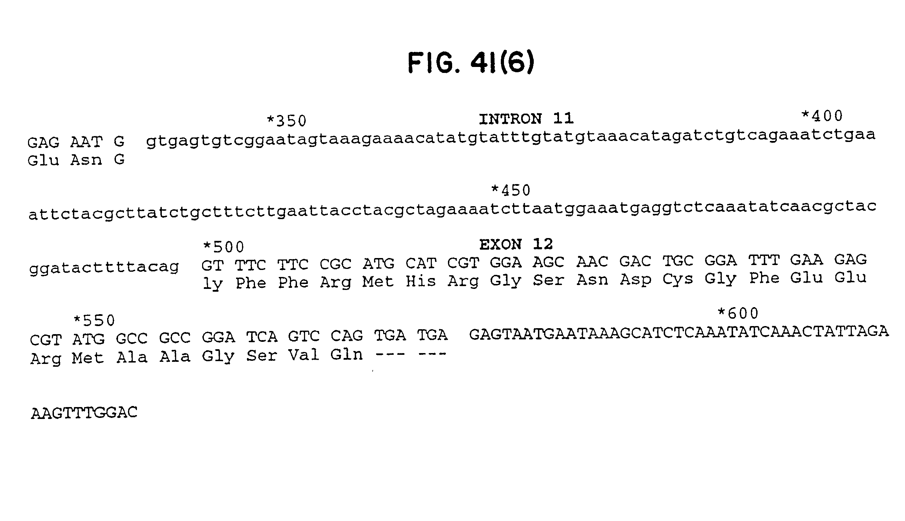

Figure 41 is the nucleotide and deduced amino acid sequence of the cysteine protease in λ007.

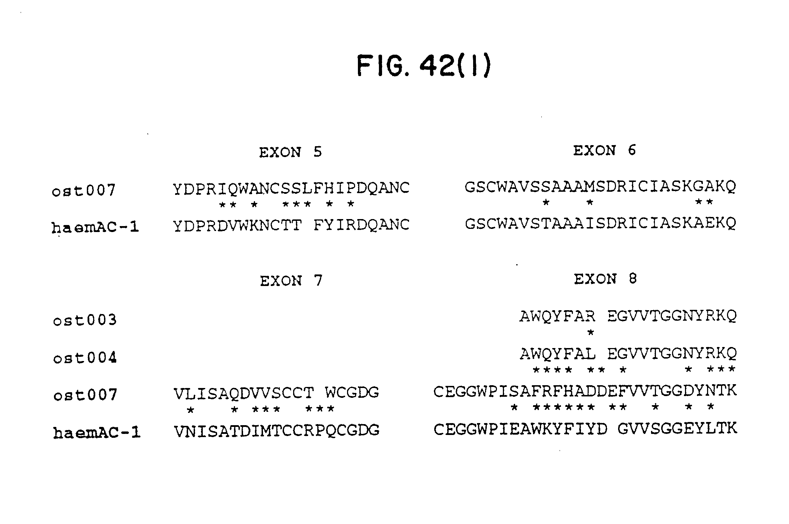

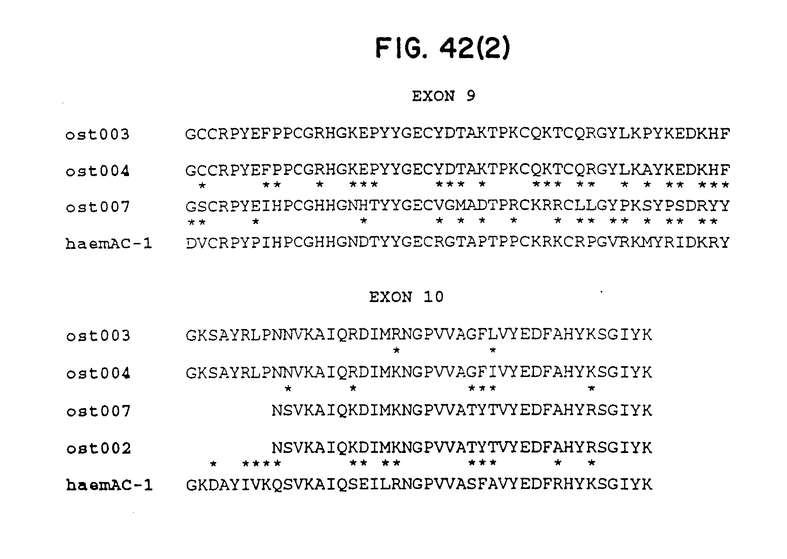

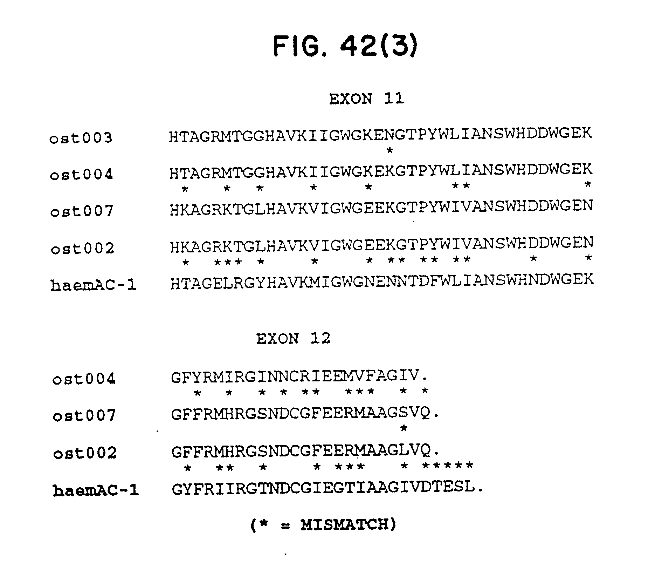

Figure 42 compares the sequences of Ostertagia cysteine protease to those of the Haemonchus AC-1 cysteine protease. Asterisks indicate amino acids that are different.

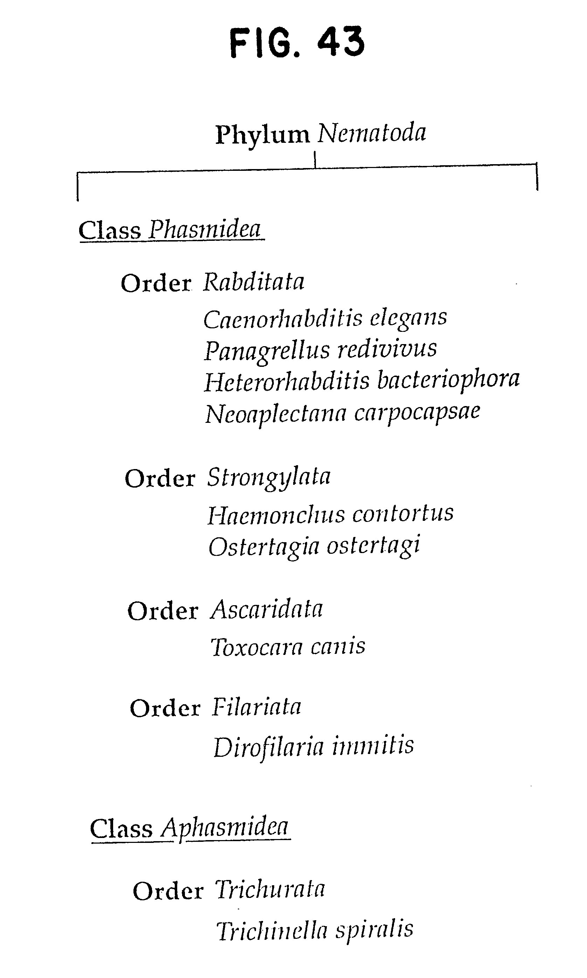

Figure 43. Phylogenetic relationship of nematodes examined for the presence of collagens that react with the anti-peptide antisera. The nematodes analyzed are listed according to their Class and Order designations, which were taken from Schmidt and Roberts (1981).

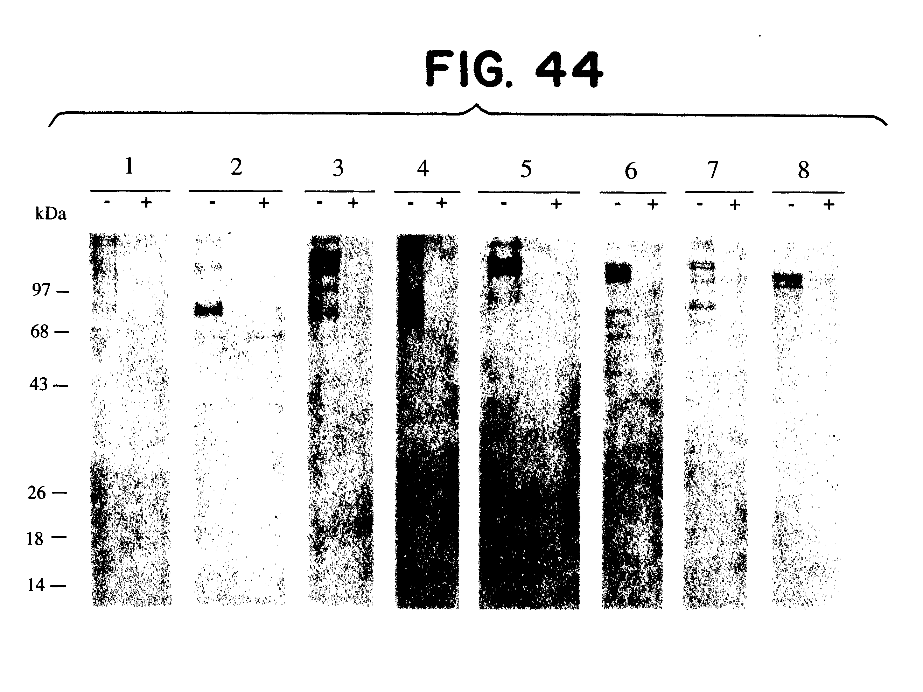

Figure 44. Western blot analysis of various nematodes for the presence of collagens that cross-react with the anti-peptide antisera. SDS + BME extracts of the various nematodes were precipitated with acetone, air-dried, resuspended in buffer (-lanes) or buffer + collagenase (+ lanes) and incubated overnight at 37°C. After dilution into SDS sample buffer, the extracts were electrophoresed on a 12% SDS gel, blotted onto nitrocellulose filters and incubated with immune Rb-9582 serum. A portion of each extract was analyzed on a separate SDS gel and stained for protein with Coomassie blue (gels not shown). Gel lanes correspond to: (1) C. elegans: (2) P. redivivus; (3) N. carpocapsae; (4) H. bacteriophora; (5) O. ostertagi; (6) T. canis; (7) D. immitis; and (8) T. spiralis.

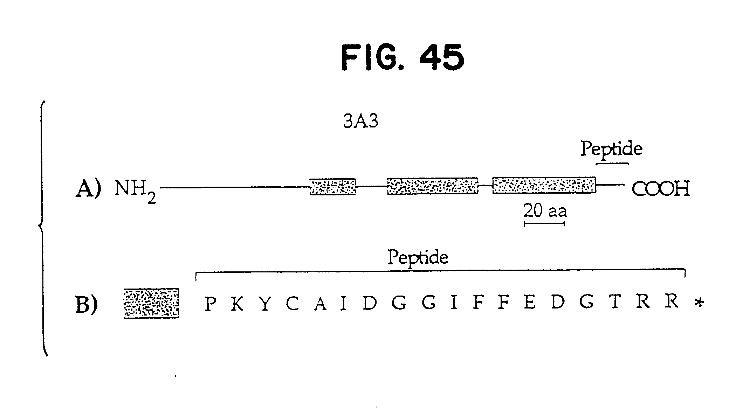

Figure 45. Location and amino acid sequence of the Haemonchus contortus collagen peptide immunogen. A schematic diagram depicting the domain organization of the predicted 3A3 collagen protein is shown in (A) and is taken from Shamansky et al. (1989). The boxed, stippled regions represent the presumed triple-helical domains in which glycine is every third amino acid. The straight horizontal lines represent non-triple-helical regions. The location of the peptide in the 3A3 protein is bracketed. The amino acid sequence of the peptide (bracketed region) is shown in (B). Amino acids are indicated by standard one-letter abbreviations. The boxed, stippled region represents the final triple helical domain of the protein and is shown for orientation purposes. The asterisk indicates the termination codon.

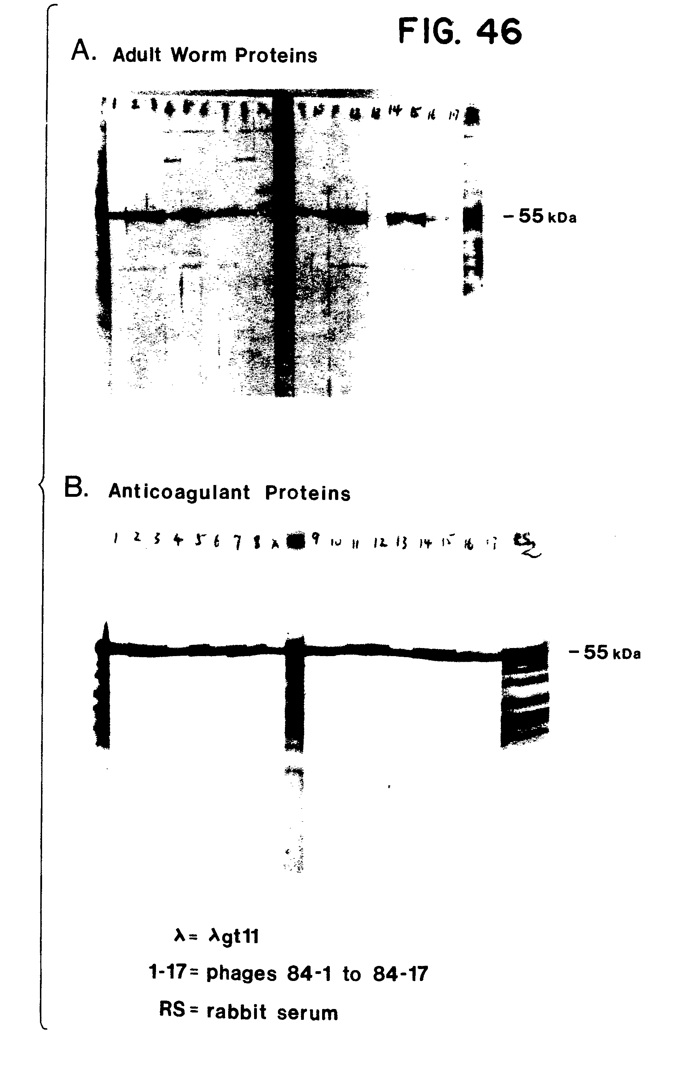

Figure 46 shows Western blots of H. contortus adult worm proteins (A) and purified anticoagulant proteins (B) reacted with antibodies selected by phage clones 84-1 to 84-17, which were isolated with Rb-10284 antiserum. The position of the 55 kDa band is indicated.

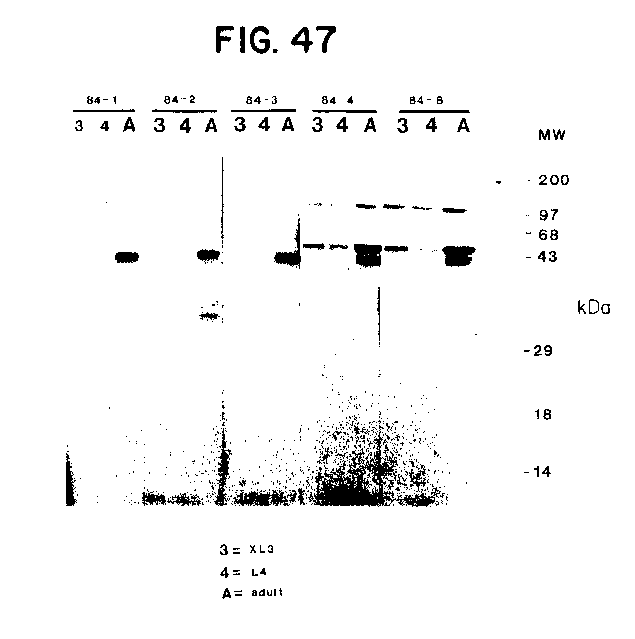

Figure 47 shows a developmental Western blot of H. contortus proteins reacted with antibodies selected by phages 84-1, 84-2, 84-3, 84-4 and 84-8 from the Rb-10284 antiserum. Developmental stages analyzed are XL3s (L3), L4s (L4) and adults (A). Positions of molecular weight markers are indicated.

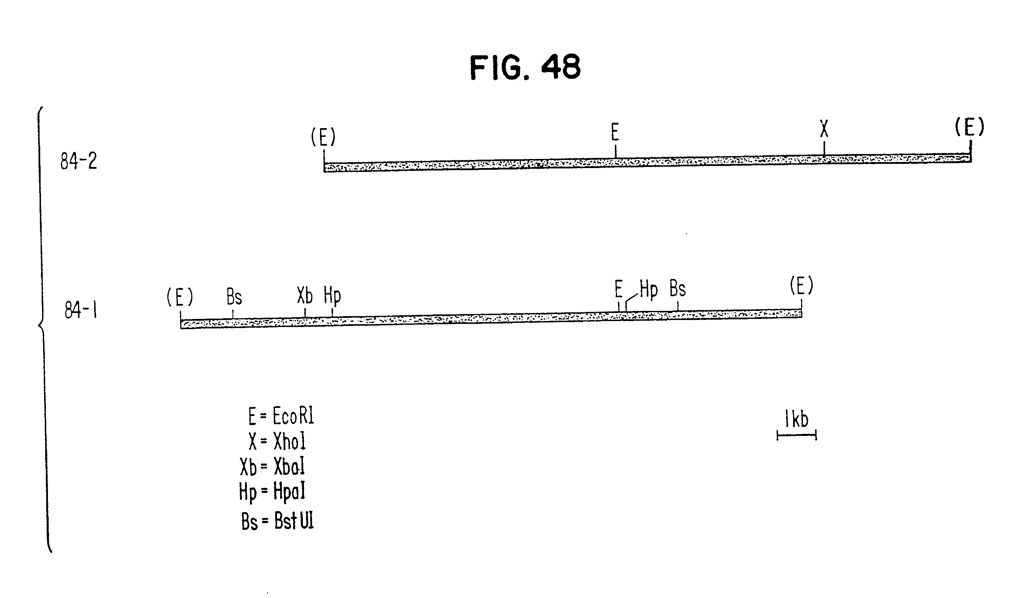

Figure 48 shows partial restriction enzyme maps of cDNAs 84-1 and 84-2.

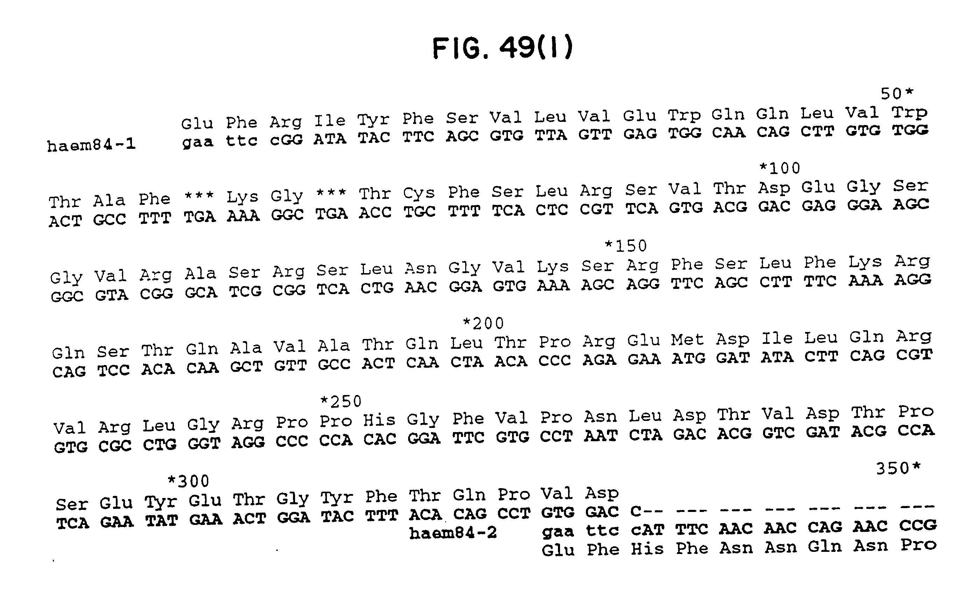

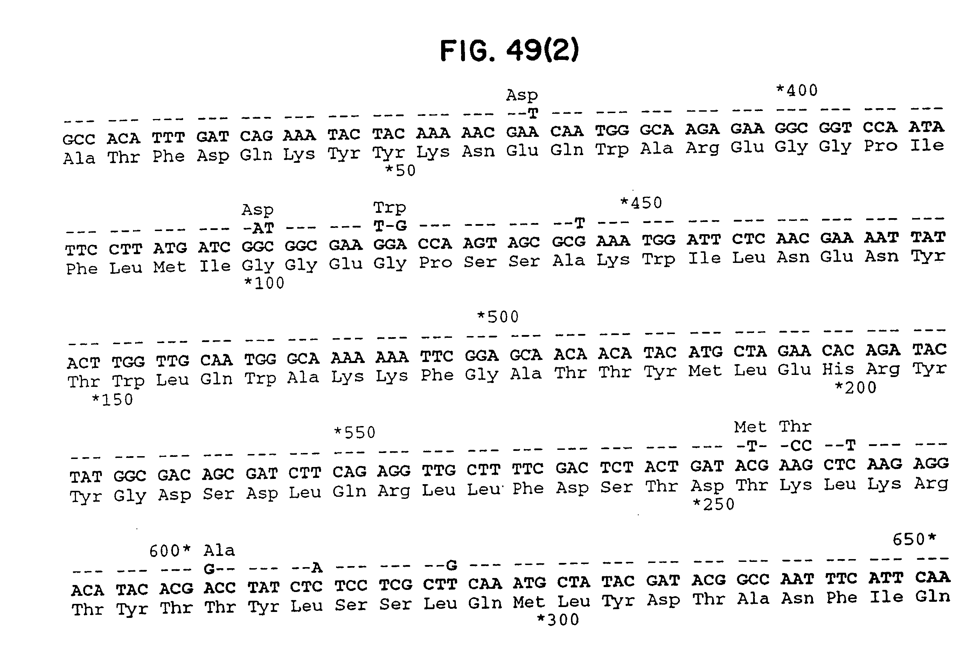

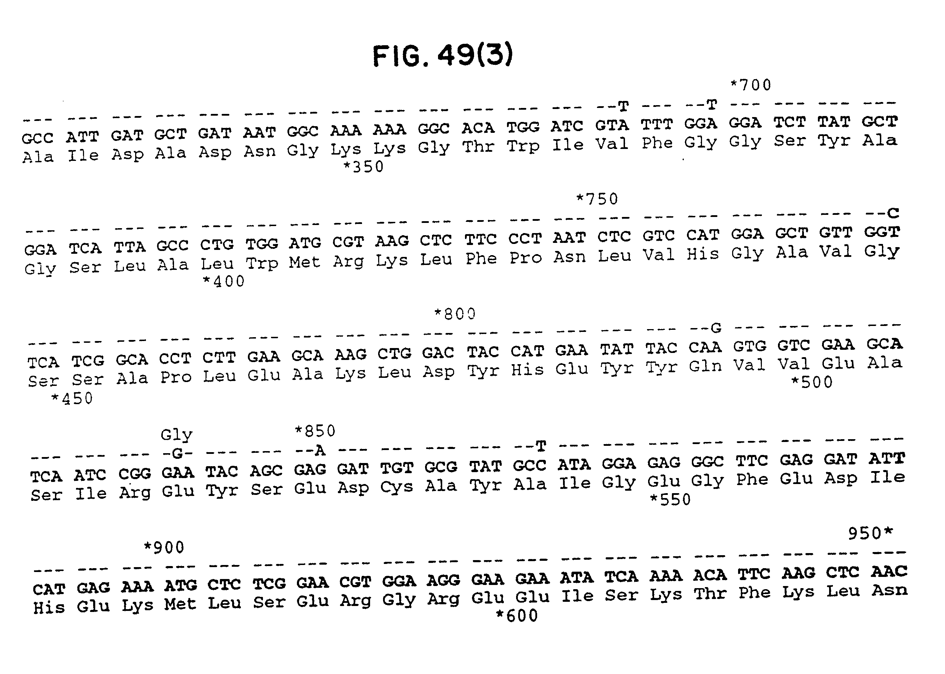

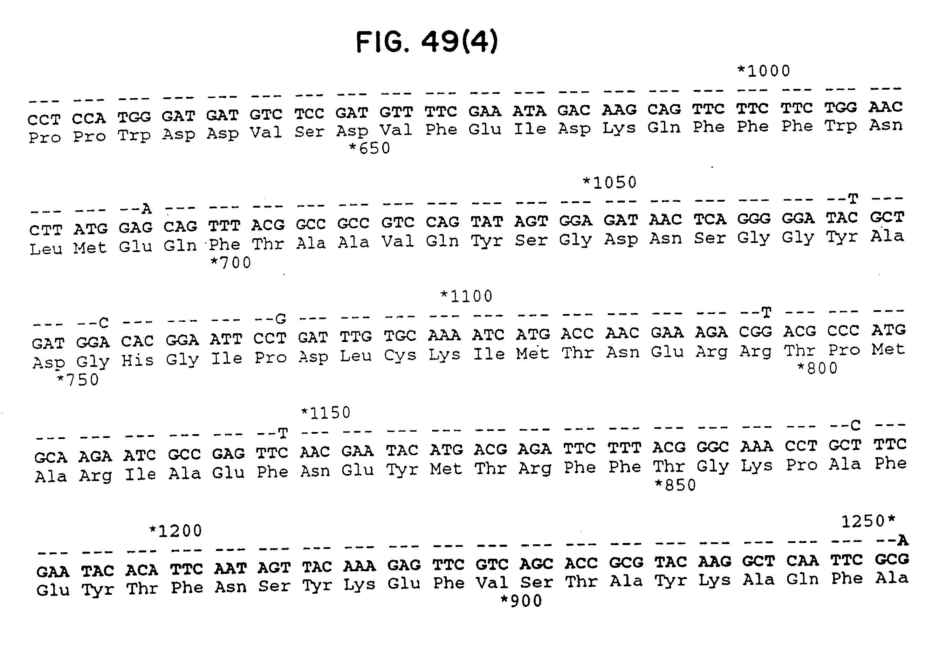

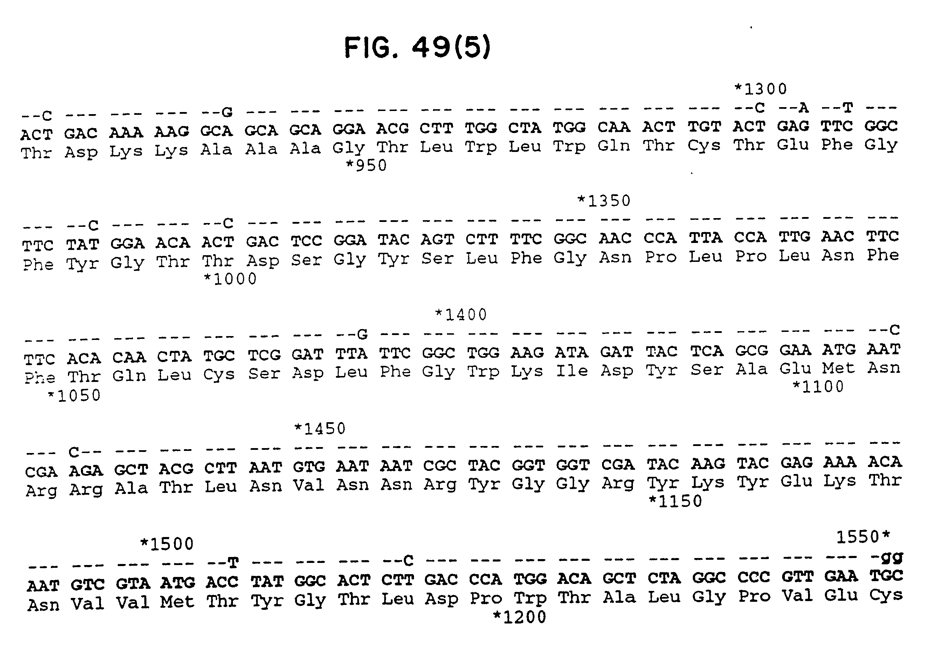

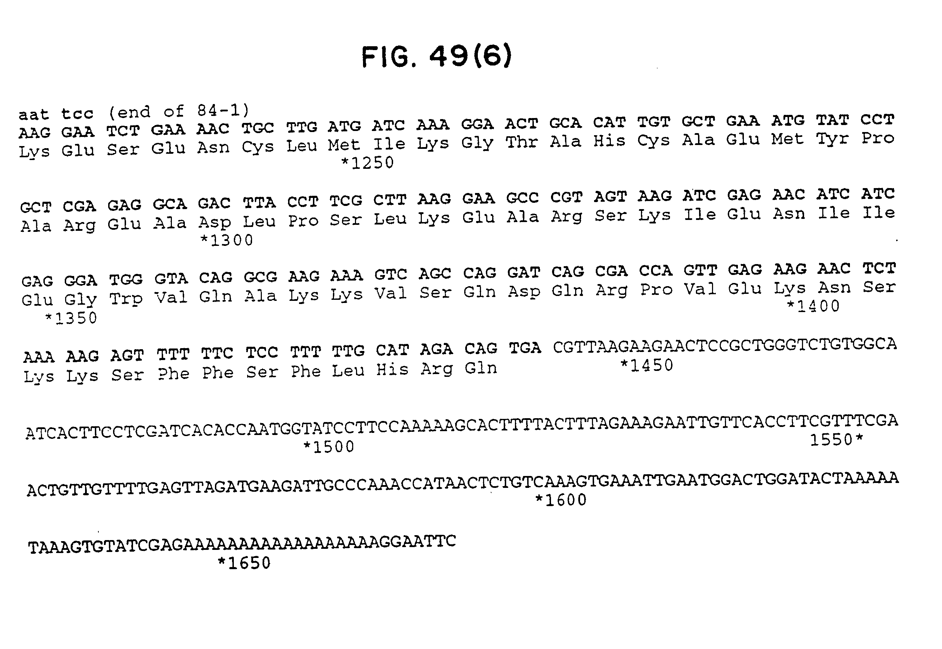

Figure 49 shows the complete nucleotide and deduced amino acid sequences of cDNAs 84-1 and 84-2. Dashes indicate nucleotides that are identical in cDNAs 84-1 and 84-2.

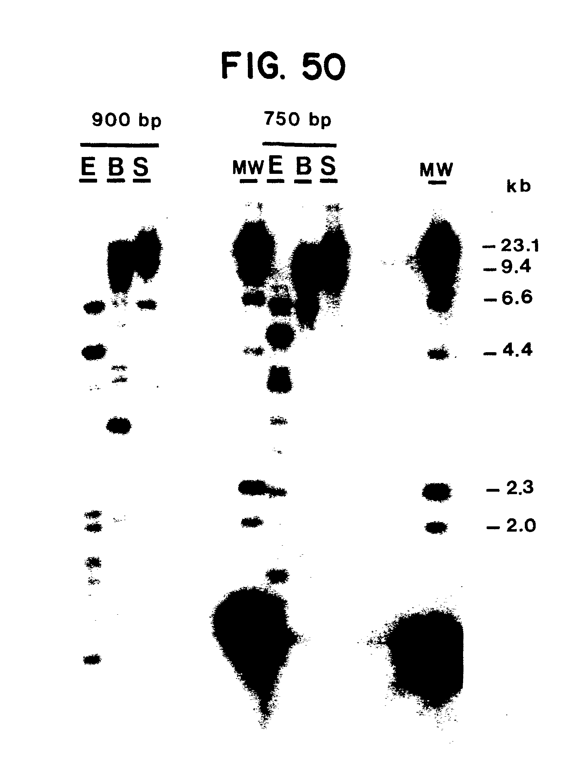

Figure 50 shows Southern blots of H. contortus DNA digested with EcoRI (E), BamHI (B) and SalI (S). Duplicate blots were hybridized with the 750 bp and 900 bp EcoRI fragments of cDNA 84-2. Positions of DNA size standards are indicated in kilobase pairs.

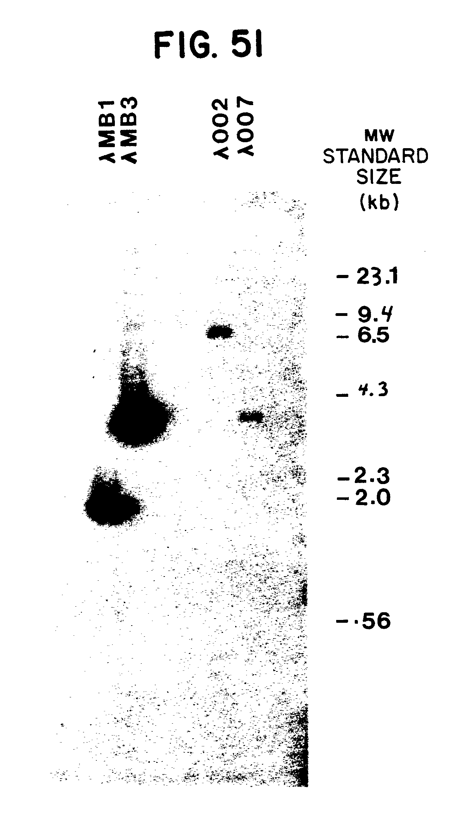

Figure 51 is an autoradiogram of a Southern blot of λMB1, λMB3, λ002 and λ007 DNAs that have been digested with restriction enzymes and hybridized to the F-1 exon 1-4 specific DNA probe using 30% formamide solutions at 32° C. λMB1 DNA was digested with SalI + EcoRI; other phage DNAs were digested with SalI + BamHI. The sizes of the hybridizing phage bands were λMB1 (1.7 kb), λMB3 (3.5 kb), λ002 (7.9 kb) and λ007 (3.6 kb). Positions of DNA size markers are indicated on the right.

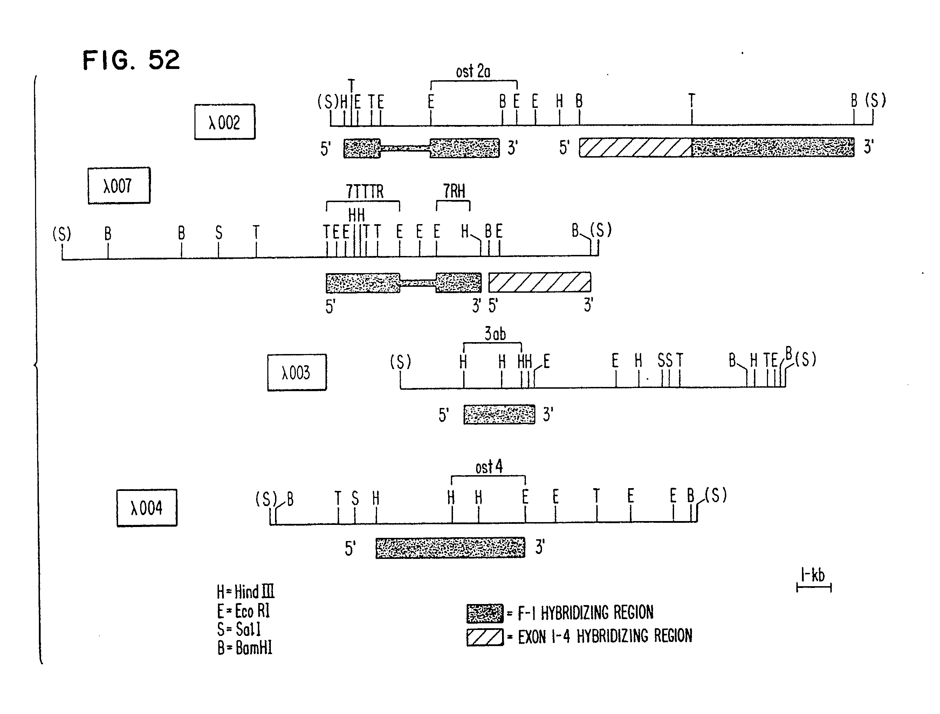

Figure 52 shows partial restriction enzyme maps of recombinant λEMBL-3 phages containing O. ostertagi cysteine protease genes. Regions of the phage DNAs that hybridize to plasmid pBR325::F-1 are indicated by black boxes. Regions of the phage DNAs that hybridize to plasmid pBR325::F1 exon 1-4 probe are indicated by striped boxes. Regions of the phage DNAs that were sequenced (sequences presented in Figures 38 to 41) are overlined. The 5' to 3' coding directions of the genes are indicated. Not all restriction enzyme sites in the phages are shown. Abbreviations are: S, SalI; B, BamHI; R, EcoRI; T, SstI; and H, HindIII. SalI sites in parentheses derive from the λEMBL-3 polylinker sequences.

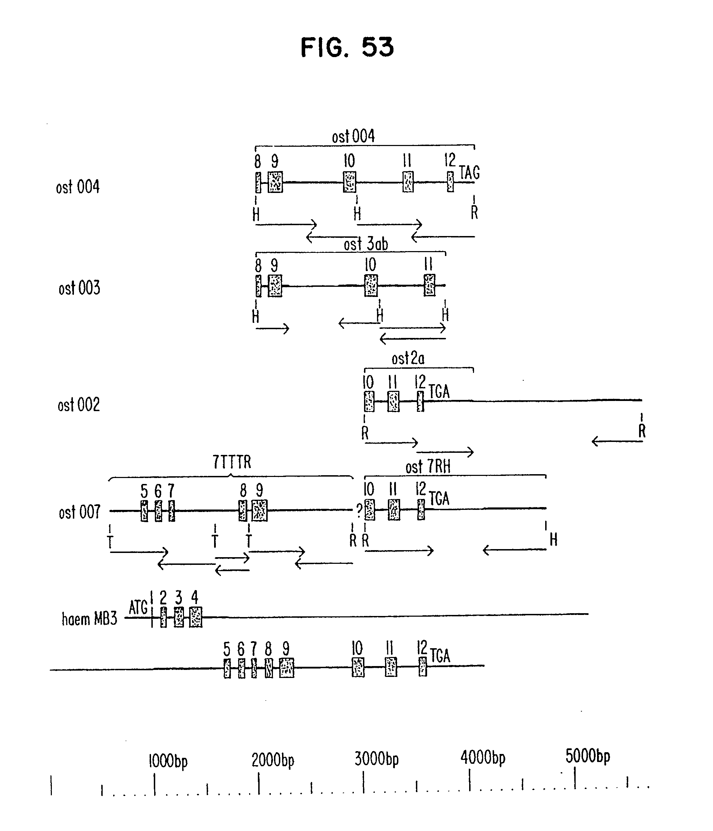

Figure 53 shows an expanded version of the regions of λ002, λ003, λ004 and λ007 that were sequenced. Location of exons are indicated by black boxes. Regions of the phage DNAs that were sequenced are indicated by arrows. Restriction enzyme sites are abbreviated as in Figure 52. λ007 contains 400 bp and 600 bp EcoRI fragments that have not been oriented or sequenced yet (their location is indicated by a question mark). One of these fragments contains DNA sequences encoding the missing amino acids of exon 10.

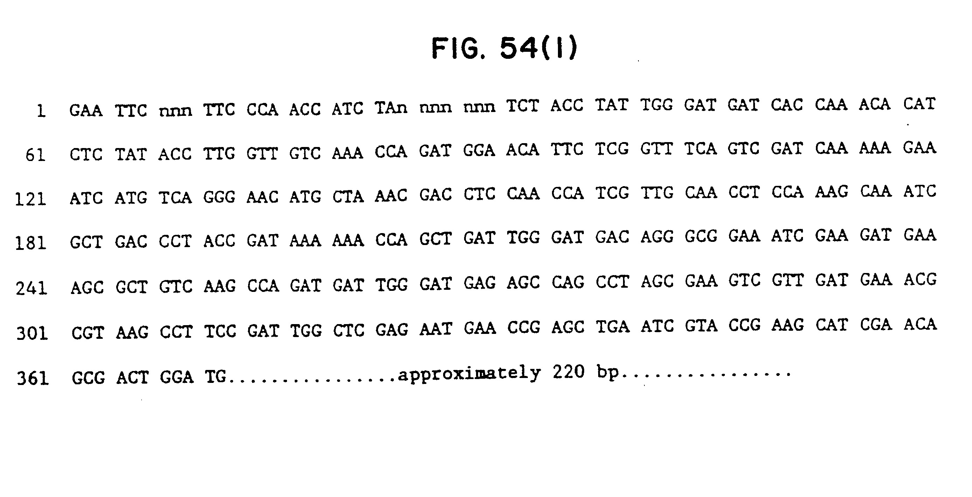

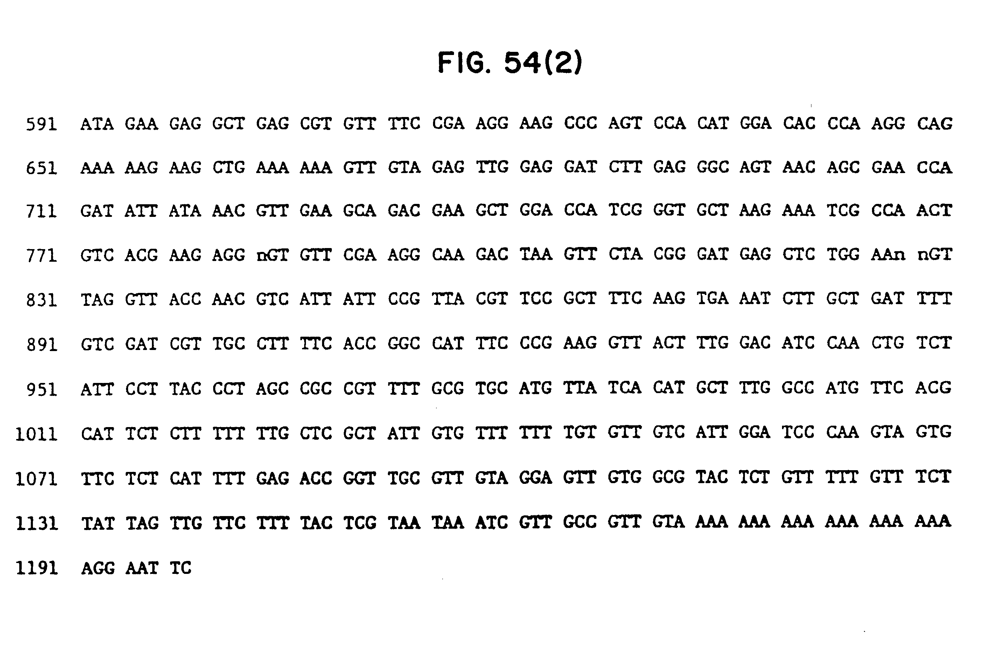

Figure 54 shows the partial nucleotide sequence of cDNA 84-4.

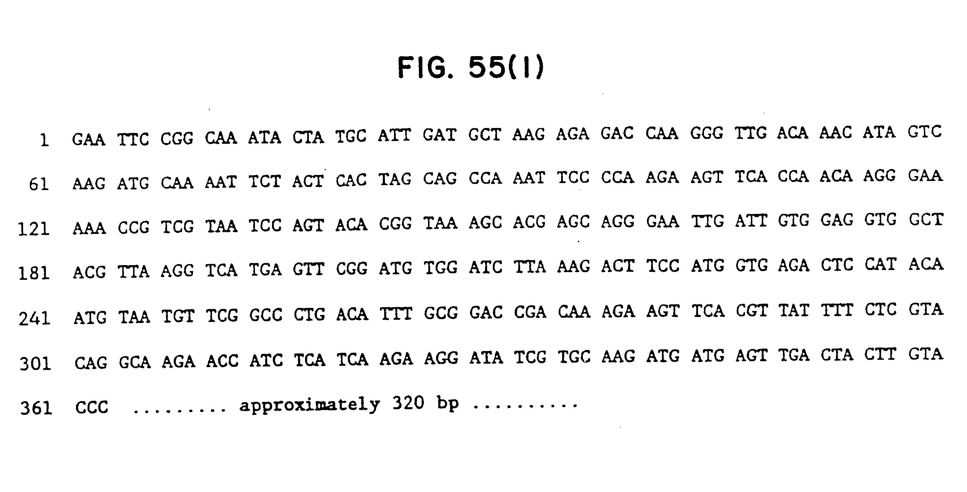

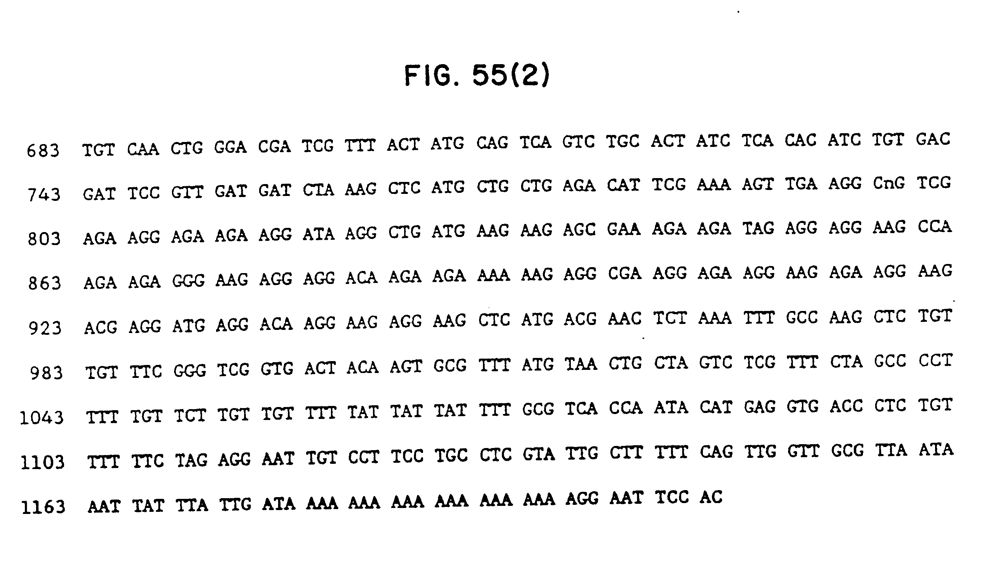

Figure 55 shows the partial nucleotide sequence of cDNA 84-8.

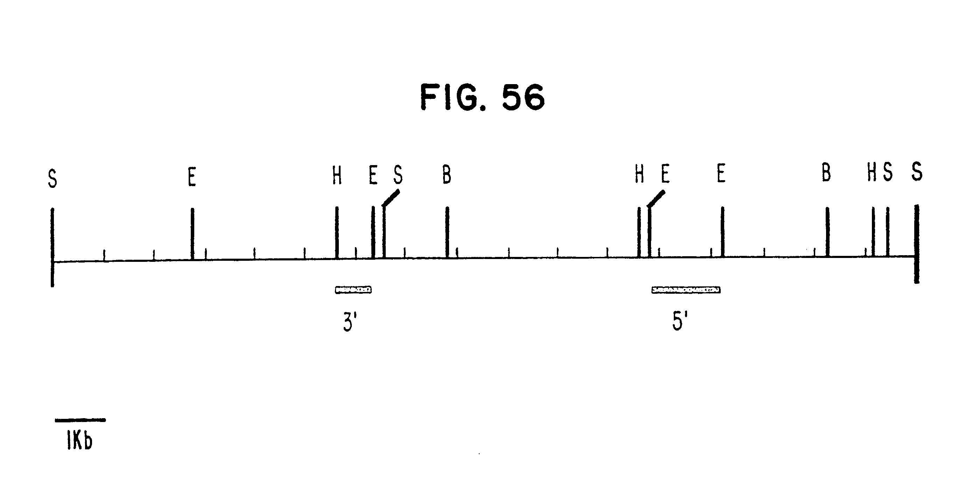

Figure 56 is a restriction enzyme map of the recombinant λEMBL3 phage 55A-11; which contains an O. ostertagi gene encoding a homologue of the H. contortus 55A protein. Regions of the phage DNA that hybridize to the 750 bp (5' region) and 900 bp (3' region) EcoRI fragments of H. contortus cDNA 84-2 are indicated. Restriction enzyme sites shown are EcoRI (E); SalI (S); HindIII (H); and BamHI (B).

## DETAILED DESCRIPTION OF THE PREFERRED EMBODIMENTS

Reference will now be made in detail to the presently preferred embodiments of the invention, which, together with the drawings and the following examples, serve to explain the principles of the invention.

The following proteins, in substantially pure form, have been discovered by the present inventors as immunogenic in sheep and useful as a vaccine to protect sheep from H. contortus infections:

Collagen peptide, cuticular proteins and an anticoagulant antiserum.

## Cuticular Proteins

The present invention provides methods of isolating cuticular, surface proteins, in relatively pure form, from helminthes. More specifically, the present invention provides methods of isolating surface proteins from two parasitic larval stages of Haemonchus contortus. Surface proteins purified by these procedures are immunogenic and induce antibodies that react with the native surface proteins on live worms.

The present inventors found that the cuticular proteins of live XL3s and L4s could be preferentially removed in relatively pure form. Live XL3s and L4s can be boiled briefly in a solution which specifically solubilizes surface proteins. To remove the surface proteins of the XL3s, it is preferred to use a solution of sodium dodecyl sulfate (SDS), and more preferably, a solution of 1% sodium dodecyl sulfate. Boiling live XL3s briefly as described in detail below in 1% SDS specifically solubilized surface proteins from this developmental state (Figure 1). This procedure should also be applicable for purifying cuticular proteins of other nematodes, particularly from those developmental stages such as the H. contortus XL3 that have a mouth and anus plugged with cuticle.

Briefly boiling in SDS was found to be not as advantageous for purifying the surface proteins of the L4s, as this procedure will solubilize both body proteins and cuticular proteins. This is most likely due to the fact that the mouth and anus of this developmental stage are open to the environment. The ability to purify XL3 surface proteins by briefly boiling them in SDS is probably due, at least in part, to the fact that both the mouth and anus of the XL3 are plugged with cuticle and closed to the environment. Thus, upon boiling, XL3 surface proteins are solubilized where as cellular proteins remained trapped within the worm.

It is also a preferred embodiment of the invention to briefly boil the XL3 and L4 in a saline solution, more preferably 100 M NaCl. In this procedure, live XL3s or L4s may be suspended in a saline solution and then boiled briefly in order to solubilize the surface proteins. Although surface proteins obtained from XL3s by the NaCl procedure are quite pure, as would be expected from the SDS results, the surface proteins extracted from L4s are greatly enriched but are contaminated to a small degree by cuticle collagens and cellular proteins.

Contamination can be detected by probing Western blots of L4 surface protein extracts with antisera prepared against a synthetic 18 amino acid long peptide derived from the sequence of a cuticle collagen gene. The NaCl extraction procedure does greatly enrich for L4 surface proteins, though, and it is possible to purify the proteins further by simple procedures such elution from SDS gels.

In a particularly preferred embodiment of the invention, live XL3s or L4s were suspended in 1 ml of 100mM NaCl, 10 mM tris-HCl pH 7.4, placed in boiling water for 2 minutes, removed from the water, and mixed by inversion for an additional 2 minutes, and then pelleted for 1 minute in a microfuge. The supernatant was drawn off, recentrifuged several times to remove all worms, frozen in a dry ice/ethanol bath and stored at -20°C. These samples were later thawed and concentrated at 4°C using a 2 ml centricon apparatus (Amicon).

Injection into sheep of worm cuticular proteins purified by this process will induce the production of specific protective antibodies in sheep.

Shorter time periods of boiling and exposure to salt solution generally produces purer L4 surface proteins preparations. Longer exposure resulted in more cellular protein contamination. For example, overnight exposure of XL3s to SDS solubilized considerable amounts of cellular proteins.

The protein extraction studies indicate that the XL3 has a single, major surface protein of 68-90 kDa, which probably covers the main portion of the XL3 surface. Other proteins of various molecular weights were also identified. The 180 kDa protein appears to be the next most abundant XL3 surface protein. Preliminary experiments indicate that a rabbit antiserum prepared against the 68-97 kDa protein electroeluted from SDS gels immunoprecipitates the $^{125}$I-labeled 180 kDa protein and reacts with the 180 kDa protein on Western blots of surface protein extracts. These preliminary results suggest that the 68-97 kDa and 180 kDa proteins are antigenically related and possible are different forms (e.g., aggregates of the same proteins). In the L4 stage, the predominant species of proteins have molecular weights of 27, 29, 75 and 200 kDa.

Another method according to certain preferred embodiments includes surface-labeling of cuticle proteins with $^{125}$I and chloramine T. Two classes of proteins were identified by this method. The properties of these proteins appear to be similar in XL3s and L4s. One class of proteins comprises those that can be extracted from the cuticle with SDS. The other class comprises those proteins that require a disulfide reducing agent for efficient solubilization. The SDS-soluble surface proteins of XL3s and L4s include relatively few major species. The pattern of labeled proteins is distinct for each developmental stage, although some of the proteins have similar molecular weights. The proteins are nearly completely extracted from worms with SDS, in the absence of a disulfide reducing agent.

The SDS-soluble surface proteins are not digestible by bacterial collagenase, indicating that they lack the repeating (Gly-X-Y)n structure characteristic of collagen, which is a major protein component of nematode cuticle. Several of the proteins are glycosylated, supporting the notion that they are extracellular, and probably located on the cuticular surface. Bone and Bottjer previously reported the binding of specific lectins to the surface of H. contortus adults and juveniles. In the above properties, the major SDS-soluble surface proteins of H. contortus resemble surface proteins described for other nematodes.

The strongest evidence that some or all of these proteins derive from the worm's surface comes from the IFA experiments using rabbit antisera prepared against SDS-extracted XL3 and L4 cuticles. The failure of these antisera to react with live worms suggests that the SDS extractions had efficiently removed the major surface proteins from XL3 and L4 cuticles. Definitive proof that these proteins are located on the cuticular surface must await the development of monospecific antisera. Since nematode cuticles are known to contain covalently cross-linked proteins, e.g., collagens, it is possible that some of the $^{125}$I-labeled proteins species describes here also are cross-linked aggregates rather than primary gene products.

In contrast to the SDS-soluble proteins, most of the SDS + BME extractable proteins that are labeled with $^{125}$I are digestible with collagenase, indicating that they are collagens. Certain of these proteins, however, were not digested by collagenase, and thus, are probably not collagens. The inventors predict that the labeling of these proteins is artifactual and that they derive from the internal portions of the cuticle and not from the cuticular surface. This prediction stems from the observations that antisera prepared against SDS-treated cuticles (which contain collagens) react weakly or not at all with live worms, suggesting that SDS has removed the major surface antigens. However, we cannot rule out the possibility that SDS-treatment of cuticles altered the conformation of the remaining, insoluble cuticle proteins so that they were able to induce the formation of antibodies capable of reacting with native proteins on the surface of live worms. Further studies will be required to determine if collagens are truly exposed on the cuticular surface.

SDS-soluble XL3 surface proteins of 24 kDa and 36 kDa appear to be less tightly associated with the cuticle than other XL3 surface proteins because they are partially released from the cuticle by sonication, in the absence of detergents. The physiological role, if any, of this difference in affinity is unclear at present. Other researchers have reported that specific surface proteins of parasitic nematodes are shed into the media during in vitro incubation studies with live worms (9, 20). We have not determined if this is the case for any H. contortus surface proteins.

The different patterns of surface proteins revealed by the $^{125}$I-labeled studies suggest that there are differences in the antigens exposed on the surfaces of XL3 and L4s. The inventors confirmed this finding using an antiserum prepared against purified XL3 surface proteins. The adult cuticle probably contains an antigenically distinct set of surface proteins because antisera raised to native adult cuticles failed to react with live XL3s or L4s in IFA experiments. Similarly, neither the anti-native XL3/L4 cuticle sera nor the anti-adult cuticle sera reacted with live SL3s, suggesting that cuticular surface proteins of this developmental stage are unique as well.

Stage-specific surface antigens have been described for several parasitic nematodes (9, 10). The ability of parasitic nematodes to change surface proteins at molts during which the old cuticle is shed and a new cuticle is formed, may be a mechanism for evading the host's immune responses and may explain in part why primary infections with H. contortus proceed to the adult stage, whereas secondary infections in immune animals generally are halted at the XL3 or L4 stages.

The amino acid compositions of XL3 and L4 protein extracts were determined and are given in Table I. Both sets of proteins are enriched for hydrophillic amino acids, and with the exception of alanine and glycine, are relatively poor in hydrophic amino acids. The major 68-97 kDa protein appears to be greatly enriched in glutamic acid and/or glutamine residues. In fact, 26 percent of the amino acids in the XL3 surface protein extract, which should largely reflect the amino acid composition of the major 60-90 kd species, were either glutamic acid or glutamine. This protein is also enriched for aspartic acid and/or asparagine residues. Together, these four amino acids account for nearly 40% of the total amino acids detected in the XL3 surface protein extract. Since no comparable analyses have been reported for surface proteins of other nematodes, the inventors do not know how common or unusual this finding will prove to be.

The L4 surface proteins, which are a more heterogeneous mixture of proteins, are not as enriched for these amino acids. The difference in relative abundance of XL3 surface proteins detected by Coomassie blue staining and $^{125}$I-label may reflect the inefficient labeling of the 68-97 kDa protein by $^{125}$I and chloramine T, which labels predominantly tyrosine residues. Tyrosine is poorly represented in the XL3 surface protein extract and, hence, in the 68-97 kDa protein. The other SDS-soluble XL3 proteins identified as surface proteins by $^{125}$I-labeling studies (i.e., the 24, 26, 30 and 36 kDa species) probably are minor components of the XL3 surface.

The cuticular surface of certain developmental stages of a number of nematodes has been shown to possess a net negative charge by virtue of the fact that the cuticular surface binds cationized ferritin particles (Himmelhoch et al.), Exp. Parasitology 41: 118-123 (1977); Murrell et al., Exp. Parasitology 55: 331-339 (1983); Abraham et al., Vet. Parasitilogy 13: 341-347 (1988). Although the present inventors have not performed similar experiments with Haemonchus contortus XL3 larvae, the amino acid composition determined for XL3 surface proteins would be considered with a net negative charge on the XL3 surface.

The IFA and immuneprecipitation experiments with the anti-XL3 surface protein serum provide further evidence that the antigens exposed on the surfaces of XL3s and L4s are immunologically different. The failure of the anti-XL3 surface protein serum to precipitate the major 27 and 29 kDa L4 surface proteins suggests that these L4 proteins are antigenically distinct from the major 68-97 kDa XL3 surface protein. The reactions of anti-L4 surface protein sera with live XL3s in IFA experiments and with $^{125}$I-labeled XL3 surface proteins in immunoprecipitation experiments probably is due to contamination of L4 surface proteins with small amounts of XL3 surface proteins. The L4s used for this study were obtained by cultivating XL3s for several days. Because development in vitro is not entirely synchronous and because large worm populations were required for surface protein isolations, the inventors were never able to obtain L4 populations that were completely free of all XL3s and of shed XL3 cuticles that had been discarded as XL3s molted into L4s. Contamination of L4s with XL3s and free XL3 cuticles was estimated to be 5-10%.

In contrast, the XL3 populations analyzed were obtained by exsheathing SL3s in vitro with $CO_2$ and contained no L4s. Therefore, the inventors believe that the stage-specificity observed with the anti-XL3 surface protein serum is a more accurate reflection of the antigenic relatedness of XL3 and L4 surface proteins. However, the inventors cannot rule out the possibility that the anti-L4 surface protein sera recognize epitopes that are conserved between XL3 and L4 surface proteins and which are not recognized by the anti-XL3 surface protein serum. Monospecific antisera and monoclonal antibodies to individual XL3 and L4 surface proteins will better clarify the antigenic relationships between surface proteins and will allow precise localisation of these proteins on the XL3 and L4 surface.

## Anticoagulant Antiserum

In another aspect of the present invention, the inventors identified the anticoagulant activity in Haemonchus contortus as a fibrinogen degrading enzyme, fibrinogenase. The fibrinogenase has been characterized as having a native molecular weight of greater than 1,000,000 by means of gel filtration chromatography on an S12 sizing column, where it elutes at the void volume of the column. Standard proteins indicated that the molecular weight corresponding to the void volume is on the order of 1 million.

Antibodies to the anticoagulant raised in rabbits were used to probe Western blots of extracts of Haemonchus contortus as well as the purified fractions. The antibodies were found to react specifically with the 35 and 55 kD bands. The antibody was further used to inhibit the activity of the enzyme. This was accomplished by incubating the antibody preparation with the anticoagulant prior to incubation with fibrinogen.

The anti-coagulase activity from adult worms has been previously determined to be a specific fibrinogen cleavage activity. As shown in Fig. 1, the alpha and beta bands of bovine fibrinogen are degraded when incubated with increasing amounts of a partially purified enzyme preparation. Analysis of the enzyme preparation on SDS-PAGE and subsequent staining with Coomassie blue demonstrate two major bands of approximately 35 and 55 kDa. When the anti-coagulase is similarly electrophoresed and stained with the more sensitive silver staining method, a number of additional polypeptides are visualized (Figure 2). Some of the higher molecular weight minor bands are believed to represent collagen polypeptides. Antiserum towards the anticoagulant preparation raised in rabbits, as well as in sheep during protection experiments, react with collagens.

Evidence that the 35 kDa polypeptide is the catalytic subunit is provided from active site labeling experiments. The fibrinogenase activity is thiol dependent and the use of appropriate inhibitors and thiol label reagents indicates that the 35 kDa polypeptide contains an active thiol. Evidence that the fibrinogenase activity may be associated with other polypeptides or is a single polypeptide aggregate is provided from native molecular weight sizing columns showing the activity to elute at a molecular weight of at least one million. Attempts to disaggregate this complex and maintain activity were unsuccessful.

The anticoagulant material is immunogenic in sheep, and can be used as a vaccine to protect sheep from Haemonchus contortus infections.

The following examples further illustrate preferred embodiments of the present invention. The examples should in no way be considered limiting, but are merely illustrative of the various features of the present invention.

## I. H. contortus Surface Protein Examples

### A. Identification of XL3 and L4 Surface Proteins

#### 1. Source of H. contortus larvae and adults

H. contortus adults and SL3 (ensheathed third-stage), XL3 (exsheathed third-stage) and L4 (fourth-stage) larvae were obtained from Dr. R.J. Boisvenue of Eli Lilly and Company (Greenfield, IN). XL3s were obtained by exsheathing SL3s in vitro with 100% $CO_2$ for 3 minutes and allowing the XL3s to crawl through a muslin filter ring suspended in physiological saline for a minimum of 8 h. To obtain L4 larvae, XL3s were washed several times with EBSS/MES medium plus antimicrobial agents, amphotericin B, penicillin and streptomycin, and centrifuged gently at 700 rpm for 5 minutes between washings.

Then the larval pellet was added to 300 ml of the medium plus antimicrobials in a 2.7l Corning plastic disposable roller bottle to provide a concentration of 12,000 XL3 per ml. The medium was gassed with 40% $CO_2$/60% air for 5 minutes to achieve a final pH of 6.2 and the bottle was capped under sterile conditions. The culture bottle was placed on a rolling mill housed in an incubator set at 39°C with a velocity of 1 rpm/1.5 minutes for a minimum of 72 h. Following gentle centrifugation for 5 minutes, an L4 pellet was collected after aspirating the supernatant. In most cultures, more than 90% exsheathment of SL3s occurred and more than 85% of the XL3s developed to the L4 stage.

Adult H. contortus worms provided by Dr. Boisvenue were obtained from the abomasum of monospecifically infected sheep inoculated intraruminally with 25,000 XL3 per animal. Approximately 30 days following worm challenge, the donor animals were euthanized by electrocution and the predominantly adult worm population was individually collected from the saline washed abomasal contents and frozen in liquid nitrogen.

2. Separation of Worm Stages

a. XL3s

XL3s that had passed through a muslin filter ring were separated from free cuticles released during the exsheathment process by suspending them in a small volume of saline, layering them over a 2 ml cushion of ice-cold 15% Ficoll (Sigma) and centrifuging them for 5 minutes at 300 g. The pellet, which contained live XL3s, was washed several times with saline, then subjected to a second 15% Ficoll step-gradient as described above. The XL3 pellet was washed several times with saline before being used in other procedures.

b. L4s

Cultures containing L4s, XL3s, and free cuticles from XL3s that had molted were centrifuged, the pellets washed with saline, layered over a 2 ml cushion of 15% Ficoll, and centrifuged for 5 minutes at 300g. Material at the Ficoll/saline interface (predominantly L4s and free cuticles) was washed with saline, layered over a 2 ml cushion of 10% Ficoll and centrifuged for 5 minutes at 300g. The L4 pellet was washed several times with saline before being used in other procedures. In most cases it was necessary to subject worms to several 10% Ficoll step-gradients to remove all free cuticles.

3. $^{125}$I-Surface Labeling of Live Worms

Surface proteins of XL3s and L4s were identified by labeling live worms with $^{125}$I using the chloramine-T procedure as described below, which labels mostly tyrosine residues. For these experiments, worms were washed several times with saline, once with PBS (10mM sodium phosphate, pH 7.4, 0.145 M NaCl) and resuspended in 1 ml of PBS. To this mixture was added 0.3 mCi of $^{125}$I (New England Nuclear) and 10 microliters of chloramine T (1 mg/ml in water). After a 2 minute incubation at room temperature, an additional 10 microliters of chloramine T solution was added. After 2 minutes, the labeling reaction was quenched with a drop of tyrosine- saturated water. Worms were washed several times with saline to remove unbound label.

Labeled worms were sonicated on ice in 1 to 3 ml of sonication buffer (10 mM Tris-HCl pH 7.4, 1 mM EDTA, 1 mM phenylmethylsulfonyl fluoride) and particulate material collected by centrifugation in a clinical centrifuge. The supernatant (called sonication supernatant) was stored at -20. The pellet was boiled for 2 minutes in 0.5 ml of ST buffer (1% SDS, 0.125 M Tris-HCl pH 6.8) and shaken overnight at room temperature. After pelleting for 2 minutes in a microfuge, the supernatant (called SDS supernatant) was stored at -20. The pellet was boiled for 2 minutes in 0.5 ml of ST buffer, 5% 2-mercaptoethanol (BME) and shaken overnight at room temperature. After centrifugation for 2 minutes in a microfuge, the supernatant (called BME supernatant) was drawn off and stored at -20. Insoluble cuticle material was dissolved with Protosol (New England Nuclear) for radioactivity determinations. In some experiments the sonication step was omitted and the labeled worms were extracted directly with SDS.

Labeled proteins released from the worms by the above procedures were analyzed by SDS-PAGE (SDS polyacrylamide gel electrophoresis), followed by autoradiography. SDS-PAGE was performed using the buffer systems described by Laemmli and Favre (J. Mol. Biol. 80:575-599, 1973). For autoradiography, gels were fixed overnight with 50% methanol/10% acetic acid, washed for 60 minutes with 10% glycerol/10% ethanol, dried under vacuum and exposed to X-ray film. Relative molecular weights were determined using $^{14}$C-labelled proteins purchased from Bethesda Research Laboratories as standards: myosin (200 kDa), phosphorylase B (97.4 kDa), bovine serum albumin (68 kDa), ovalbumin (43 kDa), alpha-chymotrypsinogen (25.7 kDa), -lactoglobulin (18 kDa), and lysozyme (14.3 kDa).

Figure 1 shows typical results for the surface labeling experiments. With XL3s, six major proteins are consistently labeled with $^{125}$I and solubilized from worms or cuticles by boiling in SDS. The most heavily labeled species has a molecular weight of 68-97 kDa and forms a characteristic broad, H-shaped band. The five less heavily labeled proteins have respective molecular weights of 24a, 26, 30a, 36 (a fuzzy band) and 180 kDa. The 24a kDa protein sometimes appeared as a double band. The 24a and 36a kDa proteins are partially solubilized from worms during the sonication process, without the addition of detergents (Figure 1).

12

When SDS extracted XL3 cuticles are further extracted by boiling in SDS + BME, proteins with molecular weights of 24b and 30b kDa are solubilized as well as a large number of minor proteins with molecular weights ranging from 40 to > 200 kDa. It is not certain if the 24b and 30b kDa SDS+BME-soluble proteins are the same as the SDS- soluble proteins with these molecular weights, so the symbols "a" and "b" are used to distinguish between them. Approximately 8% of the total radioactivity incorporated into cuticles could not be solubilized by the above treatments.

Extraction of labeled L4s with SDS revealed a different pattern of $^{125}$I-labeled surface proteins (Figure 1). The most heavily labeled species have respective molecular weights of 27, 29, and 200 kDa. The 27 and 29 kDa proteins appear as a single band in Figure 1, but are clearly resolved as two bands in autoradiograms that are exposed for shorter lengths of time. Minor proteins with respective molecular weights of 16, 18, 19, 36 (fuzzy), 42, 54, 78, 93 and 125 kDa are also solubilized by SDS. Variable quantities of the above proteins as well as a new 180 kDa protein are usually solubilized from SDS-treated L4 cuticles by boiling in SDS + BME. In addition, a large number of minor proteins with molecular weights ranging from 40 to >200 kDa were usually extracted. These proteins appeared as a background smear in this molecular weight range. Approximately 3% of the total radioactivity incorporated into L4 cuticles could not be solubilized by these procedures.

B. Characterization of XL3 and L4 Surface Proteins

1. Collagenase Digestions

XL3 or L4 surface proteins were treated with bacterial collagenase to determine if any of them was a collagen. Labeled surface proteins were mixed with 50 micrograms of an unlabeled SDS + BME extract of adult worm cuticles (this extract contains cuticle collagens), precipitated for one hour on ice with 9 volumes of ice cold acetone, and pelleted in a microfuge. After washing once with ice cold acetone, the pellet was air-dried, taken up in 40 microliters of collagenase digestion buffer (50 mM Tris-HCL pH 7.4, 10 mM CaCl$_2$, 0.15M NaCl) and incubated overnight at 37 in the presence of 2 BTC units of Clostridial collagenase (Form III, Advanced Biofactures Corp.). An additional 2 units of collagenase was added the next morning and the digestions allowed to proceed for an additional 6 hrs. Samples were then diluted with SDS sample buffer plus 5% BME, boiled for 2 minutes and analyzed by SDS-PAGE and autoradiography. Gels were stained with Coomassie blue prior to autoradiography to monitor the extent of digestion of adult cuticle collagens and to detect any non-specific proteolysis.

None of the major SDS-soluble XL3 or L4 surface proteins was digested by collagenase, indicating that none is a collagen (Figure 2). In contrast, most of the uniquely SDS + BME-soluble, labeled proteins of XL3s and L4 were digested, indicating that they probably are collagens. Exceptions were then 24 and the 30 kDa XL3 surface proteins solubilized by SDS + BME: they were not digested by collagenase.

2. Glycosidase Digestions

To determine whether any XL3 or L4 surface proteins was glycosylated, they were treated with Endoglycosidase F and N-glycanase. Both enzymes cleave N-linked sugar groups. For Endoglycosidase F digestions, $^{125}$I-labeled surface proteins in ST buffer were mixed with an equal volume of 200 mM sodium phosphate pH 8.6, 2% NP-40, 2% BME, 0.2% SDS, boiled for 5 minutes and cooled to room temperature. Endoglycosidase F or water was added and the mixtures incubated overnight at 37. For N-glycanase reactions, $^{125}$I-labeled surface proteins in ST buffer were brought to a final concentration of 10% BME, boiled for 5 minutes and cooled to room temperature. Samples were mixed with an equal volume of 0.55 M sodium phosphate pH 8.6, 3% NP-40 and incubated overnight at 37 with N-glycanase. Reactions were terminated by diluting samples with SDS sample buffer and boiling for 2 minutes. Samples were analyzed by SDS-PAGE and autoradiography. Ovalbumin was sometimes added to the digestion reactions as a control glycoprotein. Deglycosylation of ovalbumin was monitored by Coomassie blue staining of the gels.

The results for both Endoglycosidase F and N-glycanase were qualitatively similar and the Endoglycosidase F results are shown in Figure 3. Only the SDS-soluble surface proteins were analyzed in these experiments. Both the 30a and 36 kDa XL3 surface proteins disappeared after glycosidase treatment. A new band of 26 kDa appeared and the 24 kDa band became darker, suggesting that these are the molecular weights of the non-glycosylated precursors of these proteins. The 24a, 68-97 and 180 kDa XL3 proteins did not change mobilities after Endoglycosidase F or N-glycanase treatments. The 27, 29, 36 and 78 kDa L4 surface proteins were digested by these glycosidases. A new, intense band of 25 kDa (a single band even in short exposures of the gels) is apparent after glycosidase-treatment and probably is the non-

13

glycosylated precursor to the 27 and 29 kDa L4 proteins. The 78 kDa L4 surface protein changes mobility to 68 kDa after glycosidase treatment. The sensitivity of this protein to glycosidases indicates that it is distinct from the major XL3 surface protein of the corresponding molecular weight. A new, weak band of 33 kDa is present in the L4 extracts after glycosidase treatment and may be the precursor to the 36 kDa protein. We could not determine from these experiments if other minor L4 surface proteins were digested.

3. Immunofluorescence Studies

Live XL3s and L4s were analyzed in immunofluorescence assays (IFAs) using rabbit antisera prepared against either native or SDS-treated cuticles. A mixture of native XL3 and L4 cuticles was prepared from L4 worms and free XL3 cuticles that had been shed from XL3s as they molted into L4s. The L4 worms and free XL3 cuticles were sonicated together and the cuticle pieces washed extensively with sonication buffer as described above. Native adult cuticles were obtained by grinding frozen adults to fine particles with a mortar and pestle over liquid nitrogen. Worm material was then washed extensively with sonication buffer. Egg shells are the primary contaminant in adult cuticle preparations and were not removed. SDS-treated cuticles were prepared from XL3s and L4s that had been purified separately using Ficoll step gradients. After sonication and several washes with sonication buffer, the cuticle pieces were boiled for 2 min. in ST buffer and incubated overnight at room temperature with shaking. SDS-treated adult cuticles were prepared by treating native adult cuticle fragments with ST buffer in the same way. The next day, cuticle pieces were collected by centrifugation, boiled for 2 min. in fresh ST buffer and shaken at room temperature for several hours. Cuticle pieces were then washed 3 times with ST buffer and resuspended in physiological saline.

Cuticle pieces were mixed with Freund's complete adjuvant and injected intramuscularly at several sites into New Zealand white rabbits. Rabbits were boosted at monthly intervals with additional cuticle antigen mixed with Freund's incomplete adjuvant. Rabbits were bled 10 to 14 days following each boost.

For indirect immunofluorescence assays, live worms were washed several times with saline, once with PBS and incubated at room temperature with antisera diluted 1:50 with PBS. After 60 minutes the worms were washed 3 x 10 ml with PBS and incubated for 60 minutes with FITC-labelled, goat-anti-rabbit IgG second antibody diluted 1:100 with PBS. Worms were then washed 3 x 10 ml with PBS and visualized in the fluorescence microscope. Representative photomicrographs of the results obtained with these antisera are shown in Figure 4.

The rabbit anti-native cuticle serum (Rb-8061) prepared against a mixture of XL3 and L4 cuticles reacted strongly and uniformly over the entire surface of live XL3s and L4s (Figures 4A and 4C, respectively). In contrast, rabbit antisera prepared against SDS-extracted XL3 and L4 cuticles (Rb-6791 and Rb-7539 sera, respectively) failed to react, or reacted very weakly, with the surfaces of live worms in similar experiments (Figures 4B and 4D). The only significant reaction observed with these latter antisera was with the buccal cavity of XL3s. Neither the anti-native XL3/L4 cuticle serum, nor the anti-adult cuticle sera described below, reacted significantly with live SL3s (data not shown).

Rabbit antisera prepared against native or SDS-treated adult cuticles (Rb-8100 and Rb-8101 antisera, respectively) was also tested for reactions with live XL3s or L4s in IFA experiments. No significant reactions with live worms were observed with these antisera. The appearance of XL3s and L4s incubated with these antisera was comparable to that depicted in Figures 4B and 4D. These antisera do react strongly to many proteins, including cuticle collagens, of XL3s and L4s on Western blots of whole worm extracts (data not shown).

4. Reaction of $^{125}$I-labeled XL3 and L4 Surface Proteins with Immune Sheep Sera

Sera from two immune sheep (#697 and 698) and from a control, non-infected sheep (#695) were used in immunoprecipitation experiments to determine if they react with $^{125}$I-labeled surface proteins. These sera were obtained from Dr. R.J. Boisvenue of Eli Lilly and Company (Greenfield, IN). Sheep #697 and 698 had been orally infected with $2.5 \times 10^4$ H. contortus SL3 larvae, which developed into mature, egg-producing adults. About 40 days post-infection egg production abruptly ceased due to expulsion of the adult worms. At this time, the sheep were challenged with an additional $2.5 \times 10^4$ SL3 larvae. Monitoring of egg counts in the feces showed only a slight increase, indicating that the sheep resisted the challenge infection. Blood samples were taken from the sheep at various times post-challenge infection, allowed to clot and serum samples frozen at -20C. Only the SDS-soluble, $^{125}$I- labeled surface proteins were analyzed in these experiments. Immunoprecipitation experiments with these sera were performed as described in Section I(D)-(2), except that 12.5 l of affinity-purified rabbit anti-sheep IgG (Cappell Laboratories) was included in the Protein-A sepharose incubations to ensure efficient precipitation of sheep IgG.

a. XL3 surface proteins

Sera from sheep #697 and 698 specifically precipitated the 36 kDa, SDS-soluble XL3 surface protein, whereas serum from control sheep #695 and the affinity purified rabbit serum did not (Figure 5). Reaction of sera from sheep #697 and 698 with the 36 kDa XL3 surface protein was abolished, or greatly reduced, if the XL3 surface proteins were reduced with 1 - 5% 2-mercaptoethanol prior to incubation with sheep sera (Figure 6). This result suggests that the epitope(s) on the 36 kDa surface protein that is recognized by the immune sheep sera is conformationally dependent.

b. L4 surface proteins

Sera from sheep #697 and 698 precipitated the 27, 29, 36 and 200 kDa, SDS-soluble L4 surface proteins (Figure 7). Reaction with the 36 kDa protein was not observed if the L4 surface proteins were reduced with 1 - 5% 2-mercaptoethanol prior to incubation with sheep sera (as is required for deglycosylation experiments). When sera from sheep #697 and 698 were reacted with L4 surface proteins that had been deglycosylated with Endoglycosidase F (procedures as described in Section I(B)(1)), we found that the sera precipitated the 25 kDa protein that presumably is the deglycosylated precursor to the 27 and 29 kDa proteins (Figure 8). These sheep sera also reacted with the 200 kDa protein that does not change mobility after treatment with Endoglycosidase F.

C. Purification of XL3 and L4 Surface Proteins

The inventors discovered that boiling live XL3s briefly in 1% SDS specifically solubilized surface proteins from this developmental stage (Figure 9). Live XL3s (ca. 200 microliters packed volume) were suspended in 1 ml of 1% SDS, 0.125 M Tris-HCl pH 6.8, placed in boiling water for 2 min, removed from the water, mixed by inversion for an additional 2 min and pelleted for 1 min in a microfuge. The supernatant was drawn off, recentrifuged several times to remove all worms, frozen in a dry ice/ethanol bath and stored at -20C. Samples were later thawed and concentrated at 4°C using a 2 ml Centricon -10 apparatus (Amicon, 10 kDa molecular weight cut-off).

Concentrated protein samples were diluted into SDS-sample buffer and analyzed by SDS-PAGE. By Coomassie blue staining the 68-97 kDa surface protein with the characteristic H-shaped appearance on gels was by far the major species in these extracts (Figure 9). The 180 kDa surface protein generally could be detected as a faint band. Lesser amounts of proteins corresponding in molecular weight to other $^{125}$I-labeled polypeptides were visible on heavily overloaded gels and by silver-staining (gels not shown). The silver-stained gel pattern of these extracts more closely approximated the $^{125}$I-labeled surface protein pattern. The quantitative differences in patterns of surface proteins detected by $^{125}$I-labeling and by Coomassie blue staining could be due to availability of tyrosine residues for labeling and/or differential dye staining characteristics of the proteins.

The boiling SDS procedure was not as useful for purifying L4 surface proteins. The patterns of proteins obtained by this method was similar to that seen when sonicated worm lysates are boiled in SDS (gels not shown). In the light microscope, SDS-treated L4s appeared hollow and it was apparent that most cellular proteins had been solubilized, leaving empty cuticles. In contrast, SDS-treated XL3s appeared intact, although they were obviously dead.

The inventors surprisingly discovered that boiling L4 worms (and XL3s) in 100mM NaCl in buffer specifically solubilizes surface proteins. Live worms (ca. 200 microliters packed volume) were suspended in 1 ml of 100 mM NaCl, 10 mM Tris-HCl pH 7.4, placed in boiling water for 2 min, removed from the water, mixed by inversion for an additional 2 min and pelleted for 1 min in a microfuge. The supernatant was drawn off, recentrifuged several times to remove all worms, frozen in a dry ice/ethanol bath and stored at -20C. Samples were later thawed and concentrated at 4C using a 2 ml Centricon -10 apparatus (Amicon, 10 kDa molecular weight cut-off).

Concentrated protein samples were diluted into SDS-sample buffer and analyzed by SDS-PAGE. The Coomassie blue staining patterns of XL3 and L4 proteins solubilized by the NaCl procedure are shown in Figure 9. The NaCl-extract of XL3s appears essentially identical to that obtained by boiling briefly in 1% SDS, with the 68-97 kDa protein being predominant and the 180 kDa protein being the next most abundant species. The NaCl procedure seemed to yield less protein than the SDS procedure as judged by Coomassie blue staining of equivalent volumes of the extracts separated by SDS-PAGE. The NaCl procedure typically yielded 100-150 micrograms of surface proteins per $5 \times 10^6$ XL3s.

The L4 NaCl extract pattern is similar both qualitatively and quantitatively to the pattern obtained by

$^{125}$I-labeling. The predominant species have molecular weights of 27, 29, 78 and 200 kDa. The L4 NaCl extract has relatively less of a 36 kDa $^{125}$I-labeled species and more of a 42 kDa protein that is minor in $^{125}$I-labeled extracts. The amount of the latter protein was variable from extract to extract, but was never major. A protein of this molecular weight was also sometimes visible in NaCl and SDS extracts of XL3s, but again was only barely visible by $^{125}$I-labeling. The yield of L4 surface proteins was approximately the same as that obtained for XL3s.

D. Characterization of Purified XL3 and L4 Surface Proteins

1. Indirect immunofluorescence studies

New Zealand white rabbits were immunized subcutaneously with 100 micrograms of surface proteins mixed with Freund's complete adjuvant. The rabbits were boosted one month later with 50 micrograms of protein mixed with Freund's incomplete adjuvant. Rabbits were bled 10 to 14 days later. Rabbits 9446 and 153 received NaCl-extracted XL3 and L4 surface proteins, respectively (see Section C). Rabbit 154 received NaCl-extracted L4 surface proteins that had been denatured and reduced by boiling for 2 min in 1% SDS, 5% BME prior to mixing with adjuvants.

Immunized rabbits produced antibodies capable of reacting with native proteins present on the surfaces of live worms as determined by indirect immunofluorescence experiments (Figure 10). Reaction of a mixture of live XL3s and L4s with the anti-XL3 surface protein serum (Rb-9446) labels only the XL3s. In contrast, the anti-L4 surface protein serum (Rb-153) reacts with both live L4s and XL3s. Labeling of XL3s with the latter serum may be due to the 5-10% contamination of L4s with XL3s and molted XL3 cuticles in the large worm populations used to prepare the L4 surface protein extracts. The L4 has a 78 kDa surface protein that migrates in the same region of the gel as the major 68-97 kDa XL3 surface protein, so it is not possible to detect contamination by one-dimensional gel electrophoresis. Another rabbit antiserum (Rb-154), which was prepared against L4 surface proteins that had been isolated using NaCl and later denatured and reduced by boiling in 1% SDS + 5% BME, also reacted strongly with the surfaces of live XL3s and L4s (data not shown).

2. Immunoprecipitation experiments

For immunoprecipitation experiments, $^{125}$I-labeled XL3 and L4 surface proteins (either the SDS or SDS + BME extracts of the labeled worms) were boiled for 1 min, centrifuged in a microfuge for 10 min and 10- to 50-microliter aliquots mixed with 1 ml of 2% Triton-X- 100, 50 mM Tris- HCl pH 8.1, 150 mM NaCl, 0.1 mM EDTA and 25 microliters of hyperimmune or prebleed rabbit serum. After incubating overnight at 4° C with gentle rocking, 100 microliters of a Protein-A Sepharose slurry (250 milligrams Protein-A Sepharose (Sigma) in 3.5 ml of 10 mM Tris-HCl pH 8.0) was added and incubated with rocking for an additional 60 min at 4C. The Sepharose beads containing bound antigens were pelleted by centrifugation in a microfuge. The beads were washed 3 x 1 ml with a solution of 2% Triton X-100, 50 mM Tris-HCl pH 8.1, 150 mM NaCl, 0.1 mM EDTA. Bound antigens were eluted by boiling the beads for 3 min in 100 microliters of 1% SDS, 0.125 M Tris-HCl pH 6.8, 5% BME and collecting the supernatant. Samples were analyzed by SDS-PAGE and autoradiography.

The results of these experiments correlate with the IFA results, and are shown in Figure 11. The anti-XL3 surface protein serum precipitates all SDS-soluble $^{125}$I-labeled XL3 surface proteins. A weak reaction is observed with this serum against 68-97 kDa and 200 kDa proteins in the L4 SDS extract. These proteins could be of XL3 or L4 origin. The anti-XL3 surface protein serum does not precipitate the major 27 and 29 kDa SDS-soluble L4 surface proteins. In contrast, both anti-L4 surface protein sera (Rb-153 and Rb-154) precipitate all SDS-soluble $^{125}$I- labeled L4 and XL3 surface proteins. The Rb-153 and Rb-154 sera also react weakly with the 40 to >200 kDa smear in the SDS + BME extracts of $^{125}$I-labeled XL3s and L4s (lanes labeled B in Figure 3). These proteins have been shown previously to be primarily cuticle collagens (Section I(B)(1)).

3. Amino Acid Composition

The amino acid compositions of purified XL3 and L4 surface proteins were determined and are given in Table I. Surface proteins purified using the NaCl extraction procedure were analyzed in these experiments. Protein samples were brought to 1% SDS and precipitated by the addition of 9 volumes of ice-cold acetone. After washing twice with 90% acetone and air-drying, the samples were hydrolyzed <u>in vacuo</u> with 6N

hydrochloric acid for 24 hr at 110° C and analyzed on a Beckman amino acid analyzer.

Both sets of proteins are enriched for hydrophilic amino acids and, with the exception of alanine and glycine, are relatively poor in hydrophobic amino acids. The proteins are not enriched for glycine and proline residues, consistent with our previous finding that the proteins are not collagens (Section I(B)(1)). Of note is the finding that 26 percent of the amino acids in the XL3 surface protein extract, which should largely reflect the amino acid composition of the major 68-97 kDa species, were either glutamic acid or glutamine. The L4 surface proteins contained lesser amounts of these amino acids.

TABLE I

| Amino Acid Composition of Haemonchus contortus XL3 and L4 Surface Proteins[a] | | |
|---|---|---|
| Amino acid | XL3 | L4 |
| Percent of total amino acids | | |
| Cysteine | N.D.[b] | N.D.[b] |
| Aspartic Acid[c] | 13.0 | 10.8 |
| Threonine | 3.8 | 5.7 |
| Serine | 6.1 | 6.7 |
| Glutamic Acid[c] | 26.2 | 13.7 |
| Proline | 3.0 | 6.2 |
| Glycine | 10.1 | 14.1 |
| Alanine | 13.4 | 9.9 |
| Valine | 2.0 | 4.5 |
| Methionine | 4.7 | 3.8 |
| Isoleucine | 1.8 | 3.3 |
| Leucine | 4.8 | 5.1 |
| Tyrosine | 0.6 | 2.4 |
| Phenylalanine | 0.3 | 2.0 |
| Histidine | 0.3 | 1.5 |
| Lysine | 1.7 | 5.2 |
| Arginine | 8.4 | 5.2 |

a Averages of determinations for two different protein preparations purified using the NaCl procedure. Each analysis was performed on 7.5 micrograms of protein.
b Not determined.
c Includes the amidic forms.

The present inventors attempted to obtain the amino-terminal sequence of the major 68-97 kDa XL3 protein after elution of the protein from SDS gels or by direct sequencing of the XL3 NaCl extract. In neither case could sequence information be obtained, suggesting that the amino-terminal amino acid of the protein may be modified.

II. Anticoagulant Examples

1. Construction of an H. contortus:λEMBL-3 phage library

a. Isolation of H. contortus DNA

SL3s that had been frozen in liquid nitrogen were provided by Dr. R. Boisvenue of Eli Lilly and Company, Greenfield, Indiana. The frozen SL3s were ground to a fine powder with a mortar and pestle and placed in a liquid nitrogen bath. The worm powder was transferred to 30 ml Corex tubes and digested for 45 min at 65° C with Proteinase K (200 ug/ml) in a solution of 0.1 M Tris-HCl pH 8.5, 50 mM EDTA, 0.2 M NaCl, 1% SDS. The digestion mixture was alternately extracted several times with phenol and chloroform:isoamyl alcohol (24:1 ratio). DNA was precipitated from the aqueous phase by adjusting the solution to 0.3 M sodium acetate and adding 3 volumes of ethanol. The precipitated DNA was spooled out with a glass rod, air dried and resuspended in TE buffer (10 mM Tris-HCl pH 8.0, 1 mM EDTA). The DNA solution was treated with RNAse (50 ug/ml) for 60 min at 37, extracted sequentially several times with phenol and chloroform:isoamyl alcohol, brought to 0.3 M sodium acetate and the DNA precipitated by adding 3 volumes of ethanol. After air drying, the DNA was suspended in TE buffer.

b. Preparation of size-selected H. contortus DNA and ligation to λEMBL-3 phage DNA

Eighty micrograms of H. contortus DNA was partially digested with Sau3A so that the bulk of the DNA was in the 15-20 kb size range as determined by agarose gel electrophoresis. The digested DNA was heated to 65° C for 10 min, cooled to room temperature and gently layered over a 10-40% sucrose gradient in an SW41 tube (Maniatis et al., 1982). After centrifugation for 25 hr at 30,000 rpm at 20° C, the gradient was dripped from the bottom with a 23 gauge needle and fractions of 150-200 l collected. Alternate fractions were electrophoresed on a 0.7% agarose gel and fractions containing 17-20 kb DNA fragments were pooled. The pooled DNA was dialyzed extensively versus TE buffer, precipitated with ethanol and resuspended in TE buffer.

The size selected Haemonchus DNA was ligated overnight to the lambda phage vector EMBL-3 (Frischauf et al., 1983) that had been digested with EcoRI and BamHI. The ligation mix was packed in vitro using kits purchased from Boehringer Mannheim and plated on E. coli strains Q359.1 (which allows growth of only recombinant phage) and KRO (which allows growth of only non-recombinant EMBL-3 phage). These test platings revealed that a library of 160,000 recombinant phages containing H. contortus DNA inserts had been constructed. The entire library was amplified by plating on E. coli Q359.1 (10,000 phage per each of sixteen 15 cm agar plates). The amplified library consists of $6.6 \times 10^9$ recombinant phages.

2. Construction of a λgt11:adult worm cDNA library

a. Isolation of poly(A) + mRNA from adult worms

Adult worms were obtained from Dr. R. Boisvenue of Eli Lilly and Company, Greenfield, Indiana. The worms were obtained from the abomasa of experimentally infected sheep, washed with saline and frozen in liquid nitrogen. The frozen adult worms were ground to a fine powder with a mortar and pestle over a liquid nitrogen bath. The worm powder was transferred to a 50 ml plastic conical centrifuge tube and solubilized with 10 volumes of RNA lysis buffer (4 M guanidine hydrochloride, 0.13 M sodium acetate, pH 5.2, 0.5% Sarkoxyl, 1M 2-mercaptoethanol). The mixture was layered over one-half volume of 5.7 m CsCl, 50 mM sodium acetate, pH 5.2 and centrifuged at 25,000 rpm for 18 hr at 18° C in an SW28 rotor. The RNA pellet was suspended in RNA lysis buffer and precipitated by the addition of 3 volumes of ethanol. After centrifugation, the RNA pellet was suspended in 0.3 M sodium acetate, pH 5.2, ethanol precipitated, dried and resuspended in water. The above procedures are slight modifications of those described by Chirgwin et al. (1979).

Poly(A) + mRNA was isolated by passage of the RNA through a column of oligo dT cellulose essentially as described by Efstratiadis and Kafatos (1976). The poly(A) + mRNA was precipitated with ethanol, dried and resuspended in water. From 4.8 mg of total RNA the inventors isolated 191 μg of poly(A) + mRNA.

b. cDNA synthesis and ligation to λgt11 phage DNA

Double stranded cDNA was prepared from 2 μg of poly(A) + mRNA using a kit purchased from Amersham. The methods used were essentially those outlined in the protocol manual that accompanies the kit. Briefly, first strand synthesis was performed using AMV reverse transcriptase and priming the RNA with poly(dT). A small amount of $^{32}$P-dATP was included in the reaction mixture to follow the reaction and identify cDNA in subsequent isolation steps. Second strand synthesis was performed using RNAse H and E. coli DNA Pol-1 and the cDNA ends made blunt with T4 DNA polymerase. The double stranded cDNA was methylated with EcoRI methylase, extracted with phenol and ether, and isolated by passage through a 1 ml column of Sephacryl S-300 using 20 mM Tris-HCl pH 8.0, 100 mM NaCl, 1 mM Na$_2$ EDTA as the buffer. Phosphorylated EcoRI linkers, (8-mers purchased from New England Biolabs) were ligated overnight at 15° C to the cDNA using T4 DNA ligase. The cDNA was then digested with several hundred units of EcoRI and the cDNA separated from free linkers by passage through a 1 ml column of Sephacryl S-300. After ethanol precipitation and vacuum drying, the cDNA was suspended in water. Approximately 1.3μg of double

stranded cDNA was obtained.

The cDNA (10-30 ng per μg of vector) was ligated to the expression vector λgt11 (Young and Davis, 1983) that had been digested with EcoRI and dephosphorylated (purchased from Stratagene, Inc.). The ligation mix was packaged in vitro (kits purchased from Stratagene,. Inc.) and plated on E. coli strain Y1088 (Young and Davis, 1983) in the presence or absence of IPTG and XGAL to determine the number of recombinant phages containing cDNA inserts. Test platings revealed that a library of 4 x 10⁶ recombinant phages had been constructed. This library was amplified by plating on E. coli Y1088 (100,000 phages per 15 cm agar plates). After the phages were eluted with lambda dil and collected, test platings revealed that the amplified library contained a total of 7.5 x 10¹² phages of which 97% contained cDNA inserts.

B. Purification of Anticoagulant Extracts

1. Source of H. Contortus Adults

Adult H. contortus worms were recovered from young lambs infected with a pure drug-susceptible United States Department of Agriculture isolate BPL1. Approximately 35 days following experimental infection of worm-free lambs with an individual inoculum of 25,000 ± 3% ensheathed infective third-stage larvae (SL3) by intraruminal injection, donor lambs were euthanized. Immediately upon necropsy the adult worms were individually collected from the abomasum, placed in warm phosphate buffered saline until sufficient number of worms were collected and then frozen in liquid nitrogen. Frozen worms were stored in liquid nitrogen or at -70°.

2. Preparation of Anticoagulant Extracts

H. contortus adults were homogenized (10 mM MOPS, 150mM NaCl, pH 7.0), 25% (w/v) glycerol, 1mM EDTA, 1mM dithiothreitol and phenylmethanesulfonyl fluoride was then added to a final concentration of 1mM. After low-speed centrifugation (10,000 rpm for 20 min. in a JA-20 rotor), proteins in the supernatant were size-fractionated on a Sepharose CL-4B column using a buffer of 20mM Bis-Tris-propane pH 7.0 (w/v) glycerol, 1mM EDTA, 1mM dithiothreitol. Fractions were assayed for fibrinogen-degrading activity using the assay described below. The void volume contained most of the enzymatic activity. These fractions were pooled and applied to an FPLC Mono Q column. Bound proteins were eluted with a gradient of 0.05 to 0.4M NaCl in 20mM Bis-Tris-propane pH 7.0, 10% (w/v) glycerol, 1mM EDTA, 1mM dithiothreitol and fractions tested for fibrinogen-degrading activity. Active fractions were eluted and were pooled. Protein concentrations were determined using a protein assay kit purchased from BioRad Laboratories (Richmond, CA).

The fibrinogen degradation assay consisted of mixing 5-20 μl aliquots of the column fractions with an equal volume of a solution of 1% (w/v) bovine fribrinogen (Sigma) suspended in MBS, 1mM EDTA, 1mM dithiothreitol and incubating the samples for 1 hr at 37° C. Samples were then diluted into SDS sample buffer, boiled for 5 min and analyzed on 9% SDS-polyacrylamide gels (Laemmli, 1970). The gels were stained with Coomassie blue to identify fractions that degraded the fibrinogen.

C. Identification of Anticoagulant Activity in Haemonchus contortus

Adult Haemonchus contortus worms were thoroughly washed with cold MBS to remove food particles. The worms were then homogenized in a glass homogenizer using 10 ml of cold extraction buffer per gram (wet weight) of worms. The extract was then clarified by centrifuging. The supernatent was then tested for proteolytic ativity on casein agar, and for the inhibition of casein agar. When mixed with citrated sheep plasma very little increase in the coagulation time was observed. However, when the extract was allowed to incubate at 37° C with the plasma before initiation of coagulation a dramatic increase in the coagulation time was observed. This increase affected both the prothrombin time and the partial thromboplastin time. This suggested that the anticoagulant activity was directed at the final common pathway of coagulation. When the clotting reaction was initiated by the addition of purified thrombin, the coagulation time also increased upon incubation with the H. contortus extract. This demonstrated that the anticoagulant was acting on the fibrinogen in the sample.

This idea was confirmed by incubating the extract with purified sheep fibrinogen. Both the A(alpha) and the B(beta) subunits of th purified fibrinogen were degraded by the extract. The (gamma) subunit does not seem to be affected by the extract. The degradation of fibrinogen seen in the presence of the plasminogen activator streptokinase leads to different proteolytic fragments than does incubation with H. contortus extract. A further indication that the anticoagulant in the extract is not a plasminogen activation is that the fibrinogen degradation is not sensitive to certain plasminogen inhibitors. The inhibitor results are discussed in more detail later. The extract also causes the degradation of fibrinogen in plasma, as shown by western blot analysis.

After the anticoagulant activity was identified as a fibrinogen degrading enzyme, further characterization was desired. Possible protease inhibitors were tested for their effects on the fibrinogen degrading activity. Only inhibitors of thiol protease were effective in inhibiting activity. The most useful of these is E64 which is very specific for the active site thiols of protease, and is unreactive towards thiols in the active sites of other enzymes, as well as free SH groups present on proteins.

The specificity of E64 for the active sites of thiol proteases was used to advantage in radiolabeling. [$^{14}$C]-iodoacetic acid was used to label the thiols present in a partially purified preparation of the fibrinogenase. Because of enzymes, this reagent is highly selective towards thiol proteases. By preincubation the fibrinogenase with E64, the active site thiol can be blocked before reaction with the labeled IAA. This allows confirmation that a reactive thiol is indeed at the active site of a protease. When this technique was applied to partially purified preparation, several bands are labeled with IAA and are specifically protected by the E64. This labeling pattern includes the major band present in the purified fibrinogenase at about 35 kDa. Other bands are also labeled under these conditions, suggesting that they also correspond to thiol proteases. These could be related to the fibrinogenase, or they could conceivably be due to other activities which copurify with the fibrinogenase. The labeling of the 35 kDa band indicates that it contains the active site of the fibrinogenolytic enzyme.

The fibrinogenase has been purified from the extracts of H. contortus by means of gel filtration chromatography and ion exchange. Extract was applied to a sepharose 4B column. Fractions were monitored for activity by fibrinogen degradation as observed on gels. The fractions with the highest activity were pooled and applied to a MonoQ column. The activity was elutd with a salt gradient. This two step purification yielded a preparation which contained two major bands on SDS electrophoresis, as well as a number of minor bands, some of which correspond to peptides identified as thiol proteases by IAA labeling. It has not been possible to further purify this preparation while retaining activity of the fibrinogenase.

The fibrinogenase has been characterized as having a native MW of greater than 1,000,000 by means of gel filtration chromatography on an S12 sizing column, where it elutes at the void volume of the column. Standard proteins indicated that the MW corresponding to the void volume is on the order of 1 million. The activity also elutes in the void volume of the 4B column, which has an even higher cutoff.

Antiodies have been raised in rabbits to the anticoagulant. The antibodies were used to probe Western blots of extracts of H. contortus as well as the purified fractions. The antibodies reacted specifically with the 35 and 55 kDa bands. The antibody was further used to inhibit the activity of the enzyme. This was accomplished by incubating the antibody preparation with the anticoagulant, prior to incubation with fibrinogen. A further test of the inhibitory powers of the antibody was to study clotting time of plasma from the hyperimmune rabbit and control plasma. This experiment showed a substantial decrease in the effectiveness of the anticoagulant in the hyperimmune plasma.

D. Preparation of rabbit antisera to anticoagulant proteins and to individual 35 kDa and 55 kDa proteins

1. Preparation of rabbit antisera to anticoagulant extracts

Partially purified anticoagulant material from the Mono-Q column was emulsified with Freund's complete adjuvant and injected subcutaneously at several sites along the back of rabbit #9503. One month later, the rabbit was boosted with an additional protein emulsified with Freund's incomplete adjuvant. The rabbit was boosted at roughly one month intervals. Two weeks after each boost, the rabbit was bled and serum obtained after allowing the blood to clot overnight at 4° C.

2. Preparation of rabbit antisera to the 35 kDa and 55 kDa proteins

Partially-purified anticoagulant material obtained from the void volume of the Sepharose CL-4B sizing column was diluted into SDS sample buffer, electrophoresed on a 12% preparative SDS gel (0.75 mm thick) and stained for 15 min with 0.1% Coomassie blue in water to localize protein bands. Gel slices containing the 35 kDa and 55 kDa proteins were cut with a razor blade, diced, and the proteins eluted from the gel using an eluting apparatus purchased from Isco, Inc. The eluted proteins in running buffer were stored at -20° C.

Forty micrograms of the eluted 35 kDa protein in 280 $\mu$l of elution buffer was mixed with 220 ul of PBS and emulsified with 500 $\mu$l of Freund's complete adjuvant. This mixture was injected subcutaneously at several sites along the back of rabbit #10285 (Rb-10285). Approximately one month later, the rabbit was boosted with an additional 40 $\mu$g of protein emulsified with 500 $\mu$l of Freund's incomplete adjuvant. The rabbit was boosted a second time 3 weeks later. Two weeks after the final boost, the rabbit was sacrificed and bled-out. After clotting overnight at 4, the blood was centrifuged and serum collected, aliquoted and stored at -20° C until use.

Rabbit antisera to the 55 kDa protein was prepared in the same way except that 50 $\mu$g of eluted 55 kDa protein was used per injection. These rabbit antisera are designated Rb-10284 and Rb-10286.

E. Cloning of cDNAs and genes encoding the 35 kDa cysteine protease

1. Cloning of AC-1

cDNAs encoding the 35 kDa protease were isolated by screening the adult worm cDNA:λgt11 library with Rb-10285 antiserum. The adult cDNA:λgt11 expression library was plated on E. coli Y1090 (Young and Davis, 1983) at a density of 20,000 phages per 15 cm agar plate. The plates were incubated for 4 hr. at 42° C, then overlaid with nitrocellulose filters that had been wetted in 10 mM IPTG and air dried. The plates with filters were incubated overnight at 37° C. The next day the plates were cooled to 4° C for 60 min and the nitrocellulose filters were gently lifted off and batch washed 3 x 15 min in TBS (50 mM Tris-HCl, pH 8, 150 mM NaCl). The filters were incubated for 60 min in TBS + 2% BSA (bovine serum albumin), then for 2 hr with gentle rocking at room temperature with Rb-10285 serum diluted 1:200 in TBS + 2% BSA. These incubations were done in 15 cm petri dishes containing 20 ml of liquid. Two filters placed back to back were incubated per petri dish. The filters were then washed 3 x 15 min in TBS + .05% NP-40 and incubated for 60 min in 15 cm petri dishes containing horseradish peroxidase-conjugated goat anti-rabbit IgG antisera (Cappell Laboratories) diluted 1:500 in TBS + 2% BSA. After washing 3 x 15 min in TBS, the filters were placed in stain solution (200 ml TBS + 2.5 ml $H_2O_2$ + 40 ml of methanol containing 3 mg/ml 4 chloro-1-naphthol (Sigma- Aldrich Corp.)).

Screening of 80,000 phages yielded 4 positive phages. Agar plugs containing the positive phages were picked into lambda dil, replated and the phages rescreened with antibody several times as described above until plaque pure. The plaque pure phages were plated on E. coli Y1090, grown until confluent and overlain with lambda dil to create liquid lysate stocks.

The recombinant phage clones were used to affinity purify antibodies that react with their expressed antigen from the polyclonal rabbit serum ("antibody elution experiments"). The eluted antibodies were then used to probe Western blots of worm proteins to identify the target antigen corresponding to the cDNA in each phage clone. Phages were plated at a density of 1 x $10^4$ per 15 cm diameter agar plate using E. coli Y1090 and incubated at 42° C for 4 h. Nitrocellulose filters that had been impregnated with 10 mM IPTG and air-dried were placed on top of the phages and the plates were incubated overnight at 37° C. After cooling, the filters were removed, cut into 70 x 100 mm strips, incubated in TBS + 2% BSA for 1 h, and incubated overnight with Rb-10285 serum diluted 1:200 in TBS + 2% BSA.

The next day, the filter strips were washed 3 x 15 min with TBS + 0.1% (v/v) Nonidet-P40. Bound antibodies were eluted by washing the strips 3X with 300 $\mu$l of 5 mM glycine/500 mM NaCl/0.2% Tween-20/100 g ml$^{-1}$ BSA, pH 2.3. Eluted antibodies were neutralized with one-twentieth volume 1 M Tris- HCl pH 7.4, diluted approximately three-fold with TBS + 2% BSA and incubated overnight with nitrocellulose strips that had been cut from Western blots of total adult worm proteins or FPLC Mono Q-column purified anticoagulant proteins (separated on 12% SDS gels). Subsequent washes, secondary antibody incubations and staining with 4-chloro-1-naphthol and hydrogen peroxide were performed by standard procedures.

These antibody elution experiments revealed only phage 2B selected antibodies that reacted specifically with a 35 kDa protein on Western blots of adult worm extracts and in Mono Q column-purified anticoagulant extracts (Figure 13). The antibodies selected by phage 2B also consistently reacted weakly with a 37 kDa protein in the Mono Q column-purified anticoagulant preparations (Figure 13). As is suggested below, this 37 kDa protein may be a more heavily glycosylated form of the 35 kDa protein.

DNA from phage 2B was prepared by the plate lysate procedure (Davis et al., 1980) using E. coli Y1090 as the host (3 x $10^6$ phage mixed with 0.7 ml of an overnight Y1090 culture that had been centrifuged and resuspended in 1/2 volume of 10 mM MgSO$_4$ per 15 cm agar plate). Phage particles were purified by banding in CsCl step and equilibrium gradients using established procedures (Davis et al., 1980). Phage DNA was isolated by formamide extraction and ethanol precipitation (Davis et al., 1980). Phage DNA was resuspended in TE buffer.

Digestion of phage 2B DNA with EcoRI revealed that it contained a cDNA insert of about 180 bp. The nucleotide sequence of the cDNA was determined by the dideoxy nucleotide sequencing method (Sanger et al., 1977; Biggen et al., 1983) after subcloning into the EcoRI site of M13mp18. The nucleotide sequence of the cDNA revealed that it encoded only 12 amino acids fused to $\beta$-galactosidase; the remainder of the cDNA consisted of 3' untranslated sequences and a poly(A) tail. The 3' untranslated region contained a canonical poly(A) addition sequence AATAAA.

cDNA 2B was labeled with $^{32}$P by nick-translation and used to screen the cDNA library by plaque hybridization in order to identify larger cDNAs. The first such screen yielded cDNA 3-1, which was about 870 bp in length. A 40 nucleotide long oligomer that corresponds to the sequence at the 5' end of cDNA 3-1 was synthesized, end-labeled with $^{32}$P and used to screen the cDNA library for even larger cDNAs. Duplicate filters were screened with $^{32}$P-labeled cDNA 2B. The oligonucleotide, which has the sequence

5' - CACTTCAGGGTCGGGATCTTCTTTGACCATAAGATTTAGC - 3'

was synthesized on an Applied Biosystems DNA synthesizer. The labeled oligomer was hybridized to nitrocellulose filters at 32-52° C using 2X SSC/5X Denhardt's/0.5% sodium dodecyl sulfate. Filters were washed in 2X SSC/0.5% sodium dodecyl sulfate at 52° C. This screen yielded cDNAs F-1, O-1 and T-1, all of which hybridized to the oligomer and to cDNA 2B. cDNA F-1 was the largest, ~1100 bp, and was chosen for further characterization.

The relationship of cDNAs 2B, 3-1 and F-1 is shown in Figure 14, along with a composite restriction map of the cDNAs. Various regions of the cDNAs were sequenced and found to be identical in the regions in which the sequences overlapped. The one difference noted was that the 3' untranslated region of cDNAs 3-1 and F-1 were shorter than that of cDNA 2B (Figure 15). The composite nucleotide sequence and predicted amino acid sequence of the cDNAs is presented in Figure 15. This gene has been named AC-1.

The largest cDNA, F-1, contained a single long open reading frame but was missing an initiator methionine codon at its 5' end; therefore, the inventors presumed that the cDNA was not full-length. Northern blot hybridizations (see Section II(G)(1) indicated that cDNA F-1 hybridized to a 1.25 kb transcript in adult worm poly(A)[+] mRNA preparations. Primer-extension experiments using adult poly(A)[+] mRNA and the 40 nucleotide-long oligomer from the 5' end of cDNA 3-1 indicated that cDNA F-1 was about 10 nucleotides shorter than full-length at its 5' end. Nucleotide sequence analysis of the AC-1 gene isolated from an H. contortus: λEMBL-3 library (Section II(E)(3) confirmed this result and indicated that cDNA F-1 was missing the codon for only one amino acid, the initiator methionine. For completeness, the initiator methionine has been included in the sequence presented in Figure 15.

The AC-1 protein comprises 342 amino acids and has a predicted molecular weight of 38.4 kDa. At the N-terminus of the protein is a stretch of about 15 hydrophobic amino acids that could function as a signal sequence for sequestration of the protein to the rough endoplasmic reticulum, as a prelude to extracellular secretion or localization to cellular organelles. Computer analysis predicts that the signal sequence would be cleaved between amino acids 18 and 19 (Ala-Asp). There are no other significant hydrophobic regions in the protein.

The AC-1 protein contains 16 cysteine residues, two of which are present in the presumed signal sequence and would not be present in the mature protein. The protein also contains four potential N-linked glycosylation sequences (Asn-X-Ser/Thr, where X can be any amino acid), which are indicated in Figure 15. Treatment of purified anticoagulant proteins with Endoglycosidase F reduces the apparent molecular weight of the AC-1 protein to 33 kDa (Figure 18), indicating that the protein is glycosylated in vivo. The enzyme was used according to the methods provided by the supplier (Genzyme).

By Western blot analysis using Rb-10285 antiserum, the deglycosylated protein usually appeared as a dark band above a slightly smaller, lighter band, suggesting minor heterogeneity in the deglycosylated form of the protein. Although not clearly visible in Figure 18, the 37 kDa protein that reacts weakly with Rb-10285 antiserum and with eluted antibodies selected by phage 2B (see above) disappears and presumably also changes mobility to 33 kDa after Endoglycosidase F treatment, suggesting that it may be a more heavily glycosylated version of AC-1. Antisera prepared against the recombinant AC-1 protein synthesized in E. coli also react with this 37 kDa protein (Figure 26).

The primary sequence of AC-1 was compared to sequences of other known thiol proteases. These analyses revealed that AC-1 shows significant homology with mammalian cathepsin B (human, rat and mouse) and to a lesser extent with other cathepsins and with the plant protease papain (Chan et al., 1986; Cohen et al., 1986; Wada et al., 1987). AC-1 shares an overall 42% amino acid identity with human cathepsin B (Figure 17). A stretch of six identical amino acids that includes the active site cysteine of cathepsin B is present in AC-1 (Figure 19). This sequence also is conserved in papain (Figure 19) and is present in the same relative locations in all three proteases. Based upon these homologies, the inventors predict that cysteine-114 is the active site cysteine of the AC-1 protease. AC-1 can be aligned for homology with mature cathepsin B by introducing only two single amino acid gaps in the proteins (Figure 17). When these minor alignments are introduced, all 14 cysteines in the mature cathepsin B protein align with cysteine residues in AC-1, suggesting the AC-1 and cathepsin B have similar tertiary structures. In addition, towards the C-termini of the proteins there is a histidine residue in AC-1 (residue #285) that is in an identical position as the histidine residue (#278) that forms part of the active site of cathepsin B. The amino acids immediately surrounding these histidine residues are not as conserved as those surrounding the active site cysteine residues (Figure 17).

Cathepsin B is synthesized as a pre-proenzyme that contains an N-terminal signal sequence, followed by a stretch of 62 amino acids that must be cleaved from the proenzyme to generate the mature, active protease. The "pro" region also may be involved in localizing cathepsin B to lysosomes. The positions of the above amino acid cleavages in cathepsin B are marked in Figure 17. Nearly all of the amino acids that are identical between AC-1 and cathepsin B are located in the region of cathepsin B that constitutes the mature, active enzyme (Figure 17). Little similarity, other than length, exists between the "pre" and "pro" sequences of cathepsin B and AC-1. When just the mature form of cathepsin B is compared to the corresponding region of AC-1, the amino acid similarity between the two proteases increases to 49%.

## 2. Cloning of AC-3 and AC-4

Rb-9503 antiserum was used to screen the adult worm cDNA:λgt11 library using the procedures described in Section II(E)(1), with minor modification. This screen yielded cDNAs 2-1, 6-1 and 7-2. Nucleotide sequence analysis of the cDNAs revealed that they overlapped one another and encoded a protein that was related, but distinct from AC-1. The longest cDNA, 2-1, which was about 200 bp in length, was eluted from an agarose gel, labeled with $^{32}$P using random primers and used to screen the adult worm cDNA library by plaque hybridization. Several positive phages were identified and plaque-purified. DNA was prepared from pure phages and the cDNA inserts cloned into M13 phage vectors and their nucleotide sequences determined. These studies identified cDNA V-24, which is a longer version of cDNA 2-1 as well as a closely related cDNA, V-22. V-24 and V-22 share 94% amino acid sequence identity in the regions in which they overlap. The nucleotide and predicted amino acid sequences of cDNAs V-22 and V-24 are shown in Figures 29 and 28. The genes identified by cDNAs V-24 and V-22 are designated AC-3 and AC-4, respectively. The proteins predicted from the sequences of these cDNAs share ~70% amino acid sequence identity with AC-1 and AC-2. Both V-22 and V-24 are incomplete cDNAs. Longer cDNAs can be isolated by screening the adult cDNA expression library with $^{32}$P-labeled oligonucleotides derived from the 5′ end of the cDNAs (or any other region of the cDNA sequence). The complete gene sequence also can be obtained by isolating the gene from a genomic DNA library, identifying the region of DNA missing from the cDNA and sequencing this region.

## 3. Cloning of AC-2

The gene encoding AC-2 was isolated from the H. contortus λEMBL-3 phage library by screening the library with $^{32}$P-labeled cDNA 2B. The cDNA insert was isolated from 1.5% agarose gels using NA45 paper (Schleicher and Schuell). The cDNA insert was self-ligated overnight at 15° C with T4 ligase, ethanol precipitated and nick-translated with $^{32}$P. The labeled cDNA was used as a hybridization probe to screen the Haemonchus DNA:EMBL-3 library. The library was plated on E. coli LE392 at a density of 5 × 10³ phage per 10 cm plate and screened by plaque hybridization (Benton and Davis, 1977). Hybridization conditions used were 50% formamide, 0.1 M sodium phosphate pH 7.0, 0.1% SDS, 10 μg/ml sheared salmon sperm DNA, 3 X SET (1 X SET is 50 mM Tris-HCl pH 8.0, 150 mM NaCl, 1 mM EDTA) at 37° C. A screen of 100,000 recombinant EMBL-3 phages yielded two positive phages designated λMB1 and λMB2. These phages were plaque purified by repeated hybridization to the labeled 2B cDNA probe. Phages were prepared from plate lysates using E. coli LE 392 as the host and banding in CsCl gradients.

Mapping of the phage DNAs with restriction enzymes showed that the Haemonchus DNA inserts in the phages overlapped (Figure 20). The coding region of the gene was localized by hybridization of Southern blots of the restriction enzyme digests with the $^{32}$P-labeled 2B cDNA. The hybridizing region and restriction fragments upstream of this region were sequenced using the strategy shown in Figure 20. Appropriate restriction fragments were subcloned into M13 phage vectors and nucleotide sequences determined by the dideoxy chain termination method as modified for the use of [$^{35}$S]-labeled nucleotides. Comparison of the nucleotide sequence of the gene to that of the near full-length AC-1 cDNA F-1 (Figure 21) revealed that the gene contained multiple introns. This comparison also revealed that there were several nucleotide differences between the gene and the cDNA. These facts will be discussed further below. A large intron was encountered when trying to identify the region of the gene corresponding to the 5′ end of the cDNA. Sequencing of over 800 bp from this region of λMB1 phage DNA failed to locate sequences encoding the 5′ region of the cDNA. To locate the missing exon(s), Southern blots of λMB1 and λMB2 DNAs were probed with a $^{32}$P-labeled 40 nt oligomer corresponding in sequence to part of the missing region of the cDNAs. The oligomer, which has the sequence 5'-CACTTCAGGGTCGGGATCTTCTTTGACCATAAGATTTAGC-3', was synthesized on an Applied Biosystems DNA synthesizer. The oligomer was end-labeled with (γ-$^{32}$P)-ATP using polynucleotide kinase (Maxam and Gilbert, 1980) and hybridized to nitrocellulose filters at 42°C using 2X SSC//5X Denhardt's/0.5% sodium dodecyl sulfate (20X SSC = 3M NaCl/0.3M sodium citrate, pH 7.0). Filters were washed using the same solution minus Denhardt's at 45-55°C. The oligomer gave specific hybridization signals at the higher wash temperature.

These hybridisations indicated that the missing exon(s) was not present in λMB1 or 2. Therefore, the inventors rescreened the λEMBL-3 library to identify phages containing Haemonchus DNA inserts that extended further upstream of the region present in λMB1 and 2 ("chromosome walking" technique). The λEMBL-3 library was screened in duplicate with $^{32}$P-labeled restriction fragments from the left end (the 3.9 kb EcoRI fragment) and the middle (the 3.5 kb EcoRI fragment) of λMB2 (see Figure 20). The restriction fragments were eluted out of agarose gels using NA45 paper, self-ligated overnight, ethanol precipitated, resuspended in water and labeled with $^{32}$P by nick- translation. Three phages that hybridized only with the 3.9 kb EcoRI fragment were identified and plaque-purified. Hybridization of the phage DNAs with the 40 nt oligomer revealed that each of them contained the missing exon(s). One of the phages, λMB3, was mapped with restriction enzymes and shown to overlap λMB1 and 2, as expected (Figure 20). The region of λMB3 that hybridized to the 40 nt oligomer, and the region immediately upstream of it, were sequenced as outlined in Figure 20 and found to contain sequences for the missing regions of cDNAs 3-1 and F-1. cDNA F-1 does not contain an initiator methionine codon. The gene encodes a methionine three amino acids upstream of where cDNA F-1 terminates. Just upstream of this methionine codon is an in-frame TGA stop codon. Although it is possible that this methionine codon is the initiator methionine codon for the gene, the inventors do not believe so. Between this methionine codon and where cDNA F-1 terminates is the sequence TTTCAG/A, which is a consensus 3' intron acceptor splice sequence; the slash indicates where splicing would occur. If this sequence functions as a splice acceptor sequence, then the above methionine codon is present in an intron and could not be present in the mature mRNA. The upstream region was searched for other potential initiator methionine codons. Approximately 80 bp upstream of the putative 3' acceptor sequence is the sequence ATG/GTAA which fits the consensus intron 5' splice donor sequence. Splicing would join this ATG methionine codon in-frame with exon 2 and the remainder of the gene. There is an in-frame TGA termination codon 18 bp upstream of this methionine. Therefore, the inventors believe that this ATG is the actual initiator methionine for the gene. Primer extension experiments using the 3-1-40 oligomer and poly(A)[+] mRNA isolated from adult worms indicated that cDNA F-1 was approximately 10 bp shorter than full-length (Section II(E)(1)). If this analysis is correct, the cDNA F-1 is missing just the AT of the initiator ATG codon and approximately 8 nucleotides of 5' untranslated sequence.

The nucleotide sequence of the gene, including the small intron sequences is presented in Figure 21. The gene has 97% nucleotide identity with the AC-1 cDNA F-1, to which it is compared in Figure 21. Most of the nucleotide differences occur in the presumed 3' untranslated region of the gene (cDNA) and in third-based codon wobble positions that do not change amino acids. Seven nucleotide changes result in different amino acids. Overall, the gene and the F-1 cDNA have 98% protein sequence identity. At this time the inventors do not know if the gene is distinct from the gene that encodes the AC-1 cDNAs or whether the nucleotide (protein) differences are due to polymorphisms in a single gene in the H. contortus worm populations used to construct the cDNA and genomic DNA libraries. The inventors have isolated a partial cDNA (350 bp in length) that has an identical nucleotide sequence as the gene from the adult worm cDNA library, so the gene appears to be expressed (data not shown). Complicating this issue is the fact that the protease appears to be encoded by a multigene family (see below). Because of these uncertainties, the inventors have named the gene AC-2 to distinguish it from the AC-1 gene identified by cDNAs 2B, 3-1 and F-1 (Section II(E)(1)).

As shown in Figures 20 and 21, the AC-2 gene contains 11 introns that range in size from 57 bp to over 5.2 kb. Approximately 40 bp upstream of the proposed initiator methionine is a sequence that is similar to the eukaryotic TATA promoter element. The inventors have no evidence as yet that this sequence functions as a promoter for the AC-2 gene. Downstream of the TGA stop codon is a canonical AATAAA poly(A) addition sequence. These sequences are underlined in Figure 21.

The active site of the AC-1 protease has been tentatively identified by homology with the active site sequences of Cathepsin B and papain. The AC-1 protease, Cathepsin B and papain have an identical six amino acid sequence that includes the active site cysteine of these other proteases. These six amino acids (Cys-Gly-Ser- Cys-Trp-Ala; the underlined Cys is the active site cysteine of papain and cathepsin B) also are conserved in the predicted AC-2 protein (marked in Fig. 21). Intron 7 interrupts this conserved domain between Gly and Ser, indicating that the conserved active site sequence has not evolved as a single exon unit. Other constraints must act to preserve the conservation of this sequence in the polypeptide chains. As noted previously, AC-1 has four potential N-linked glycosylation sites (Asn-X-Ser/Thr, where X can be any amino acid). All four potential glycosylation sites are conserved in AC-2 and are marked in Figure 21.

F. AC proteases comprise a multi-gene family in H. contortus

Because of the differences in the nucleotide sequences of AC-1, AC-2, AC-3 and AC-4 it was of interest to determine the number of copies of the gene that were present in the H. contortus genome. Southern blot hybridizations of H. contortus genomic DNA under low stringency conditions revealed multiple hybridizing bands with several restriction enzymes (Figure 22). H. contortus genomic DNA (2-3 $\mu$g) was digested with restriction enzymes, size-fractionated on agarose gels and blotted onto nitrocellulose filters. Filters were hybridized in a solution of 50% formamide/0.1M sodium phosphate pH 7.4/0.1% sodium dodecyl sulfate/10 $\mu$g ml$^{-1}$ salmon sperm DNA/3X SET (1X SET =0.15M NaCl/0.5M Tris/1 mM EDTA, pH of a 20X stock adjusted to pH 7.9) at 30°C and washed in 0.1X SET/0.1% sodium dodecyl sulfate at 39°C. DNA size standards, $\lambda$ phage DNA digested with HindIII and $\phi$X174 DNA digested with HaeIII, were purchased from Bethasda Research Laboratories. The labeled probe used for these hybridizations was the AC-1 cDNA 2B, which hybridizes to a single 1.0 kb EcoRI fragment in $\lambda$MB1 (this fragment is marked in Figure 20). There is a 1.0 kb genomic DNA fragment that hybridizes to cDNA 2B and presumably corresponds to the 1.0 kb band in $\lambda$MB1 (Figure 22). The four other hybridizing bands detected in EcoRI digests of H. contortus DNA must derive from additional gene copies of the protease. The multiple hybridizing bands detected with the 2B cDNA probe is in contrast to the single hybridizing band detected with a tropomyosin gene probe (data not shown). These data indicate that there are multiple copies of the gene for the AC protease in the H. contortus genome. This result is consistent with the finding of 4 distinct gene sequences (AC-1, AC-2, AC-3 and AC-4) encoding the 35 kDa protease. Other members of the gene family can be isolated by DNA cross-hybridization using the hybridization conditions described above. Either the $\lambda$gt11 cDNA library or the $\lambda$EMBL-3 library can be screened by plaque hybridization. The proteins encoded by other family members will have related, but not necessarily identical, DNA and predicted amino acid sequences.

G. Developmental expression of AC protease mRNAs and proteins

1. Developmental expression of AC protease mRNAs

Northern blot hybridizations of adult worm poly(A)$^{+}$ mRNA with a $^{32}$P-labeled plasmid containing the 1.0 kb EcoRI fragment of AC-1 cDNA F-1 (Section II(E)(1)) showed a single hybridizing mRNA band of about 1250 nt in length under low-stringency conditions (Figure 23). The RNA was size-fractionated on denaturing formaldehyde gels (Lehrach et al., 1977), and blotted onto nitrocellulose filters. The filters wer hybridized in the solutions described for Southern blots and washed 4 X 30 min in the same solution at 37°C. The probe used for Northern blots was plasmid pBR325::F-1, which contains the 1.0 kb EcoRI fragment of AC-1 cDNA F-1 inserted into the EcoRI site of pBR325. Plasmid DNA was prepared according to Clewell (1972) and labeled with $^{32}$P by nick-translation (Rigby et al., 1977). RNA size standards (0.16- 1.77 kb) were purchased from Bethesda Research Laboratories. The size of this mRNA is in good agreement with the size of the largest AC-1 cDNA (F-1 = 1100 bp) and the predicted size of the gene. The data suggest that the multiple AC protease genes produce mRNAs of similar sizes. A weakly hybridizing band of the same size was detected in poly(A)$^{+}$ mRNA isolated from a mixture of third- and young fourth-stage larvae.

2. Developmental expression of AC proteins

a. Preparation of antisera against the recombinant AC-1 protein.

Rabbit antisera were prepared against an AC-1:- galactosidase fusion protein in order to study expression of the protein in various developmental stages of H. contortus. Because of the high degree of amino acid sequence identity between the predicted AC-1 and AC-2 proteins, the inventors expect that antisera raised against the AC-1 protein will cross-react with the AC-2 protein; however, it has not proven possible yet to test this assumption. The gene fusion was constructed by subcloning a fragment of cDNA 3-1 into the $\beta$-galactosidase expression plasmid pSEV6. Plasmid pSEV6 was derived from plasmid pSEV4, which is identical to plasmid pLG2 (Guarente et al., 1980), except that one of the two EcoRI sites of pLG2 has been destroyed, leaving a unique EcoRI site in the $\beta$-galactosidase gene. pSEV4 DNA was digested to completion with SphI and then partially digested with AatII. T4 DNA polymerase was used to make the DNA ends blunt. After agarose gel electrophoresis, the 7.6 kb partial digestion product was electroeluted, ethanol precipitated, dried, resuspended in buffer and ligated overnight with T4 DNA ligase to seal the blunt ends. This step destroys the SphI and AatII restriction sites. The ligation mixture was used to transform E. coli AMA1004 (Casadaban et al., 1983) and the cells plates in the presence of ampicillin, IPTG and XGAL. Plasmid DNA was isolated from blue colonies and one plasmid with the proper configuration was designated pSEV5. To create pSEV6, pSEV5 DNA was digested with NcoI and ligated overnight with complimentary DNA adapters of sequence 5' CATGAGATCTGGTAC 3' and 5' CATGGTACCAGATCT 3'. After transformation of E. coli AMA1004, plasmid DNA was isolated from several blue colonies and analyzed for the presence of unique NcoI, KpnI and BglII sites in the proper orientation. One such plasmid was designated pSEV6. A map of pSEV6 is shown in Figure 24.

To construct pSEV6::AC-1, a pBR322 plasmid containing AC-1 cDNA 3-1 was digested with EcoRV and ligated to EcoRI linkers with the sequence 5' CCGGAATTCCGG - 3'. After digestion with EcoRI, the ~800 bp fragment containing most of the AC-1 coding sequence was eluted from an agarose gel. The restriction fragment was subcloned into the unique EcoRI site of the $\beta$-galactosidase gene in plasmid pSEV6 (Figure 24). cDNAs in the proper orientation with respect to the $\beta$-galactosidase gene were selected by antibody screening and by digestion with XhoI, which cleaves asymmetrically within the 3-1 cDNA.

Bacteria containing the proper construct were grown in LB broth contain 50 $\mu$g ml$^{-1}$ ampicillin until the optical density at 600 nanometers was 0.3. Isopropyl--D-thiogalactopyranoside was then added to 1 mM and the culture shaken for an additional 2 h at 37° C. Bacteria were harvested by centrifugation and lysed by boiling briefly in SDS sample buffer (Laemmli and Favre, 1973) and vortexing.

The AC-1:$\beta$-galactosidase fusion protein has a molecular weight of 140 kDa and contains the final 241 amino acids of AC-1. Antiserum from rabbit #10285, which was immunized with the natural 35 kDa protein isolated from adult worms and whose serum was used to isolate cDNA 2B from the $\lambda$gt11:adult worm cDNA expression library, reacts on Western blots with the recombinant AC-1:$\beta$-galactosidase fusion protein (Figure 25), confirming that the modified cDNA was joined in the proper reading frame.

The AC-1:$\beta$-galactosidase fusion protein was electroeluted from preparative SDS gels and used to immunize rabbits #8552 and #9190. Rabbits were given primary injections of 100 $\mu$g of total protein emulsified in Freund's complete adjuvant. Subsequent booster injections given at monthly intervals were with 100-200 $\mu$g of total protein mixed with Freund's incomplete adjuvant. All injections were given subcutaneously. Serum was obtained 10-14 d after each booster injection. Both immune rabbit antisera reacted with a 35 kDa protein in adult worm extracts (Figure 26). In addition, both immune sera react with an identical size polypeptide in "anticoagulant" extracts that have been purified from adult worms using a fibrinogen-degradation assay. Both immune antisera also reacted with a 37 kDa protein in adult worm extracts and in anticoagulant extracts. Rb-8552 serum reacted more intensely with this protein than did the Rb-9190 or Rb-10285 antisera. Endoglycosidase F digestion experiments suggest that the 37 kDa protein is a more heavily glycosylated form of the 35 kDa protein because the apparent molecular weights of both proteins are reduced to 33 kDa after Endoglycosidase F treatment. The above analyses indicate that AC-1 (AC-2) encodes the 35 kDa protease present in anticoagulant extracts prepared from H. contortus adult worms and provide additional evidence that the 35 and 37 kDa proteins in these extracts are antigenically related and possibly different forms of the same protein.

b. Western blot analyses of AC proteins during parasite development

H. contortus is ingested by sheep as an SL3 (third-stage larva that retains the second larval-stage cuticle). In the rumen the SL3 sheds the second-stage cuticle and the resulting XL3 larva migrates to the abomasum. Within a few days, the XL3 molts into an L4 (fourth-stage larva). The L4 is the first stage that actively feeds by sucking blood. The L4 molts into a young adult after a few days. Very little growth occurs between the SL3 and young adult stages. Young adults are about 0.5 mm in length and grow to over 25 mm in length during the next few weeks. Nourishment for this growth is provided by metabolism of host blood components. The Rb-8552 and Rb-9190 antisera were used to probe Western blots (Towbin et al., 1979) of protein extracts of SL3s, XL3s, L4s and mature adults. For technical reasons the XL3 and L4 worms analyzed in these experiments were obtained by culturing worms in vitro in a defined media and were not isolated from infected sheep. As shown in Figure 27, the 35 kDa protein (and the 37 kDa form) was detected only in extracts of adult worms. Identical results were obtained with Rb-10285 serum, which was prepared against the natural 35 kDa protein isolated from adult worms (Figure 27). These results suggest that all members of the 35 kDa protease gene family (at least those members encoding proteins recognized by these antisera) have similar developmental expression patterns.

H. In vivo protection experiments with anticoagulant proteins

1. Protection Experiment #1

a. Experimental Design

Worm-free sheep approximately 12 months old were injected intramuscularly at several sites with purified anticoagulant material prepared as described in Section II(B) emulsified with adjuvant (vaccinated group) or with adjuvant only (control group). The time course of injections, the amount of anticoagulant used per injection and the type of adjuvant used are presented in Table I. Ten days after the final boost the sheep were challenged with 2500 H. contortus ensheathed third stage larvae (SL3s) given via a single intraruminal injection. Fecal samples were collected over the next 2 months and the number of H. contortus eggs per several milligram samples determined. These numbers were converted to eggs per gram of feces (EPG) by multiplying by the appropriate conversion factor. At 56 days post-worm challenge the sheep were sacrificed and their abomasa removed and examined for the presence of adult worms. Any worms found were counted and sexed. Serum samples were collected prior to the test, post-antigen injection, pre-worm challenge and at necropsy. Serum samples were used to probe Western blots of total adult worm protein extracts, partially purified anticoagulant or purified 35 kDa and 55 kDa proteins to assay for antibodies reactive with these proteins. Anthelmintic activity was scored by a reduction in worm egg production and by a reduction in worm populations at necropsy.

Table I

| Schedule of Anticoagulant Injections | |
|---|---|
| Day | Amount |
| 0 | 50 μg in CFA[b] |
| 7 | 75 μg in IFA[c] |
| 14 | 100 μg in IFA |
| 21 | 100 μg in IFA |
| 31 | 400 μg in IFA |
| 41 | worm challenge |
| 97 | necropsy |
| [a] Control sheep received adjuvant only according to the same schedule. | |

[b] Freund's complete adjuvant.
[c] Freund's incomplete adjuvant.

b. Results

Sheep vaccinated with the anticoagulant material exhibited both reduced group average EPGs and total worm counts at necropsy as shown in Table II. The group average EPGs of the vaccinated sheep were 77% lower than the group average of the control sheep at 42 days post-worm challenge. At the time of necropsy (56 days post-challenge), 3 of the 4 vaccinated sheep were negative for egg counts. The average total worm count at necropsy for the vaccinated group was 79% lower than that of the control group.

2. Protection Experiment #2

a. Experimental design

This trial was a repeat of the first anticoagulant experiment and all parameters of the experiment were identical except that sheep were divided into 3 groups: Group 1 was treated identically to the vaccinated group of trial #1; Group 2 was treated identically to the control group of trial #1; Group 3 was treated identically as Group 1 except that one-fifth of the initial anticoagulant injection was given intravenously. The booster injections for Group 3 were given intramuscularly and in an identical manner to Group 1.

b. Results

As with the first trial, sheep vaccinated with the anticoagulant material exhibited both reduced group average EPGs and total worm counts at necropsy as shown in Table III. At the termination of the study, 56 days post-worm challenge, all of the Group 1 sheep were negative for worm counts versus a group average of 325 for control Group 2. The total worm counts at necropsy for Group 1 sheep were 86% less than the total for the control sheep. Seven of the 8 sheep vaccinated with the anticoagulant material (Groups 1 and 3) had an average total worm count at necropsy of 42 versus an average of 269 for the control sheep. Only one of the vaccinated sheep in Group 3 (sheep #330) had a very high number of worms at necropsy and appears to be a non-responder animal.

3. Protection Experiment #3

a. Experimental design

This trial was designed to test the efficacy of the individual 35 kDa and 55 kDa proteins as vaccines. Preparative SDS gels of Mono-Q column-purified anticoagulant proteins were run and the 35 kDa and 55 kDa bands individually cut out and eluted essentially as described in Section II(D)(2). The sheep in the test were divided into 6 groups. Group A sheep received anticoagulant plus adjuvant; Group B sheep received anticoagulant that had been denatured in 0.1% SDS and emulsified in adjuvant; Group C sheep received adjuvant only; Group D sheep received adjuvant + 0.1% SDS; Group E sheep received the 35 kDa protein plus adjuvant; and Group F sheep received the 55 kDa protein plus adjuvant. Each group contained 4 sheep and they were injected with protein according to the schedule outlined in Table I, except that Groups E and F sheep received injections of 50, 75, 100, 100, 100 $\mu$g of protein on successive weeks, rather than the larger amounts of anticoagulant proteins given. The primary injection used Freund's complete adjuvant, while booster injections used Freund's incomplete adjuvant. All injections were given intramuscularly.

b. Results

Sheep vaccinated with anticoagulant proteins with or without SDS, as well as the sheep vaccinated with the 35 or 55 kDa proteins exhibited both reduced group average EPG's and total worm counts at necropsy as compared to adjuvant only injected sheep (Table IV). At necropsy, three out of the four sheep in each of the vaccinated groups receiving anticoagulant or 35 or 55 kDa proteins were negative for egg counts or produced only infertile eggs. Similarly, three out of four sheep in the anticoagulant vaccinated or 35/55 kDa vaccinated groups had no normal appearing adult worms at necropsy. Some of the sheep had small, infertile adults which suggested an immunological reaction directed against the worms.

4. Conclusion

The inventors conclude from protection experiments 1, 2 and 3 that the anticoagulant material is immunogenic in sheep and can be used as a vaccine to protect sheep from H. contortus infections as determined by two measurements of anthelmintic activity: reduction in worm egg counts and reduction of worm populations. Further, the inventors conclude that either the 35 kDa protein or the 55 kDa protein can be used individually as vaccines to protect sheep from H. contortus infections.

I. Expression of the Haemonchus 35 kDa protein in E. coli

The Haemonchus AC-1 protease has been expressed in E. coli in two forms. One construct, designated X-8, begins at Asp-19; the second construct, designated RV-2 and 3, begins at Ile-87. These constructs have been expressed using plasmids containing the T7 phage promoter or the Tac promoter to drive expression of the recombinant protein. All constructs use a translational coupler to enhance synthesis of the recombinant protein.

1. T7 promoter plasmid - pT5T

a. X-8 Construct

cDNA F-1 was digested with XbaI. The cut DNA was ligated to synthetic linkers of sequence:

MetAspGluAsnAlaAlaGlnGlyIlePro

5' GATCCGATCTTGGAGGATGATTAAATGGACGAAAACGCTGCACAGGGTATCCCG - 3'

3' - GCTAGAACCTCCTACTAATTTACCTGCTTTTGCGACGTGTCCCATAGGGCGATC - 5'

The ligation mixture was digested with EcoRI and the 1062 bp DNA fragment gel-purified. This DNA piece was ligated with T7 promoter plasmid pT5T DNA ( ) that had been digested with EcoRI and BamHI. The ligated DNA was used to transform E. coli strain BL21/DE3 and colonies containing the plasmid selected on ampicillin plates. Plasmid DNAs from several colonies were purified using the rapid boiling method (Homes and Quigley, 1981) and digested with EcoRI and BamHI to confirm that they contained the proper size DNA insert. Two plasmids with inserts of the correct size were identified and designated pT5T::X-1 and pT5T::X-8.

b. RV Constructs

cDNA F-1 was digested with EcoRV and ligated with synthetic linkers of sequence:

Met

5' GATCCGATCTTGGAGGATGATTAAATG - 3'

3'- GCTAGAACCTCCTACTAATTTAC - 5'

The ligated DNA was digested with EcoRI and the 857 bp band gel-purified. This DNA piece was ligated with plasmid pT5T DNA that had been digested with EcoRI and BamHI. The ligated DNA was used to transform E. coli strain BL21/DE3 and colonies containing the plasmid selected on ampicillin plates. Plasmid DNAs from several colonies were purified and digested with EcoRI and BamHI to make sure that they contained the proper size DNA insert. Two plasmids with correctly sized inserts were identified and designated pT5T::RV-2 and pT5T::RV-3.

2. Tac promoter plasmid - pT3XI-tac10

The X-8 construct was transferred to the TAC promoter plasmid pT3XI-2-tac10 as follows. Plasmid pT5T::X-8 DNA was digested with SmaI (the SmaI site occurs in the polylinker sequence downstream of the X-8 sequences), ligated with SacII linkers of sequence: 5' GCCGCGGC 3', and digested with BamHI and SacII. The 1078 bp BamHI:SacII fragment was gel-purified and ligated with pT3XI-2-tac10 DNA that had been digested with BamHI and SacII and gel-purified. The ligation mixture was used to transform E. coli JM107 and colonies selected on tetracycline plates. A colony containing the desired construct, called pT3XI-2-tac10::F-1(X-8)-8, or Tac X-8 for short, was selected by colony hybridization (Grunstein and Hogness, 1975) using the $^{32}$P-labeled F-1 cDNA insert as a hybridization probe. Tac X-8 was verified by restriction mapping and by DNA sequencing of the 5' end of the insert.

3. Synthesis of the recombinant Haemonchus protease

E. coli BL21/DE3 containing the T7 promotor:RV2 (pT5T::RV2) and T7 promoter:X-8 (pT5T::X-8) plasmids were grown in LB broth containing 12.5 ^mg ml$^{-1}$ of tetracycline until the $OD_{600}$ = 0.3. At this time expression of the protein was induced by adding IPTG to 1mM. The cells were grown for various lengths of time, harvested by centrifugation, lysed in SDS sample buffer and electrophoresed on 12% SDS gels. A Coomassie blue stain of the gel is shown in Figure 30 and a Western blot of the proteins using antisera against the 35 kDa protein purified from adult Haemonchus (Rb-10285 sera; see section II(D)(2) is shown in Figure 30.

The RV-2 construct produces a 30 kDa band that reacts with Rb-10285 sera and increases in abundance upon induction with IPTG. The X-8 construct produces proteins of molecular weights of 38 and 32 kDa that react with Rb-10285 serum. The 32 kDa protein is more abundant than the 38 kDa protein. Both proteins decrease in abundance upon induction with IPTG.

Expression of Tac X-8 in E. coli JM107 yielded results similar to expression of the X-8 construct in PT5T. The major form of the protease produced is 32 kDa (Figure 30). A 38 kDa form also is produced. Both the 32 and 38 kDa proteins react with Rb-10285 serum (Figure 30) and both proteins decrease in abundance upon induction with 1 mM IPTG.

4. Isolation of Recombinant 35 kd Protein

The TAC X-8 construct in JM107, described above, was grown to late log phase (OD$_{660}$ 1.0 to 2.0) in Luria broth (LB) with 12.5 ^mg/ml of tetracyclin. The cells were harvested without induction. The pelleted cells were resuspended in 25 mM TRIS:Cl pH 8.0, 5 mM DDT and 15 mM NaCL at a proportion of 20 ml to 1 gm wet weight of cells. The suspension was put through a French Press at 18,000 psi three times. The cell lysate was then centrifuged for 30 minutes at 30,00 x g. The pellet was resuspended with a loose-fitting dounce in a solution of 6 M urea and 100 mM 2-mercaptoethanol at a volume of 10 ml to each original gm cells. Following resuspension 0.7 M borax pH 7.0 and 10% polyethyleneimine were added at 0.15 ml and 0.05 ml, respectively, to each 10 ml of resuspended volume. The resuspended solution was centrifuged for 30 min at 30,000 x g.

The supernatant was then loaded onto an S-Sepharose column (Pharmacia) in a ratio of 10 ml per 1 ml of packed beads with a column height to diameter size of 11.0 cm x 1.0 cm. The column was equilibrated in buffer containing 25 mM TRIS:HCl pH 8.0, 5 mM DTT, 1 mM EDTA and 6 M urea. Following application of the sample the same buffer was used to wash the column. Proteins bound to the column were eluted with a linear gradient of NaCl up to 500 mM. The recombinant 35 kDa protein was eluted between 125 and 175 mM NaCl. Both the 37 and 32 kDa species were present and in the same ratios observed in the original lysate. Scanning of Coomassie stained SDS-PAGE gels of the final cooled recombinant material indicated that the 37 and 32 kDa species represented greater than 60% of the stained protein. Figure 31 demonstrates protein profiles representative of fractions obtained by this procedure.

J. Anti-coagulant Antiserum Experiments

As shown below, rabbit serum specific towards the SDS-denatured 35 Kd polypeptide does not directly inhibit the fibrinogenase activity in the standard enzyme assay. However, this antiserum can be used to select out this activity, thus supporting the evidence that this polypeptide is at least in part, if not totally, responsible for the fibrinogenase activity. That the other major polypeptide, the 55 kd protein, may also be part of the fibrinogen cleavage activity could not be ruled out.

Serum from control sheep and those immunized with the anticoagulant preparation showed partial inhibition of the fibrinogenase activity. IgG fractions isolated from these sera continued to demonstrate inhibition of the enzyme and therefore a direct link between protection and enzyme inhibition could not be made. The sera from immunized sheep do react with the 35 and 55 kDa polypeptides by Western blot analysis. It is possible that direct inhibition is not required for the effective protection to worm challenge, but that immune clearance or complement-mediated damage may be mechanisms for protection.

The anti-coagulase activity from adult worms had been determined to be a specific fibrinogen cleavage activity. As shown in Figure 32, the alpha and beta bands of bovine fibrinogen are degraded when incubated with increasing amounts of the partially purified enzyme preparation. Analysis of the enzyme preparation of SDS-PAGE and subsequent staining with Coomassie blue demonstrate two major bands of approximately 35 and 55 kDa. When the anti-coagulase is similarly electrophoresed and stained with the more sensitive silver staining method a number of additional polypeptides are visualized (Figure 33). Some of the higher molecular weight minor bands are believed to represent collagen polypeptides. Antiserum towards the anti-coagulant preparation raised in rabbits, as well as in sheep during protection experiments, react with collagens.

Support that the 35 kDa polypeptide is the catalytic subunit comes from active site labeling experiments. The fibrinogenase activity is thiol dependent and the use of appropriate inhibitors and thiol labeling reagents indicate that the 35 kDa polypeptide contains an active thiol. Evidence that the fibrinogenase activity may be associated with other polypeptides or is a single polypeptide aggregate comes from native molecular weight sizing columns showing the activity to elute at a molecular weight of at least one million. Attempts to disaggregate the complex and maintain activity were unsuccessful.

2. Inhibition Studies With Rabbit Serum

In order to facilitate cloning of the 35 and 55 kDa polypeptides, polyclonal rabbit sera were raised to these polypeptides. Preparative quantities of anti-coagulant were electrophoresed on SDS polyacrylamide gels and the proteins eluted from the gels. The purified and SDS denatured proteins were injected into rabbits and the serum tested for reactivity on Westerns. One serum obtained was specific for the 35 Kd, however the second serum was contaminated during the isolation and/or injection steps of the process and produced a serum reactive to both the 35 and 55 kDa polypeptides.

Direct Enzyme Inhibition Studies. The rabbit sera were tested for their ability to directly inhibit the fibrinogenase activity. The serum and enzyme were preincubated and then substrate was added and following incubation analyzed by gel electrophoresis. Both the preimmune and immune serum (anti-35 and anti-35/55) were inhibitory to the fibrinogenase. In order to address this apparent non-specific inhibition, the IgG was fractionated from each of these serum samples. Control Western analysis indicated that the reactivity towards the polypeptides did indeed separate with the IgG fraction.

Repeat inhibition studies with the isolated IgG fractions from the pre-immune and immune serum indicated that the preimmune no longer inhibited the enzyme. However, as shown in Figure 34 the IgG fraction specific for the 35 kDa polypeptide did not show significant inhibition of the fibrinogenase in this assay. Similar results were obtained with fractionated IgG from the antiserum specific for the 35 and 55 kDa polypeptides. The fact that these antisera were raised against SDS-denatured polypeptides may explain their failure to inhibit the native enzyme.

Immune Selection Inhibition Experiments. In order to further utilize these IgG fractions and give support that the 35 kDa polypeptide is indeed part of the fibrinogenase activity, the following experiment was done. The IgG fractions were incubated with the anti-coagulase preparation. Then the IgG and any polypeptides to which the immunoglobulins had reacted were removed from the preparation by incubation with fixed Staph A cells. Following centrifugation the Staph A cell step was repeated. The supernatants to this contrifugation were assayed for fibrinogenase activity.

The results of the enzyme assays is shown in Figure 35. As evidenced by this figure a number of controls were required. Close inspection of lanes 5 and 8 for the pre-immune and antiserum towards the 35 kDa polypeptide and lanes 11 and 14 for pre-immune and antiserum towards both 35 and 55 kDa polypeptides indicate both immune IgG preparations effected the reduction of enzyme activity in comparison to their respective pre-immune IgG fractions. Thus, both IgG fractions reactive with the polypeptides diminished the activity from the anti-coagulase preparation. This observation is reproducible.

The second part of this experiment was to confirm that the loss of activity was indeed due to the selection of the particular polypeptides in question. This was done by combining the Staph A cells from each sample and boiling off both the IgG and any polypeptides they had selected from the anticoagulase preparations. These samples were electro-phoresed on an SDS-PAGE and blotted to nitrocellulose. The Western was then incubated with antisera specific to the 35 and 55 kDa polypeptides. This Western is shown in Figure 36. Because the original sera was made in rabbit, the second antibody used for color visualization was goat anti-rabbit IgG linked to HRP. Therefore all of the complexed rabbit IgG reacted and obliterated any chance of seeing the 55 kDa polypeptide if it had been selected out. However, in the appropriate lanes (8 and 14) evidence that the 35 kDa polypeptide was complexed is evident.

Because one serum was specific towards the 35 kDa polypeptide, it is strong evidence that this polypeptide is responsible for the fibrinogenase activity. That the 55 kDa polypeptide or other polypeptides may also be involved cannot be ruled out. Serum specific for the 55 kDa or other polypeptides would help in discerning the possible participation of these other proteins.

### 3. Enzyme Inhibition With Immune Sheep Serum

Immune and control sheep serum from anti-coagulant challenge experiment #1 were tested for their ability to inhibit the fibrinogenase. Similar to the rabbit serum experiments, serum from both control and test sheep inhibited the fibrinogenase. Therefore, IgG from the two control and four test sheep sera was isolated and tested for inhibition of the enzyme. The results, shown in Figure 37, indicate that both the control and the four test IgG fractions show partial inhibition of the enzyme. Close inspection suggests that the immune IgG fractions may inhibit slightly more than the test serum IgG. Unfortunately, the conclusion must be that the results are equivocal. It did not determine whether the inhibition is due to non-specific factors or specific immunoglobins towards the anti-coagulant. Additional steps towards eliminating the non-specific inhibition would be required. Also, other more sensitive assays could be used to measure inhibition.

It has already been established that the test sheep serum are reactive with the 35 and 55 kDa polypeptides. Therefore a similar experiment of immune selection described above by analogy should give a similar result.

### K. Isolation of the H. contortus gene(s) encoding the AC-3 and AC-4 cDNAs

The H. contortus:λEMBL-3 library was screened by plaque-hybridization with $^{32}$P-labeled cDNA V-22 using procedures similar to those described in Section II.E.3. The hybridization and wash temperatures were 37°C. cDNA V-22 had been eluted from agarose gels and labeled with $^{32}$P using random primers.

EP 0 434 909 A2

Three phages out of ca. 90,000 hybridized to the cDNA and were plaque-purified. The phages have been named λMB4, λMB5, and λMB6. The regions of the phages that hybridize to the V-22 and F-1 cDNAs can be determined using procedures similar to those described in Section II for mapping the AC-2 gene in λMB1. DNA sequences encoding exons 1-4 can be identified using the F-1 exon 1-4 probe under low stringency hybridization conditions (30% formamide solutions-see Section II.P.3). If sequences corresponding to exons 1-4 are not present in λMB4-6, then "chromosome walking" techniques can be employed to rescreen the library to identify recombinant λEMBL-3 phages that contain H. contortus DNA sequences extending 5' of the coding sequences present in λMB4-6. Procedures for doing so are fully presented in Section II.E.3, which describes the isolation and mapping of λEMBL-3 phages encoding exons 1-4 of the H. contortus AC-2 gene. Regions of the λEMBL-3 phages that hybridize to cDNAs V-22, F-1, or the F-1 exon 1-4 probe can be sequenced to identify DNA sequences corresponding to coding regions of the gene that are not present in cDNAs V-22 and V-24.

L. Construction of a DNA probe specific for exons 1-4 of nematode cysteine protease genes

Plasmid pBR325::F-1, which contains the ca. 1.0 kb EcoRI fragment of cDNA F-1 inserted into the EcoRI site of pBR325, was digested with XbaI and ligated with oligonucleotide adaptors of sequence:
5'-GATCCATGAAATACTTGGTGCTTGCACTTTGCACCT
3'- GTACTTTATGAACCACGAACGTGAAACGTGGA
ATCTTTGTTCCCAAACCGGAGCAGACGAGAATGCTG
TAGAAACAAGGGTTTGGCCTCGTCTGCTCTTACGAC
CCCAAGGCATTCCT -3'
GGGTTCCGTAAGGAGATC -5' .

The ligation mixture was digested with EcoRI + BamHI and the 1.1 kb band containing the F-1 oligonucleotide adapter DNA sequences was gel-purified. The eluted DNA band was ligated with plasmid pcDNA1 vector DNA (Invitrogen, San Diego, CA) that had been digested with EcoRI + BamHI and gel-purified. The ligation mixture was used to transform E. coli strain MC1061/P3 (Invitrogen). Colonies containing plasmids were selected on ampicillin + tetracycline plates, by taking advantage of the SupF gene in the plasmid. Plasmids containing the correctly ligated H. contortus F-1 sequence were identified by restriction mapping and nucleotide sequencing. One such plasmid was named pcDNA::F1.

A 280 bp DNA probe can be prepared by digesting pcDNA::F-1 DNA with BamHI + XhoI. The DNA fragment can be purified from agarose gels, labeled with $^{32}$P and used in Southern blot hybridization experiments to locate DNA sequences encoding exons 1-4 of nematode cysteine protease genes. This DNA fragment contains sequences corresponding to exons 1-4 of the H. contortus AC-2 gene, plus sequences encoding 3 amino acids of exon 5. The inventors used this exon 1-4 probe to identify exon 1-4 coding sequences in λMB1, λMB3, λ002 and λ007. The exon 1-4 probe hybridized specifically to a 1.7 kb EcoRI fragment in λMB1 and a 3.5 kb fragment in λMB3 (Figure 51) using hybridization solutions containing 30% or 40% formamide at 32C (see Section II.P.3. for hybridization solution recipes). The location of the hybridizing regions in λMB1 and λMB3 are shown in Figure 20. The fact that the probe hybridizes to λMB1 DNA indicates that there is a second cysteine protease gene encoded in part (exons 1-4) by H. contortus DNA sequences in λMB1. The remainder of this second gene can be identified by using chromosome walking techniques (Section II.E.) to isolate new λEMBL-3 phages that extend 3' of the coding sequences present in λMB1. cDNA F-1 can be used as a hybridization probe to localize exons 5-12 on these new λEMBL-3 phages.

The inventors discovered that it was necessary to reduce the formamide concentrations in hybridization solutions (Section II.P.3.) to 30% (at 32° C) to detect DNA sequences that cross-hybridized with the exon 1-4 probe in λ002 and λ007. Under these conditions, the exon 1-4 probe hybridized to a 3.6 kb BamHI fragment in λ007 and to a 7.9 kb BamHI fragment and a 3.5 kb SstI/BamHI fragment in λ002. The locations of these restriction fragments are marked in Figure 52. From the locations of the exon 1-4 hybridizing regions, the inventors inferred that both phages contain DNA sequences encoding portions of two linked cysteine protease genes.

M. Isolation of cDNAs encoding the 55 kDa protein present in anticoagulant extracts

The H. contortus adult worm cDNA expression library was screened with rabbit and sheep antisera raised against the 55 kDa protein eluted from preparative SDS gels. The rabbit antisera used were Rb-10284 and Rb-10286, which are described in Section II.D.2. The sheep sera used were a pool of sera from sheep #641, 648, 673, and 842, which were part of sheep protection experiment # 3 (Section II.H.3.).

32

The library was screened with antisera essentially as described in Section II.E.1. Rb-10284 serum was used at a dilution of 1:500. Screening of $4 \times 10^5$ recombinant phages with this antiserum yielded 17 positive phages which were plaque-purified and named 84-1 to 84-17. The cDNA expression library also was screened with Rb-10286 serum at a dilution of 1:500. This screen yielded cDNAs 86-5, 7 and 8. The cDNA library also was screened with the pool of sheep sera described above. Screening of $1.2 \times 10^6$ phages yielded 5 positive phages. The phages were named SH-27a, -29a, -34a, -34b and -37a. DNAs were prepared from the phages as described in Section II.E.1. The sizes of the cDNA inserts were determined by agarose gel electrophoresis after digestion of the phage DNAs with EcoRI. Selected cDNA inserts were eluted from the gel, labeled with $^{32}P$ by nick-translation and hybridized to nitrocellulose filters containing ordered arrays of the phages in order to determine which cDNAs were related. These experiments indicated that cDNAs 84-1, -2, -3, -5, -7, -11, -12, -14, -15, and cDNAs SH-29a, -34a, and -37a cross-hybridized, and therefore, are products of the same or related genes. cDNAs 84-8, 86-7 and 86-8 also cross-hybridized and are products of the same or closely related genes. cDNA 84-4 appears to be unique.

H. contortus proteins encoded by the cDNAs 84-1 to 84-17 were identified by antibody elution experiments, which were performed essentially as described in Section II.E.1. Rb-10284 serum was used at a dilution of 1:500. Antibodies selected by phages 84-1, -2, -3, -5, -7, -11, -12, -14 and -15 reacted with a 55 kDa protein in whole adult worm extracts and in purified anticoagulant extracts (Figure 46). The protein reactive with the antibodies selected by these phages sometimes appeared as a doublet at 55 kDa. These results indicated that this group of cDNAs encode the major 55kDa protein, called 55A, present in anticoagulant extracts. A developmental Western blot using antibodies selected by phage 84-2 showed that the 55A protein is expressed by adult worms but not by XL3s or L4s (Figure 47).

Antibodies selected by phages 84-8, 86-7, and 86-8 reacted strongly with a 55 kDa protein in whole adult worm extracts, but weakly with purified anticoagulant extracts (Figure 46). Thus, the inventors concluded that these cDNAs encode a 55 kDa protein, called 55B, that is a minor component of anticoagulant extracts. The affinity-purified antibodies selected by these phage clones also reacted weakly with an ca. 100kDa protein in whole worm extracts (Figure 46). The 100 kDa protein probably is an aggregate of the 55 kDa protein. A developmental Western blot using antibodies selected by phage 84-8 indicated that the 55B protein and the antigenically related 100 kDa protein encoded by these cDNAs is expressed by XL3s, L4s and adults (Figure 47). Antibodies selected by phage 84-4 also reacted strongly with a 55 kDa protein and weakly with a 100 kDa protein present in whole adult worm proteins (Figure 46). This protein, called 55C, also appears to be a minor component of purified anticoagulant extracts. Protein 55C is expressed by XL3s, L4s and adults (Figure 47).

Partial restriction enzyme maps and the cross-hybridization studies allowed the cDNAs that encode the 55A protein to be aligned. These studies showed that cDNAs 84-1 and 84-2 were the largest. The relationship of the two cDNAs is shown in Figure 48.

The nucleotide sequences of cDNAs 84-2 and 84-1 were determined using procedures described in Section II.E.1. The nucleotide sequences and predicted amino acid sequences are shown in Figure 49.

Partial nucleotide sequences of cDNAs 84-4 and 84-8 were determined using similar procedures and are shown in Figures 54 and 55, respectively.

Larger or full-length cDNAs encoding the H. contortus 55A, B, and C proteins can be isolated by rescreening the adult worm cDNA library with $^{32}P$-labeled restriction fragments of the cDNAs or with $^{32}P$-labeled oligonucleotide probes corresponding to the sequence of the cDNAs. Examples of these procedures are outlined in Section II.E., which describes the isolation of larger cDNAs encoding the H. contortus 35 kDa AC-1 cysteine protease.

Expression of the proteins encoded in part by cDNAs 84-2 (or 84-1) 84-4 and 84-8 can be achieved in E.coli using procedures similar to those described in Section II. The purified recombinant proteins can be administered to sheep to protect them from H. contortus infections.

The 55A protein was expressed in E. coli in the following way: Plasmid pT5T DNA was digested with BamHI and ligated to synthetic linkers of sequence:

5´ GAT CCG ATC TTG GAG GAT GAT TAA ATG G -3´
3´ -GC TAG AAC CTC CTA CTA ATT TAC C TTAA -5´

The mixture was digested with EcoRI and the vector band purified after agarose gel electrophoresis. cDNA 84-2 was partially digested with EcoRI and the 1.7 kb cDNA band purified after agarose gel electrophoresis. The gel-purified 1.7 kb cDNA band and the EcoRI-digested vector band were ligated overnight and used to transform E. coli BL21/DE3. Tetracycline-resistant bacterial colonies were analyzed for those that contain the 55A cDNA in the proper orientation by antibody screening and by restriction enzyme digests of miniprep DNAs. E. coli cells containing the 55A cDNA in the proper orientation were induced to synthesize the 55A protein as described in Section II.I.3.

The recombinant 55A protein can be purified from extracts of the expression host cell by standard techniques of protein chemistry until the recombinant protein is sufficiently pure to be used in pharmaceutical preparations. In certain preferred embodiments, the procedures to be used for purification of the recombinant protein may include, but are not limited to, some or all of the following: ion exchange chromatography (e.g., Q-, S-, and DEAE-Sepharose ion exchange columns), gel permeation chromatography (e.g., Superose sizing columns), chromatofocusing (e.g., Mono-P columns), hydrophobic interaction chromatography (e.g., octyl- and phenyl-Sepharose HIC columns), affinity chromatography (e.g., zinc, copper, and mercury metal-affinity columns).

N. Isolation of the gene encoding the H. contortus 55A kDa protein

The H. contortus:EMBL-3 library was screened by plaque-hybridization with $^{32}$P-labeled cDNA 84-2 using procedures similar to those described in Section II.E.3. The hybridization and wash temperatures were 37°C. Duplicate nitrocellulose filters were screened with the 750 bp and 900 bp EcoRI fragments of cDNA 84-2. Eleven phages out of ~90,000 phages screened hybridized with the labeled probes and were plaque-purified. The phages have been names ^g MB7 to 17. Regions of the phages that hybridize to cDNAs 84-2 and 84-1 can be determined by restriction enzyme digest and Southern blot hybridization experiments. The 5′ to 3′ coding direction of the gene(s) can be determined using the known sequence of cDNA 84-2, e.g., the 750 bp EcoRI fragment encodes the 5′ half of the gene whereas the 900 bp EcoRI fragment encodes the 3′ half of the gene. Separate Southern blot experiments with these two labeled probes will allow the 5′ to 3′ direction of the gene to be determined. Known restriction enzyme sites within the cDNAs (e.g., an XhoI site near the 3′ end of cDNA 84-2 (Figure 48) also can be used to orient the 5′ ^$ 3′ coding direction of the 55A gene(s). Any coding sequences missing from cDNAs 84-1 and 84-2 can be determined by sequencing portions of the EMBL-3 phages that lie 5′ of where the cDNAs terminate.

O. Genomic Southern blot experiments with cDNAs encoding the 55A kDa protein

H. contortus genomic DNA was digested with EcoRI, SalI or BamHI, size-fractionated on agarose gels and blotted onto a nitrocellulose filter as described in Section II.F. Duplicate filters were hybridized with the two EcoRI fragments of cDNA 84-2. Filters were hybridized and washed at 32°C using solutions described in Section II.F. These experiments showed that both probes hybridized to multiple bands in each digest (Figure 50), suggesting that there is more than one gene encoding the 55A kDa protein in the H. contortus genome. This result is consistent with the finding that cDNAs 84-1 and 84-2 differ slightly in DNA sequence and suggests that these cDNAs may be products of two closely related, but distinct, genes.

P. Isolation of Ostertagia ostertagi cysteine protease genes

1. Isolation of DNA from Ostertagia ostertagi

Ensheathed third-stage larvae (SL3s) that had been frozen in liquid nitrogen and stored at -70C were obtained from Dr. R. Boisvenue (Eli Lilly and Company, Greenfield, IN). The worms were ground to a fine powder in a mortar and pestle over liquid nitrogen. The worms were then digested with Proteinase K at 65C as described in Section II(1). After alternating extractions with phenol and chloroform, the DNA was precipitated by adding one-tenth volume of 3M sodium acetate and 3 volumes of ethanol. High molecular weight DNA was spooled out using a glass capillary, air- dried and resuspended in 10 mM Tris-HCl pH 8.0, 1 mM EDTA (TE buffer). The DNA was treated with RNAse, extracted with phenol and chloroform, ethanol precipitated, dried and resuspended in TE buffer. Approximately 300μg of DNA was obtained.

2. Construction of an O. ostertagi:EMBL-3 library

This library was constructed essentially as described in Section II(1). One hundred micrograms of O. ostertagi DNA were partially digested with Sau3A and size-fractionated on a 10-40% sucrose gradient. Fractions containing DNA fragments of 15-20 kb were identified by agarose gel electrophoresis, pooled, and dialyzed versus 3X SET then TE buffer. The dialyzed DNA was precipitated with ethanol and collected by centrifugation (25,000 rpm for 30 min at 4C using an SW28 rotor). The DNA was resuspended in TE buffer. Approximately 2μg of size-fractionated DNA was obtained. Aliquots of this DNA were ligated to EcoRI/BamHI digested λEMBL-3 DNA (Stratagene Corporation), packaged in vitro (Gigapack Plus kits purchased from Stratagene Corporation) and plated on E. coli strain Q359.1. A total of 1X 10⁶ recombinant phage was obtained from several ligations and packagings. To create an amplified library, approximately 4.4X 10⁵ phage were plated on lawns of E. coli Q359.1 (25,000 phage per 15 cm diameter plate) until the plaques were visible but not overlapping. The plaques were overlaid with λ dil, left at 4°C overnight and liquid collected the next day. The titer of the amplified library was 1X 10¹⁰ pfu/ml.

3. Identification of O. ostertagi cysteine protease genes

The O. ostertagi:EMBL-3 library was screened by plaque hybridization (Benton and Davis, 1977) using a ³²P-labeled nick-translated plasmid containing the 1014 bp EcoRI fragment of H. contortus cDNA F-1 as a hybridication probe. The EcoRI fragment of cDNA F-1 is inserted into the EcoRI site of pBR325. The library was plated at a density of 8X 10³ phages per 10 cm diameter plate using E. coli LE392 as the host. Nitrocellulose filters were prehybridized for 60 min in a solution of 40% formamide (Kodak, Spectrograde)-/3X SET/0.1M sodium phosphate pH 7.4/0.1% sodium dodecyl sulfate/10µg ml⁻¹ sheared salmon sperm DNA. The filters were then hybridized overnight at 32°C in fresh hybridization buffer. The next day the filters were washed 4X 30 min with several hundred ml of fresh hybridization buffer, then 1-2X with 2X SET buffer. After overnight exposure to X-ray film, positive phages were picked and rescreened using similar procedures until plaque-pure. In some experiments nitrocellulose filters were prehybridized, hybridized and washed using solutions containing 30% formamide rather than 40% formamide. Lower formamide concentrations increased non-specific background hybridization. Screening of 10 plates yielded nine positive phages that were plaque-purified. These phages were named lambda 001-009. DNA from plaque-pure phages was obtained by the plate lysate technique (Davis et al., 1980). Restriction enzyme maps of the phages were prepared using single and double restriction enzyme digests (Figure 52). The restricted DNAs were size-fractionated on agarose gels, blotted onto nitrocellulose filters, the filters were baked at 80C, and hybridized to the ³²P-labeled F-1 cDNA plasmid using the hybridization conditions described above. Restriction fragments that hybridized to the plasmid were eluted from preparative agarose gels using NA-45 paper (Schleicher and Schuell), subcloned into M13 phage vectors and sequenced using the dideoxy termination method.

Regions of λ002, λ003, λ004 and λ007 that were sequenced are marked in Figures 52 and 53. The nucleotide sequences of these regions are presented in Figures 38 to 41. The O. ostertagi cysteine protease genes in these phages have identical intron/exon organizations as the H. contortus AC-2 gene (Figure 42). The deduced amino acid sequences of the O. ostertagi cysteine protease genes are shown in Figures 38 to 42. Ostertagia cysteine protease amino acid sequences are compared to the corresponding regions of two Haemonchus cysteine protease sequences in Figure 42. Haemonchus and Ostertagia cysteine proteases share ~70% amino acid sequence identity on average within the regions compared.

To obtain the complete sequence of O. ostertagia cysteine protease genes on λ001-009, one need only sequence the remainder of the phage DNAs that hybridize to H. contortus cDNAs F-1 or the F-1 exon 1-4 probe (sections II(E)(3) and II(L)) and compare the nucleotide and amino acid sequence to those of cDNAs F-1 to V-24. If a portion of the gene is not present in λ001-009, then restriction fragments from these phages can be used to screen the EMBL-3 library for new phages that extend further upstream or downstream of the coding sequences in λ001-009. The library can be rescreened by this method until a phage is identified that hybridizes to the missing regions of cDNAs F-1 or the F-1 exon 1-4 probe. Procedures for doing this are detailed in Section II.E.3., which describes the isolation of λMB3.

Alternatively, one can use the H. contortus cDNAs F-1 and V-24 or any of these O. ostertagia hybridizing regions to isolate cDNAs for cysteine proteases. Poly A(+) mRNA can be isolated from O. ostertagi XL3s, L4s or preferably from adults and used to make cDNA libraries using techniques described in section II (2). The cDNA libraries can be screened using ³²P- labeled F-1 or V-24 cDNAs or ³²P-labeled restriction fragments of O. ostertagi cysteine protease genes (purified from agarose gels) using hybridization conditions described in sections II(F) and II(K)(3). One can isolate full-length cDNAs for O. ostertagi cysteine proteases by rescreening the cDNA libraries using oligonucleotides from the 5′ ends of incomplete cDNAs as described in section II(E)(1).

Alternatively, it is possible to construct a "pseudo-cDNA" using synthetic oligonucleotides based upon the nucleotide and deduced amino acid sequences of the O. ostertagi genes. Another alternative method for constructing a "pseudo-cDNA" would be to delete the intron sequences by in vitro mutagenesis.

The O. ostertagi cysteine protease cDNAs or "pseudo-cDNAs" can then be expressed in an expression vector, such as those described in Section II(I), to cause synthesis of recombinant protein. The recombinant protein can be administered to cattle to protect them from infection with O. ostertagi.

Similar procedures to those described for H. contortus and O. ostertagi can be used to isolate cysteine protease genes from any parasitic nematode. Examples of parasitic nematodes for which cysteine protease genes can be used as vaccines include, but are not limited to, other Haemonchus species, other Ostertagia species, Trichostrongylus species, Cooperia species, Ascaris species, Toxocara species, hookworms (Ancylostoma and Necator species), filarial nematodes (Dirofilaria, and Brugia species) and Trichinella species.

Q. Isolation of O. ostertagi homologues of the H. contortus 55A, 55B and 55C kDa proteins

O. ostertagi (and any other parasitic nematode) genes encoding homologues of the H. contortus 55A, 55B and 55C kDa genes can be isolated from cDNA or genomic DNA phage libraries using cDNAs 84-1 and 84-2 (55A), 84-8 (55B), or 84-4 (55C) as hybridization probes. Suggested hybridization conditions for isolation of these genes are those described in Section II.P.3., which details the isolation of O. ostertagi cysteine protease genes using an H. contortus cysteine protease DNA probe (cDNA F1). Suggested pre-hybridization, hybridization and wash solutions should contain 30% or 40% formamide. Other reduced hybridization stringency conditions known to those skilled in the art also could be used successfully.

An example of the isolation of a 55 kd homologue from O. ostertagi using the H. contortus 55A (84-2) cDNA is shown in Figure 56. The figure depicts a map of an EMBL-3 genomic clone selected from an O. ostertagi library using the 55A cDNA. The cDNA was cut into 5$'$ and 3$'$ regions by restriction with EcoRI. The smaller fragment, 750 bp, represents the 5$'$ region of the clone; the longer fragment, 900 bp, represents the 3$'$ region. Each fragment was labeled and used as a separate hybridization probe.

The 55A probes were hybridized to sets of double lifts of an O. ostertagi EMBL-3 library as described in Section II.P.3. using 40% formamide at 32°C. Twelve initial positives, 0055A-1 through -12, cross-hybridizing with both probes, were selected and plaque purified. The map of O. ostertagi 55A-11 genomic clone shown in Figure 56 represents one homologue of the 55 kd protein from this species selected in this manner.

III. Collagen Peptide Examples

A. Collagen Peptide Protection Experiment

1. Experimental Design

Worm-free sheep approximately 12 months old were injected intramuscularly at several sites with 1 mg of the collagen peptide:KLH conjugate (as described in Section III(B) emulsified in Freund's complete adjuvant (vaccinated group) or with adjuvant only (control group). One month later the sheep were boosted by intramuscular injection of 0.5 mg of the collagen peptide:KLH conjugate emulsified in Freund's incomplete adjuvant (vaccinated group) or with adjuvant only (control sheep). Ten days later the sheep were challenged with 2500 Haemonchus contortus ensheathed third stage larvae (SL3s) given at a dose of 500 larvae per day for 5 days. The larvae were given by intraruminal injection. Fecal samples were collected at intervals over the next 2 months and the number of Haemonchus contortus eggs per several milligram samples determined. These numbers were converted to eggs per gram of feces (EPG) by multiplying by the appropriate conversion factor. At 56 days post-worm challenge the sheep were sacrificed and their abomasa removed and examined for the presence of adult worms. Any worms found were counted and sexed. Blood samples were collected prior to the test, post-antigen injection, pre-worm challenge and at necropsy. Serum samples were used to probe Western blots of total adult worm protein extracts to assay for the presence of antibodies reactive with worm collagens. Antihelmintic activity was scored by a reduction in worm egg production (EPG) and by a reduction in worm populations as necropsy.

2. Results

The EPGs and total worm counts in the vaccinated and control sheep are presented in Table 4. The vaccinated sheep exhibited reduced total worm counts at necropsy compared to the control, non-vaccinated sheep. The total worm counts at necropsy were 73% less in the vaccinated sheep when compared to the control sheep. The total worm counts between the two groups were significantly different (p >0.05) by statistical analysis.

3. Conclusions

We conclude from this study that the collagen peptide is immunogenic in sheep and can be used as a vaccine to protect sheep from Haemonchus contortus infections as determined by reduction of worm populations.

We examined representative nematodes from several other Orders and one other Class in the Phylum Nematoda for the presence of collagens with an antigenically related sequence. We examined two free-living nematodes (mixed-stage populations of C. elegans and Panagrellus redivivus), two insect parasitic nematodes (dauer-larvae of Heterorhabditis bacteriophora and Neoaplectana carpocapsae), and four animal parasitic nematodes (Ostertagia ostertagi SL3s, Dirofilaria immitis and Toxocara canis adults and Trichinella spiralis first-stage larvae). The phylogenetic relationship of these nematodes is shown in Figure 43. Each nematode possessed multiple high molecular weight proteins that reacted strongly with the antiserum (Figure 44). The pattern of reactive proteins was species-specific. With few exceptions (e.g., several proteins in P. redivivus), the reaction proteins were collagenase-sensitive. Coomassie-blue stained gels of these proteins samples indicated that the collagenase-sensitivity of the reacting proteins was specific and not due to generalized proteolysis (data not shown).

The fact that antisera raised against the peptide react with these nematodes indicates that the peptide can be used as a vaccine to protect mammals from infections caused by the nematodes and other nematodes in their Orders and Classes.

B. Nematode sources and extraction procedures

Haemonchus contortus SL3s and adults were harvested from sheep as described previously. Haemonchus contortus XL3s were obtained by exsheathing SL3s in vitro with $CO_2$ and allowing the XL3s to crawl through mulin filter rings. The XL3s were collected by centrifugation and either stored frozen or cultivated in vitro to the L4 stage. Ostertagia ostertagi SL3S were collected from the feces of experimentally infected calves using slight modifications of the procedures developed for Haemonchus contortus. Populations containing mixed developmental stages of C. elegans and Panagrellus redivivus were obtained from Susan Bektesh (Synergen, Inc.). Adult Dirofilaria immitis and Toxocara canis were kindly provided by Robert Grieve (Colorado State University). Trichinells spiralis L1 were kindly provided by Donald Wassom (University of Wisconsin). Dauer larvae (arrested third-stage larvae similar to the SL3 stage of Haemonchus contortus) of Heterorhabditis bacteriophora, and Neoaplectana carpocapsae were kindly provided by James White (BIOSYS Corporation). Worms were stored frozen at -70°C until use. The H. bacteriophora and N. carpocapsae worms sent to us were shipped at 0°C and stored at -70°C upon arrival.

Extracts of Haemonchus contortus larvae, P. redivivus and O. ostertagi were prepared by sonicating worms in 10mM Tris-HCl pH 7.4, 1mM ethylenediaminetetraacetate, 1mM phenylmethanesulfonyl fluoride, diluting the mixtures to final concentration of 1% SDS, 0.125 M Tris-HCl pH 6.8, 5% BME and boiling the samples for 2 mn. The mixed C. elegans population and larvae of H. bacteriophora and N. carpocapsae were boiled for 2 min in a solution of 1% SDS, 0.125 M Tris-HCl pH 6.8, 5% BME. Adult Haemonchus contortus, T. canis and D. immitis were broken open by chopping with a razor blade or grinding the frozen worms with a mortar and pestle over liquid nitrogen prior to boiling in 1% SDS, 0.125 M Tris-HCl pH 6.8, 5% BME. After boiling, the worms were shaken overnight at room temperature and the extracts stored at -20°C until use. Insoluble material, which is derived mainly from cuticles, was not removed from the extracts.

C. Peptide synthesis and coupling to KLH

An 18 amino acid long peptide with the sequence shown in Figure 45 was synthesized using an Applied Biosystems, Model 430A, peptide synthesizer. The peptide was coupled to KLH using a procedure developed by Suzanne Horvath and communicated to the present inventors by John Abelson. Ten mg of KLH was dissolved in 1 ml of PBS and reacted for 30 min at room temperature with 2400 nanomoles of succinimidyl 4-(N-malaimido-methyl) cyclohexane-1 carboxylate (Pierce). Twelve mg of peptide was dissolved in 300 μl of 4 M guanidine hydrochloride:PTS pH7.5, reduced with dithiothreitol for 30 min at 37° and the pH of the solution adjusted to 3-4 with 17% $H_3PO_4$. The modified KLH and reduced peptide were then layered on top of a 5 ml spin column of Sephadex G-10 equilibrated with 4 M guanidine hydrochloride:PBS pH 7.5. The column was spun for 1 min at setting 6 in an IEC clinical centrifuge, washed with 300 μl of 4 M guanidine hydrochloride:PBS pH 7.5, and spun again. The KLH:peptide conjugate in the flow-through was stored at 4°C. A recovery of 8 mg of KLH was assumed and used in the calculation to determine the immunizing doses for the rabbits. The coupling efficiency of the peptide to KLH was not determined.

REFERENCES

Biggen, M.D., Gibson, T.J. and Hong, G.F. (1983)
Proc. Natl. Acad. Sci. USA
80, 3963-3965.
Benton, W.D. and Davis, R.W. (1977)
Science
196, 180-182.
Carne, A. and Moore, C.H. (1978)
Biochem. J.
173, 73-83.
Casadaban, M.J., Martinez-Arias, A., Shapira, S.K. and Chou, J. (1983)
Meth. Enzymology
100, 283-308.
Chan, S.J., Segundo, B.S., McCormick, M.B. and Steiner, D.F. (1986)
Proc. Natl. Acad. Sci. USA
83, 7721-7725.
Chirgwin, J.M., Przybyla, A.E., MacDonalds, R.J. and Rutter, W.J. (1979)
Biochemistry
18, 5294-5299.
Clewell, D.B. (1972)
J. Bacteriology
110, 667-676.
Cohen, L.W., Coghlan, V.M. and Dihel, L.C. 91986)
Gene
48, 219-227.
Davis, R.W., Botstein, D. and Roth, J. (1980)
Advanced Bacterial Genetics.
Cold Spring Harbor Laboratory, Cold Spring Harbor, N.Y.
Efstratiadis, A. and Kafatos, F.C. (1976)
Methods in Molecular Biology
8, 1-124.
Frischauf, A., Lehrach, H., Poustka, A. and Murray, N. (1983)
J. Mol. Biol.
170, 827-842.
Grunstein, M. and Hogness, D. (1975)
Proc. Natl. Acad. Sci. USA
72, 3961.
Guarente, L., Lauer, G., Roberts, T.M. and Ptashne, M. (1980)
Cell
20, 543-553.
Holmes, D.S. and Quigley, M. (1981)
Anal. Biochem.
114, 193.
Hotez, P.J., Trang, N.L., McKerrow, J.H. and Cerami, A. (1985)
J. Biol. Chem.
260, 7343-7348.
Laemmli, U.K. and Favre, M. (1973)
J. Mol. Biol.
80, 575- 599.
Lehrach, H., Diamond, D., Wozney, J.M. and Boedtker, H. (1977)
Biochemistry
16, 4743-4751.
Maxam, A.M. and Gilbert, W. (1980)
Meth. Enzymology
65, 499- 560.
Rigby, P.W.J., Dieckmann, M., Rhodes, C. and Berg, P. (1977)
J. Mol. Biol.

113, 237-251.

Sanger, F., Nicklen, S. and Coulson, A.R. (1977)

Proc. Natl. Acad. Sci. USA

74, 5463-5467.

Takio, K., Towatari, T., Katunuma, N., Teller, D.C. and Titani, K. (1983)

Proc. Natl. Acad. Sci. USA

80, 3666-3670.

Towbin, H., Staehelin, T. and Gordon, J. (1979)

Proc. Natl. Acad. Sci. USA

76, 4350-4354.

Wada, K., Takai, T. and Tanabe, T. (1987)

Eur. J. Biochem.

167, 13-18.

Young, R.A. and Davis, R.W. (1983)

Science

80, 1194-1198.

## TABLE II
## Anti-coagulant Protection Experiment #1

| | Worm Egg Counts Per Gram of Feces at Days Post-Worm Challenge | | | | | | | | Total Worm Counts[1] |
|---|---|---|---|---|---|---|---|---|---|
| Lamb No. | 0 F/M | 21 | 28 | 35 | 42 | 49 | 56 | Total | |
| Group 1: (Anticoagulant Antigen) | | | | | | | | | |
| 359 | 0 | 0 | 0 | 100 | 0 | 100 | 0 | 14 | 6/8* |
| 363 | 0 | 0 | 100 | 500 | 400 | 800 | 900 | 442 | 55/45 |
| 381 | 0 | 0 | 0 | 100 | 300 | 200 | 0 | 10 | 6/4* |
| 385 | 0 | 0 | 0 | 0 | 0 | 0 | 0 | 10 | 6/4* |
| | (0) | (0) | (25) | (175) | (175) | (275) | (225) | (119) | |
| Group 2: (Controls) | | | | | | | | | |
| 268 | 0 | 0 | 0 | 100 | 0 | 100 | 200 | 103 | 55/45 |
| 367 | 0 | 0 | 500 | 300 | 500 | 600 | 1300 | 700 | 55/45 |
| 375 | 0 | 0 | 200 | 300 | 700 | 800 | 500 | 872 | 55/45 |
| 384 | 0 | 0 | 700 | 1300 | 1800 | 1600 | ** | N.A. | -- |
| | (0) | (0) | (350) | (500) | (750) | (775) | (666) | (558) | |

\* Actual worm counts; other data are percentages.

\*\* Died.

F/M = female/male worms

(n) = group average

[1] Necropsy at 56 days post-worm challenge. {{#TxtRmF3txtBOLDLOFF}}

EP 0 434 909 A2

## TABLE III
## Anticoagulant Protection Experiment #2

| | Worm Egg Counts Per Gram of Feces at Days Post-Worm Challenge | | | | | Total Worm Counts[1] | | | |
|---|---|---|---|---|---|---|---|---|---|
| Lamb No | 0 | 21 | 28 | 35 | 42 | 49 | 56 | Total | F/M |
| **Group 1:** (Anticoagulant Antigen-IM) | | | | | | | | | |
| Block 1 | | | | | | | | | |
| 261 | 0 | 0 | 0 | 100 | 100 | 0 | 0 | 30 | 18/12 |
| 331 | 0 | 0 | + | + | 0 | 0 | 0 | 81 | 48/33 |
| Block 2 | | | | | | | | | |
| 254 | 0 | 0 | + | 0 | 0 | 0 | 0 | 17 | 10/7 |
| 240 | 0 | 0 | 0 | 100 | 0 | 0 | 0 | 22 | 20/2 |
| | (0) | (0) | (0) | (50) | (25) | (0) | (0) | (37) | |
| **Group 2:** (Controls) | | | | | | | | | |
| Block 1 | | | | | | | | | |
| 336 | 0 | 0 | + | + | 100 | 500 | 500 | 249 | 133/116 |
| 237 | 0 | 0 | 400 | 100 | 200 | 300 | 100 | 205 | 117/88 |
| Block 2 | | | | | | | | | |
| 255 | 0 | 0 | 600 | 800 | 500 | 700 | 600 | 496 | 280/216 |
| 238 | 0 | 0 | 100 | 300 | 600 | 200 | 100 | 127 | 66/61 |
| | (0) | (0) | (275) | (300) | (350) | (425) | (325) | (269) | |
| **Group 3:** (Anticoagulant) – IV/IM | | | | | | | | | |
| Block 1 | | | | | | | | | |
| 251 | 0 | 0 | 0 | 100 | 100 | 100 | 100 | 74 | 43/31 |
| 229 | 0 | 0 | 0 | 0 | + | 0 | 100 | 32 | 17/15 |
| Block 2 | | | | | | | | | |
| 330 | 0 | 0 | 0 | 300 | 400 | 700 | 700 | 454 | 286/168 |
| 232 | 0 | 0 | + | + | 100 | 100 | 200 | 39 | 21/18 |
| | (0) | (0) | (0) | (100) | (150) | (225) | (275) | (150) | |

[1] Necropsy 56 days

NEG = Nematodiriasis

+ = Positive, less than 100 EPG

IV/IM = Intravenous/intramuscular

(a) = Group average

EP 0 434 909 A2

**TABLE IV**

**Anticoagulant Protection Experiment #3**

| Treatment Group | Worm Count at Necropsy | | Last Egg Count |
|---|---|---|---|
| (EPG) | Normal Adults | Stunted Adults[a] | (EPG) |
| A. Anticoagulant + Adjuvant (~725 g injected per sheep) | | | |
| Sheep #650 | 0 | 0 | 0[b] |
| 657 | 0 (197) | 400 | 300[b] (350) |
| 640 | 0 | 0 | 0 |
| 843 | 788 | 0 | 1400 |
| B. Anticoagulant + SDS + Adjuvant (~725 g injected per sheep) | | | |
| Sheep #655 | 0 | 0 | 0 |
| 849 | 0 (336) | 7 | 0 (250) |
| 645 | 0 | 104 | 0 |
| 647 | 1344 | 0 | 1000 |
| C. Adjuvant | | | |
| Sheep #651 | 0 | 26 | 0 |
| 682 | 0 (636) | 0 | 0 (925) |
| 642 | 1504 | 0 | 2800 |
| 652 | 1040 | 0 | 2900 |
| D. Adjuvant + SDS | | | |
| Sheep # 653 | 1442 | 0 | 2900 |
| 656 | 1272 (739) | 0 | 1500 (1350) |
| 646 | 243 | 0 | 1000 |
| 649 | 0 | 14 | 0 |
| E. 35 kDa band - SDS gel purified (~200 g injected per sheep) | | | |
| Sheep # 654 | 0 | 0 | 0 |
| 681 | 0 (232) | 0 | 0 (550) |
| 639 | 928 | 0 | 2200 |
| 857 | 0 | 384 | 600[b] |
| F. 55 kDa band - SDS gel purified (~425 g injected per sheep) | | | |
| Sheep #673 | 0 | 0 | 0 |
| 842 | 0 (186) | 78 | 0 (275) |
| 641 | 744 | 0 | 1100 |
| 648 | 0 | 298 | 0 |

[a] Adults were small, infertile and bluish, not red, in color.
[b] Eggs were infertile.
[c] (n) = group means

**Claims**

1. A surface protein identified by $^{125}$I-surface labeling studies and solubilized from XL3s by 1% SDS, comprising at least one of the group consisting of a 24a kDa XL3 surface protein, a 26 kDa XL3 surface protein, a 30 kDa XL3 surface protein, a 36 kDa XL3 surface protein, a 68-97 kDa XL3 surface protein and a 180 kDa XL3 surface protein.

2. A surface protein identified by surface labeling studies and solubilized from XL3s by 1% SDs and 5% BME, comprising at least one of the group consisting of a 24b kDa XL3 surface protein and a 30b kDa XL3 surface protein.

3. A surface protein identified by $^{125}$I-labeling studies and solubilized from worms by 1% SDS, comprising at least one of the group consisting of a 27 kDa L4 surface protein, a 29 kDa L4 surface protein, a 36 kDa L4 surface protein, a 78 kDa L4 surface protein, a 200 kDa L4 surface protein, a 16 kDa L4 surface protein, a 18 kDa L4 surface protein, a 19 kDa L4 surface protein, a 42 kDa L4 surface protein, a 54 kDa L4 surface protein and a 93 kDa L4 surface protein.

4. A surface protein identified by $^{125}$I-surface labeling studies and solubilized from L4s by 1% SDS and 5% BME comprising a 180 kDa L4 surface protein.

5. A method for purifying surface proteins from parasitic nematodes comprising boiling parasitic nematodes in 1% SDS or 100mM NaCl in a buffer, centrifuging out the worms and concentrating the surface proteins in the supernatant liquid.

6. An extract for protecting sheep from Haemonchus contortus prepared by the process comprising homogenizing Haemonchus contortus worms in buffer, size-fractioning soluble proteins resulting from said homogenization on molecular weight sizing columns and subsequently performing FPLC Mono Q column chromatography.

7. An extract as claimed in claim 6, wherein said extract exhibits the ability to degrade fibrinogen in a fibrinogen-degradation assay.

8. A Haemonchus contortus protein obtained from the extract according to claim 6.

9. A Haemonchus contortus protein as in claim 8 selected from the group consisting of a 35 kDa protein, a 37 kDa protein and a 55 kDa protein.

10. A Haemonchus contortus protein corresponding to at least one of the proteins in the extract according to claim 6 selected from the group consisting of a 35 kDa protein, a 37 kDa protein and a 55 kDa protein.

11. A Haemonchus contortus protein as in claim 10, wherein said protein is obtained by recombinant DNA methods.

12. A Haemonchus contortus cysteine protease, comprising a protease encoded by a gene or cDNA that cross-hybridizes to DNA sequences of AC-1, AC-2, AC-3 and AC-4, produced by recombinant DNA methods.

13. An Ostertagia ostertagi cysteine protease encoded at least in part by DNA sequences present in λ001-λ009 produced by recombinant DNA methods.

14. An Ostertagia ostertagi cysteine protease that cross-hybridizes to Haemonchus contortus genes AC-1, AC-2, AC-3, AC-4 or to Ostertagia ostertagi cysteine proteases present in λ001-λ009 produced by recombinant DNA methods.

15. An Ostertagia ostertagi protein that reacts with antisera prepared against at least one of the Haemonchus contortus proteins present in the extract according to claim 6.

16. A protein as in claim 15, wherein said protein reacts with antisera prepared against at least one of Haemonchus contortus 35 kDa proteins and 55 kDa protein.

17. A parasitic nematode cysteine protease encoded by a gene or cDNA that cross-hybridizes to Haemonchus contortus genes AC-1, AC-2, AC-3, AC-4 or to Ostertagia ostertagi cysteine proteases present in λ001-λ009 produced through recombinant DNA methods.

18. A parasitic nematode protein that reacts with antisera prepared against Haemonchus contortus proteins in the extracts according to Claim 6.

19. A protein as in claim 18, wherein said protein reacts with antisera prepared against at least one of the Haemonchus contortus 35 kDa and 55 kDa proteins.

20. A protein as in claim 19, wherein said protein is obtained by recombinant DNA methods.

21. Peptide sequences of proteins of the extract according to claim 6 that cause production of antibodies that bind to the proteins of the extract according to claim 6.

22. Antisera or monoclonal antibodies that bind to the proteins or peptide sequences of proteins of the extract according to claim 6 and which can be used therapeutically or prophylactically to protect plants, animals or humans against parasitic nematode infections.

23. A Haemonchus contortus 55 kDa protein comprising a protein encoded at least in part by cDNAs 84-1, 84-2, 84-4 or 84-8, produced through recombinant DNA methods.

24. A Haemonchus contortus 55 kDa protein, comprising a protein encoded at least in part by a gene or cDNA that cross-hybridizes to cDNAs 84-1, 84-2, 84-4 or 84-8, produced through recombinant DNA methods.

25. A Haemonchus contortus 55 kDa protein encoded at least in part by DNA sequences present in λMB7-17.

26. A parasitic nematode protein encoded by a gene or cDNA that hybridizes to DNA sequences encoding the Haemonchus contortus 55A, 55B or 55C proteins, produced through recombinant DNA methods.

27. A Haemonchus contortus cysteine protease encoded at least in part by DNA sequences present in λMB4-6, produced through recombinant DNA methods.

28. Organic molecules that bind to or inhibit the enzymatic activity of the proteins of Claim 6 and which can be used prophylactically or therapeutically to protect animals, plants or humans against nematode infections.

29. A method for vaccinating an animal against nematodes comprising administering to the animal an effective dose of substantially pure collagen peptide having a sequence corresponding to nematode collagen peptide and a pharmaceutically acceptable carrier.

# FIG. 1

# FIG. 2

FIG. 3

# FIG. 4

# FIG. 5

# FIG. 6

# FIG. 7

# FIG. 8

FIG. 9

# FIG. 10

FIG. II

# FIG. 12

# FIG. 13

FIG. 14

# FIG. 15(1)

```
  1   ATG AAA TAC TTG GTG CTT GCA CTT TGC ACC TAT CTT TGT TCC CAA ACC GGA GCA
  1   Met Lys Tyr Leu Val Leu Ala Leu Cys Thr Tyr Leu Cys Ser Gln Thr Gly Ala

 55   GAC GAG AAT GCT GCC CAA GGA ATT CCT CTA GAA GCG CAA AGG TTG ACT GGT GAG
 19   Asp Glu Asn Ala Ala Gln Gly Ile Pro Leu Glu Ala Gln Arg Leu Thr Gly Glu

109   CCG CTT GTG GCT TAC CTT AGG AGG AGT CAG AAC CTC TTC GAG GTC AAT TCA GCT
 37   Pro Leu Val Ala Tyr Leu Arg Arg Ser Gln Asn Leu Phe Glu Val Asn Ser Ala

163   CCG ACA CCT AAT TTC GAG CAA AAA ATA ATG GAC ATA AAA TAC AAA CAT CAG AAG
 55   Pro Thr Pro Asn Phe Glu Gln Lys Ile Met Asp Ile Lys Tyr Lys His Gln Lys

217   CTA AAT CTT ATG GTC AAA GAA GAT CCC GAC CCT GAA GTG GAT ATC CCA CCC AGC
 73   Leu Asn Leu Met Val Lys Glu Asp Pro Asp Pro Glu Val Asp Ile Pro Pro Ser

271   TAC GAT CCT CGA GAT GTC TGG AAA AAC TGC ACT ACG TTC TAT ATT CGC GAT CAA
 91   Tyr Asp Pro Arg Asp Val Trp Lys Asn Cys Thr Thr Phe Tyr Ile Arg Asp Gln
                                                ---  -------

325   GCC AAT TGC GGC TCA TGT TGG GCT GTT TCC ACG GCA GCT GCA ATT TCA GAT CGT
109   Ala Asn Cys Gly Ser Cys Trp Ala Val Ser Thr Ala Ala Ala Ile Ser Asp Arg

379   ATT TGC ATT GCA AGC AAA GCT GAA AAA CAG GTG AAT ATT TCT GCC ACT GAC ATC
127   Ile Cys Ile Ala Ser Lys Ala Glu Lys Gln Val Asn Ile Ser Ala Thr Asp Ile
                                                        ---  -- - - - --

433   ATG ACG TGC TGC AGG CCA CAG TGC GGT GAC GGG TGT GAA GGA GGA TGG CCT ATC
145   Met Thr Cys Cys Arg Pro Gln Cys Gly Asp Gly Cys Glu Gly Gly Trp Pro Ile

487   GAA GCT TGG AAA TAC TTC ATA TAT GAC GGC GTT GTT TCT GGA GGA GAA TAC CTC
163   Glu Ala Trp Lys Tyr Phe Ile Tyr Asp Gly Val Val Ser Gly Gly Glu Tyr Leu

541   ACT AAA GAT GTA TGC CGC CCT TAT CCA ATT CAC CCA TGT GGA CAT CAC GGA AAC
181   Thr Lys Asp Val Cys Arg Pro Tyr Pro Ile His Pro Cys Gly His His Gly Asn
                                                                        ---
```

EP 0 434 909 A2

# FIG. 15(2)

```
595   GAC ACC TAC TAC GGG GAA TGC CGT GGA ACA GCG CCA ACC CCA CCG TGC AAA AGG
199   Asp Thr Tyr Tyr Gly Glu Cys Arg Gly Thr Ala Pro Thr Pro Pro Cys Lys Arg
      - - - - - -

649   AAA TGC CGG CCC GGC GTT AGG AAA ATG TAC AGG ATA GAC AAG CGA TAC GGA AAA
217   Lys Cys Arg Pro Gly Val Arg Lys Met Tyr Arg Ile Asp Lys Arg Tyr Gly Lys

703   GAC GCC TAC ATC GTG AAA CAG TCG GTT AAA GCC ATT CAG AGT GAA ATA CTA AGG
235   Asp Ala Tyr Ile Val Lys Gln Ser Val Lys Ala Ile Gln Ser Glu Ile Leu Arg

757   AAT GGA CCG GTT GTG GCC TCG TTT GCC GTC TAT GAA GAT TTC AGG CAC TAC AAA
253   Asn Gly Pro Val Val Ala Ser Phe Ala Val Tyr Glu Asp Phe Arg His Tyr Lys

811   TCA GGA ATT TAT AAG CAC ACA GCT GGT GAG CTA CGA GGT TAC CAT GCT GTA AAG
271   Ser Gly Ile Tyr Lys His Thr Ala Gly Glu Leu Arg Gly Tyr His Ala Val Lys

865   ATG ATT GGA TGG GGA AAT GAA AAT AAT ACA GAC TTC TGG CTC ATT GCC AAC TCT
289   Met Ile Gly Trp Gly Asn Glu Asn Asn Thr Asp Phe Trp Leu Ile Ala Asn Ser
                              - - - - - - - -

919   TGG CAC AAC GAT TGG GGA GAA AAA GGG TAT TTC CGC ATA ATT CGC GGA ACT AAC
307   Trp His Asn Asp Trp Gly Glu Lys Gly Tyr Phe Arg Ile Ile Arg Gly Thr Asn

973   GAC TGC GGA ATT GAG GGA ACC ATA GCC GCT GGG ATT GTC GAC ACA GAA AGT CTA
325   Asp Cys Gly Ile Glu Gly Thr Ile Ala Ala Gly Ile Val Asp Thr Glu Ser Leu
                                                          ▼

1027  TGA   TATTGCACCATGTCAAAGCAAACTGAATAAATTTACTAATTGTATGTGCATGCAGAATCGTACTTC
      *

1096  GAAGAAGCCTCTGTAATGTATTCGATAAAAGCTGGATTACA₁₈
```

EP 0 434 909 A2

# FIG. 16

kb

1.77 —
1.52 —
1.28 —

0.78 —

0.53 —

0.40 —

# FIG. 17(1)

```
  1    MKYLVLALCTYLCSQTGADENAAQGIPLEAQRLTGEPLVAY.LRRSQNLF
       *  *    **  *       *               *  ** *    *
  1    MWQLWASLCCLLVLAN.A......RSRPSFHPVSDE.LVNYVNKRNTTWQ
            signal        A              pro


 50    EV.NSAPTPNFEQKIMDIKYKHQ.KLNLMVKEDPDPEVDIPPSYDPRDVW
         *            *       *    *      *    * * * * *
 43    AGHNFYNVDMSYLKRLCGTFLGGPKPPQRVMFTEDLK..LPASFDAREQW
                                          A   mature


 97    KNC.TTFYIRDQANCGSCWAVSTAAAISDRICIASKAEKQVNISATDIMT
       * *    ****  ******    ********   *   *  ** *   *
 91    PQCPTIKEIRDQGSCGSCWAFGAVEAISDRICIHTNAHVSVEVSAEDLLT


147    CCRPQCGDGCEGGWPIEAWKYFIYDGVVSGGEYLTKDVCRPYPIHPCGHH
       **    *****  **  * ***       *  ****  *    **** *  **  **
141    CCGSMCGDGCNGGYPAEAWNFWTRKGLVSGGLYESHVGCRPYSIPPCEHH
```

EP 0 434 909 A2

# FIG. 17(2)

```
197  GNDTYYGECRGTAPTPPCKRKCRPGVRKMYRIDKRYGKDAYIVKQSVKAI
      *       * *    ** *    * **    * ** **    * *  * * *
191  VNGSRPP.CTGEGDTPKCSKICEPGYSPTYKQDKHYGYNSYSVSNSEKDI


247  QSEILRNGPVVASFAVYEDFRHYKSGIYKHTAGELRGYHAVKMIGWGNEN
      **  ****    * ** **  **** * *   **   * **    *** **
240  MAEIYKNGPVEGAFSVYSDFLLYKSGVYQHVTGEMMGGHAIRILGWGVEN


297  NTDFWLIANSWHNDWGEKGYFRIIRGTNDCGIEGTIAAGIVDTESL
      *  ** ****  ***  * * * **   ****    ***   *
290  GTPYWLVANSWNTDWGDNGFFKILRGQDHCGIESEVVAGIPRTDQYWEKI
                                                    ▲
```

# FIG. 18

# FIG. 19

| | | | | | | | | | | | | | | | | | | |
|---|---|---|---|---|---|---|---|---|---|---|---|---|---|---|---|---|---|---|
| AC-1 | Y | I | R | D | Q | A | N | C | G | S | C | W | A | V | S | T | A | A | A |
| Cathepsin B | E | I | R | D | Q | G | S | C | G | S | C | W | A | F | G | A | V | E | A |
| Papain | P | V | K | N | Q | G | S | C | G | S | C | W | A | F | S | A | V | V | T |

# FIG. 20

EP 0 434 909 A2

# FIG. 21(I)

```
•1                                                               •50
GTTCCGCACAGGATGAACATATTTGGTTTAAAGTTATAGCATCTGCAAAAATTTGCACCACTTTGAACATAATAAACACAGGA

          •100                                                    •150
TTGAATTAGAGAACTACTTCAGAGCAAAGGAATGCATTAAACAAAACTTTCACTCTTTGCAACCCTTTCTTCGGCAGATTCCT

                         •200
CTCGCTTTCTTCTTATCATAGACAAAGTAGTTATCTTTGACAGGTGGTTAATACCAATATAAGCAGAATTCGACATCTCAAAA

•250              ▼                                 •300
ATCGGAGGCAAT ATG gtaagttacctcgctgactttcttttacaataattttttttccatttggattgaacggcagattgaa
             Met                                   Intron 1
                  •350                                           C      A
gatatatgtttcag AAA TAC TTG GTG CTT GCA CTT TGC ACC TAT CTT TGT TCA CAA TCC GGA GCA
               Lys Tyr Leu Val Leu Ala Leu Cys Thr Tyr Leu Cys Ser Gln Ser Gly Ala
                                                                           Thr
     •400                                          •450
GAC GAG AAT G gttggtaacaatttctggcccataactaatgtaattgagagaacatacttggtcgcttacctcttacag
Asp Glu Asn A                                      Intron 2
                  •500
CT GCC CAA GGA ATT CCT CTA GAA GCG CAA AGG TTG ACT GGT GAG CCG CTT GTG GCT TAC CTT
la Ala Gln Gly Ile Pro Leu Glu Ala Gln Arg Leu Thr Gly Glu Pro Leu Val Ala Tyr Leu
```

EP 0 434 909 A2

# FIG. 21(2)

```
         •550                                                              •600
AGG AGG AGT CAG AAC CTC TTC GAG gtgattcattctgtaaacagaaatttctcatccaaaatcttaatccttcct
Arg Arg Ser Gln Asn Leu Phe Glu                                        Intron 3


                C            A            •650            GA
tttgcag GTC AAT TCA GAT CCG ACA CCT GAT TTC GAG CAA AAA ATA ATG AGC ATA AAA TAC AAA
        Val Asn Ser Asp Pro Thr Pro Asp Phe Glu Gln Lys Ile Met Ser Ile Lys Tyr Lys
                Ala          Asn                         Asp


                         •700                                   EcoRV
CAT CAG AAG CTA AAT CTT ATG GTC AAA GAA GAT CCC GAC CCT GAA GTG GAT ATC CCA CCC AG
His Gln Lys Leu Asn Leu Met Val Lys Glu Asp Pro Asp Pro Glu Val Asp Ile Pro Pro Se


        •750
gtgagtacctctcttgtcaatcgcctcact.....................ggttaaagaaaatagatttacag C TAC
                 Intron 4  ~5.2 kb                                       r Tyr


    •6000        T                       T                    T   6050•    T
GAT CCT CGA GAC GTC TGG AAA AAC TGC ACA ACG TTC TAT ATT CGC GAC CAA GCC AAC TGC G
Asp Pro Arg Asp Val Trp Lys Asn Cys Thr Thr Phe Tyr Ile Arg Asp Gln Ala Asn Cys G
                                                                         -----

                         •6100
gtgagctcagctgcaagagataccaatgactccgaaaaattcaattcggaaagagttagaatttttcag GC TCA TGT TGG
                 Intron 5                                             ly Ser Cys Trp
                                                                      --------------
```

# FIG. 21(3)

```
       •6150              A        T
GCT GTT TCC ACG GCA GCT GCA ATT TCG GAT CGC ATT TGC ATT GCA AGC AAA GCT GAA AAA CAG
Ala Val Ser Thr Ala Ala Ala Ile Ser Asp Arg Ile Cys Ile Ala Ser Lys Ala Glu Lys Gln
---
•6200                                              •6250
gtgcaagttaatctgtgatataataagcaatcgtttacttcaatgtgcaacatttag GTG AAT ATT TCT GCC ACT
        Intron 6                                           Val Asn Ile Ser Ala Thr
                            •6300
GAC ATC ATG ACG TGC TGC AGG CCA CAG TGC GGT GAC GG gtaaaatttcgtagtattgactctcagagcacg
Asp Ile Met Thr Cys Cys Arg Pro Gln Cys Gly Asp Gl


     •6350                                       •6400
gaaataagtatatagccccatttgtcttaataattcattgtttcag G TGT GAA GGA GGA TGG CCT ATC GAA GCT
        Intron 7                                  y Cys Glu Gly Gly Trp Pro Ile Glu Ala


                              •6450
TGG AAA TAC TTC ATA TAT GAC GGC GTT GTT TCT GGA GGA GAA TAC CTC ACT AAA gtgagataacta
Trp Lys Tyr Phe Ile Tyr Asp Gly Val Val Ser Gly Gly Glu Tyr Leu Thr Lys


     •6500                                          •6550
ttcctaatattatttgccgaaatgttctagcagtcttgacattag GAT GTA TGC CGC CCT TAT CCA ATT CAC
        Intron 8                                 Asp Val Cys Arg Pro Tyr Pro Ile His


                                   •6600
CCA TGT GGA CAT CAC GGA AAC GAC ACC TAC TAC GGG GAA TGC CGT GGA ACA GCG CCA ACC CCA
Pro Cys Gly His His Gly Asn Asp Thr Tyr Tyr Gly Glu Cys Arg Gly Thr Ala Pro Thr Pro


                         •6650
CCG TCC AAA AGG AAA TGC CGG CCC GGC GTT AGG AAA ATG TAC AGG ATA GAC AAG CGA TAC G
Pro Cys Lys Arg Lys Cys Arg Pro Gly Val Arg Lys Met Tyr Arg Ile Asp Lys Arg Tyr G
```

EP 0 434 909 A2

# FIG. 21(4)

```
                6700                                              •6750
gtaagcgtaagatctatcagatagtaacgttagaaatgcagtatctgaaaggaagcttggtccttcttcaaagcaaaaaataa
                          Intron 9


                      •6800
atgcaaacaagtaaacaaaacttagagaaattgttgctattagtcatgacagtagaagcaggtcactcgggtattaacagcac


    •6850                                         •6900
gtcacgaggccgattggagaagtttttgttgaaaaaatatgtggggatctgtgatctttacaatctaatcttctttttctcagc


          •6950                                              •7000
aaatgatggaagcgattagtgcagcatgttaaagacagagttcacccggatgtttgacactcgaatcataaattggaaaccac


                      •7050
acaggtttccaatttatgattcgaaaacacacgaaaccacacacaaccacatgaggttcttcagttagctattcatttgtgaa


    •7100                                         •7150
acgtgactaaatacattgcctcggcttcttgtatgatgtggtgtttatcaaaagttaaccaatgatctgccgcttcatgatat


                  •7200                                        G       •7250
gcgcttgaacggatttcttcgagcttcgtagagattgaattttttag GA AAA GAC GCC TAC ATC GTA AAA CAG
                                                  ly Lys Asp Ala Tyr Ile Val Lys Gln


                                    G                   7300•  C
TCG GTT AAA GCC ATT CAG AGT GAA ATA CTA AAG AAT GGA CCG GTT GTG GCT TCG TTT GCC GTC
Ser Val Lys Ala Ile Gln Ser Glu Ile Leu Lys Asn Gly Pro Val Val Ala Ser Phe Ala Val
                                    Arg
```

# FIG. 21(5)

•7350
TAT GAA GAT TTC AGG CAC TAC AAA TCA GGA ATT TAT AAG gcgagtcttctaaataaacgtcagttctaac
Tyr Glu Asp Phe Arg His Tyr Lys Ser Gly Ile Tyr Lys

•7400                                                                    •7450
tgttagtattcgcaagcaacagcttgccattgtgaattcgcttggaaaaaagagctcattgaaaatgcaatctatatgcatcc
                                                    Intron 10

                        •7500
agatatgatctatacaatctgcacagaaagttgcggaaaagaaaatagaatgtcagcaagagatgattatag CAC ACA GCT
                                                                           His Thr Ala

•7550              T                                          •7600
GGT GAG CTA CGA GGG TAC CAT GCT GTA AAG ATG ATT GGA TGG GGA AAT GAA AAT AAT ACA GAC
Gly Glu Leu Arg Gly Tyr His Ala Val Lys Met Ile Gly Trp Gly Asn Glu Asn Asn Thr Asp

                                        •7650
TTC TGG CTC ATT GCC AAC TCT TGG CAC AAC GAT TGG GGA GAA AAA G gtaagtcactcatgccaaaagt
Phe Trp Leu Ile Ala Asn Ser Trp His Asn Asp Trp Gly Glu Lys G

        •7700                                          •7750
gtttgtacatttgtactttgcttggttgtcgcagttttcttacgcgacccatagattaggccagtacctgaagcaacgccgcca
                Intron 11
                                        •7800
ccatacaccacgccggctcagaaacagcagaaacacgctgcttctgtccatttttcgagagaggtaagactatggtccaatcga

EP 0 434 909 A2

# FIG. 21(6)

```
    •7850            G                     A              C           C  7900•      G           A
tagctattttcag GA TAT TTC CGC ATA GTT CGC GGA AGT AAC GAC TGT GGA ATT GAA GGA ACC ATC
             ly Tyr Phe Arg Ile Val Arg Gly Ser Asn Asp Cys Gly Ile Glu Gly Thr Ile
                             Ile           Thr

                                                  •7950        A       C           T
GCC GCT GGG ATT GTC GAC ACA GAA AGT CTA TGA TATTGCACCATGTCACAGCAAAATGAATAAATTAACTAAT
Ala Ala Gly Ile Val Asp Thr Glu Ser Leu  *

                                                         A         A •8050
TG        C•8000        C  A
CATATGTGCATGTAGAATCGTACTTTGAGGAAGCCTCTGTAATGTATTCGATGAAAGCTGGGTTACATCGTATTGCACGTGCTG
                                                                        ▲
                      •8100                                                      •8150
AGGAGCCTAATTGGATTTTTTGACGATGTTCAACACATAGATGATCGCAGGGGCTGTATTGCTACTGCTCACTGAAGATGGTTG

                                          •8200
AACATACAGACGCGCTCAGAATATTCGGCGAAATGCTTCGTCAGTTATGCTTTTCAGTCGTAACTCCTTTCGTACTGAACCTAA

          •8250                                      •8300
TCATGCGAAATTTCAATATGTGCAGCTTGGGTGTAGTGTGAATATCTACCACTATTAACCTGCAATCTGTTCATGTTCCAGATA

                  •8350                          •8378
TCAAAAACGGCTTGAAAAATCAGCTGGAAACACGAACGATGGTCCTTCTAGAATTC
```

EP 0 434 909 A2

# FIG. 22

# FIG. 23

L3/L4     Adult

— 1.25 kb

# FIG. 24

FIG. 25

# FIG. 26

FIG. 27

## FIG. 28

```
                    10            20            30            40            50            60
                    *             *             *             *             *             *
  G AAT TCC  GGA AAG GAT AAT  GGT ACA GAC TAC  TGG CTC ATC GCC  AAT TCT TGG CAC  GAC GAT
              G   K   D   N    G   T   D   Y    W   L   I   A    N   S   W   H    D   D

                    70            80            90           100           110           120
                    *             *             *             *             *             *
  TGG GGT GAG AAT  GGA TTC TTC CGT  ATG ATT CGA GGC  ACC AAC GAC TGT  GGC ATA GAA GGG
   W   G   E   N    G   F   F   R    M   I   R   G    T   N   D   C    G   I   E   G

                   130           140           150           160           170           180
                    *             *             *             *             *             *
  CAA GTA GAC GCC  GGA ATC GTC GAC  GTT GAG AGT CTC  TAA GAC AGT ATA  AGG TGG AAT CAA
   Q   V   D   A    G   I   V   D    V   E   S   L    -

                   190           200           210           220           230
                    *             *             *             *             *
  ACG AAA TGA AAT  GGA AAC ATT AAT  CTC ACA AAA AAA  TGA AGC TTT GAA  TGA|7
```

79

# FIG. 29(1)

```
        10              20              30              40              50              60
        *               *               *               *               *               *
  C  TCA TGC TGG GCA GTC TCC ACT GCA GCT GCC ATT TCG GAT CGC ATT TGC ATA GCC ACC AAA
     S   C   W   A   V   S   T   A   A   A   I   S   D   R   I   C   I   A   T   K

        70              80              90             100             110             120
        *               *               *               *               *               *
  GGG AAA AAG CAG GTA TAT GCC TCT GAT ACT GAT ATT TTG ACA TGC TGT GGA GCA CGA TGT
   G   K   K   Q   V   Y   A   S   D   T   D   I   L   T   C   C   G   A   R   C

       130             140             150             160             170             180
        *               *               *               *               *               *
  GGG TTG GGG TGC AGA GGA GGT TGG CCC ATA GAA GCC TGG AAA TTC TTC GAA TAC GAT GGA
   G   L   G   C   R   G   G   W   P   I   E   A   W . K   F   F   E   Y   D   G

       190             200             210             220             230             240
        *               *               *               *               *               *
  GTC GTT TCT GGA GGA CCT TAC CTT GGC AAG GGG TGC TGC AGC CCT TAT CCA CTT CAT CCT
   V   V   S   G   G   P   Y   L   G   K   G   C   C   S   P   Y   P   L   H   P

       250             260             270             280             290             300
        *               *               *               *               *               *
  TGT GGA CGC CAT GGT AAC GAC ACC TTT TAT GGA AAC TGC GTT GGA ATG GCG CCC ACT CCG
   C   G   R   H   G   N   D   T   F   Y   G   N   C   V   G   M   A   P   T   P

       310             320             330             340             350             360
        *               *               *               *               *               *
  CCA TGC AAA AGG AAA TGC CAA CCT GGC TTC AGG GGA ATG TAC AGA GTT GAC AAG CGA TAT
   P   C   K   R   K   C   Q   P   G   F   R   G   M   Y   R   V   D   K   R   Y
```

EP 0 434 909 A2

# FIG. 29(2)

EP 0 434 909 A2

```
        370             380             390             400             410             420
         *               *               *               *               *               *
GGC GAG CCT GGC AGG ACT TAC ACA TTG CCG AGA TCT GAG GTA AAA ATT CGA AGA GAT ATC
 G   E   P   G   R   T   Y   T   L   P   R   S   E   V   K   I   R   R   D   I

        430             440             450             460             470             480
         *               *               *               *               *               *
AAG GAA AGA GGA TCG GTG GTT GCC GTA TTC GCT GTT TAT GAA GAC TTC TCT CAC TAC CAA
 K   E   R   G   S   V   V   A   V   F   A   V   Y   E   D   F   S   H   Y   Q

        490             500             510             520             530             540
         *               *               *               *               *               *
TCA GGA ATT TAT AAG CAC ACG GCT GGT AGG TTC ACA GGT GGT TAT CAT GCA GTG AAG ATG
 S   G   I   Y   K   H   T   A   G   R   F   T   G   G   Y   H   A   V   K   M

        550             560             570             580             590             600
         *               *               *               *               *               *
ATA GGA TGG GGA AAG GAC AAT GGC ACG GAC TAC TGG CTC ATC GCC AAT TCT TGG CAC GAC
 I   G   W   G   K   D   N   G   T   D   Y   W   L   I   A   N   S   W   H   D

        610             620             630             640             650             660
         *               *               *               *               *               *
GAT TGG GGT GAG AAT GGA TTC TTC CGT ATG ATT CGA GGC ATC AAC AAC TGT GGC ATA GAA
 D   W   G   E   N   G   F   F   R   M   I   R   G   I   N   N   C   G   I   E

        670             680             690             700             710             720
         *               *               *               *               *               *
GAG CAA GTA GAC GCC GGA ATC GTC GAC GTT GAG AGT CTC TAA GAC AGT ATA AGG TGG AAT
 E   Q   V   D   A   G   I   V   D   V   E   S   L   -

        730             740             750             760             770             780
         *               *               *               *               *               *
CAA ACG AAA TGA AAT GGA AGC ATT AAT CTT ACG AAA AAA TGA AGC TTC GAA TGA GTT CA10
```

# FIG. 30

FIG. 31

FIG. 32

FIG. 33

E  G  G1

68—

43—

25—

FIG. 34

FIG. 35

FIG. 36

FIG. 37

# FIG. 38(1)

```
        10              20              30    Exon 10   40              50              60
        *               *               *               *               *               *
G AAT TCG GTG AAA GCT ATC CAG AAA GAT ATT ATG AAA AAC GGA CCG GTT GTC GCC ACC TAT
  N   S   V   K   A   I   Q   K   D   I   M   K   N   G   P   V   V   A   T   Y

        70              80              90             100             110             120
        *               *               *               *               *               *
ACT GTT TAT GAA GAT TTC GCA CAC TAC AGA TCA GGA ATT TAT AAG gt gaa ttt ttc ata
  T   V   Y   E   D   F   A   H   Y   R   S   G   I   Y   K

       130             140             150             160             170             180
        *               *               *               *               *               *
ggg aat ctc ttg cta tac cac tta tgg atc tac tcc tac acc tca agc agt ttg cct tga

       190             200             210             220             230             240
        *               *               *               *               *               *
gga aac ctt ctg agc aaa ctt gtg atc tac tta tag CAC AAG GCT GGA CGC AAA ACA GGT
                                                  H   K   A   G   R   K   T   G

       250             260             270    Exon 11  280             290             300
        *               *               *               *               *               *
CTC CAT GCT GTG AAG GTG ATC GGA TGG GGA GAG GAA AAG GGA ACA CCC TAT TGG ATC GTT
  L   H   A   V   K   V   I   G   W   G   E   E   K   G   T   P   Y   W   I   V

       310             320             330             340             350             360
        *               *               *               *               *               *
GCC AAC TCG TGG CAC GAC GAC TGG GGA GAG AAT G gtgag tgt cgg aat agt aaa gaa aac
  A   N   S   W   H   D   D   W   G   E   N
```

EP 0 434 909 A2

## FIG. 38(2)

```
          370           380           390           400           410           420
           *             *             *             *             *             *
gaa tgt att tgt act taa aca tag atc tgt cag aaa tct gaa att cag gtt aat ccg ctt

          430           440           450           460           470           480
           *             *             *             *             *             *
tct tga att aac taa acg cta gaa aat ctt aat gga aat gag gta tca aat atc aac gtt

          490           500           510  Exon 12  520           530           540
           *             *             *             *             *             *
acg gat act ttt acag GC TTC TTC CGC ATG CAT CGT GGA AGC AAC GAC TGC GGA TTC GAA
                   G   F   F   R   M   H   R   G   S   N   D   C   G   F   E

          550           560           570           580           590           600
           *             *             *             *             *             *
GAG CGT ATG GCC gcn GGA TTA GTC CAG TGA TGA GAA TAA TGA ATA AAG CAT CTT AAA TAT
E   R   M   A   A   G   L   V   Q   -   -                 .

          610           620           630           640           650           660
           *             *             *             *             *             *
CAA AAC CAA TTG GAA AAG TTT GGA TCT CAA AGA ATG ATT TCA GGA TAA GGT CGG AGA TAG

          670           680           690           700           710           720
           *             *             *             *             *             *
AAA ATG GAA GCT GGC TAA GCG CGA GAC GGT CCC TCC AGT ATT GTG CGC CAG AGT CCA CGA
```

EP 0 434 909 A2

# FIG. 38(3)

```
         730            740            750            760            770            780
          *              *              *              *              *              *
TCC GTC GCG CGA ATC CGA ACC GTG CGA CGA ATC GCG GAC TCT GAC ACC AGC GTA AGC TGC

         790            800            810            820            830            840
          *              *              *              *              *              *
GGA ACG GCG CGC GTT TTG CAA CAA GAT TAT GCC CCC GGC CGC CCG GCT CTG CTA GAA GAC

         850            860            870            880            890            900
          *              *              *              *              *              *
GAC CGC ACC GAT GAG GGC ATT AAT GTG GAA ATT ACC CAT GAG CGT GGG GAT GCC TTA AAG

         910            920            930            940            950            960
          *              *              *              *              *              *
GCA CGC TCC ACG ATT AGG CGC GCT TTG CCT CTT CCA TCC GGG AGT GGT AGG GCA GGT GGA

         970            980            990           1000           1010
          *              *              *              *              *
CAA GTG TTG TTG TCG CAC CCT TTT TTG CCG CTT GAA AGT GCG GAC AAA GG
```

EP 0 434 909 A2

# FIG. 39(1)

```
        10              20      Exon 8  30              40              50
        *               *               *               *               *
AA GCT TGG CAG TAT TTC GCA CGA GAA GGT GTT GTG ACT GGT GGA AAT TAC CGC AAA CAG
   A   W   Q   Y   F   A   R   E   G   V   V   T   G   G   N   Y   R   K   Q

60              70              80              90              100             110             120
*               *               *               *               *               *               *
gtcg cta cca tcg gca ttt att gcc cat aag att cta atg aag gac tgt ttt cag GGT TGT
                                                                          G   C


            130             140     Exon 9  150             160             170             180
            *               *               *               *               *               *
TGT CGC CCC TAC GAG TTT CCA CCA TGT GGA AGG CAT GGA AAA GAG CCG TAC TAT GGT GAA
C   R   P   Y   E   F   P   P   C   G   R   H   G   K   E   P   Y   Y   G   E

            190             200             210             220             230             240
            *               *               *               *               *               *
TGC TAC GAT ACG GCG AAG ACT CCA AAG TGT CAA AAA ACA TGT CAG AGA GGG TAT TTG AAA
C   Y   D   T   A   K   T   P   K   C   Q   K   T   C   Q   R   G   Y   L   K

            250             260             270             280             290             300
            *               *               *               *               *               *
CCA TAC AAG GAG GAT AAG CAC TTC G gtaca ctt ttt tat aca taa cct ata act tac acg
P   Y   K   E   D   K   H   F

            310             320             330             800             810             820
            *               *               *               *               *               *
aag agt tca gct ggg tac cga cag agg ccg cgt nnn nnn ntt tta gca gag aga cag ggt
                                             (unsequenced region, ~460bp)
```

EP 0 434 909 A2

# FIG. 39(2)

```
          830          840          850          860          870          880
           *            *            *            *            *            *
ttt agc gtt gat gac gtc atc acc gat gat act cgc tta agt gga agt caa cag gga taa

          890          900          910          920          930          940
           *            *            *            *            *            *
caa tgg cga cgt ttt ctt cgg tat ttc ttc act gct gcc aat ctc agc acc tct gtt ttc

          950          960          970          980          990         1000
           *            *            *            *            *            *
att ctc aac gag gac cac ctg cga aaa acc cac gat acc tgt tct gca ctt ggt ttt cct

         1010         1020         1030         1040         1050         1060
           *            *            *            *            *            *
gct tta aag ctg ctg gca atc caa atc tga gaa act ttt tga gac ttc gca tta aat att

         1070         1080         1090         1100         1110         1120
           *            *   Exon 10   *            *            *            *
gcag GC AAG AGC GCC TAC CGA TTG CCG AAT AAT GTG AAA GCA ATC CAA AGA GAT ATC ATG
      G   K   S   A   Y   R   L   P   N   N   V   K   A   I   Q   R   D   I   M

         1130         1140         1150         1160         1170         1180
           *            *            *            *            *            *
AGG AAC GGA CCT GTG GTG GCC GGG TTC CTC GTC TAT GAA GAC TTC GCG CAC TAC AAA TCA
 R   N   G   P   V   V   A   G   F   L   V   Y   E   D   F   A   H   Y   K   S
```

EP 0 434 909 A2

# FIG. 39(3)

```
        1190            1200            1210            1220            1230            1240
          *               *               *               *               *               *
GGA ATA TAT AAG gta aag ctt tag ttc cct tgt aaa att gca gga tat ggt cct aaa tgt
 G   I   Y   K

        1250            1260            1270            1280            1290            1300
          *               *               *               *               *               *
caa att acc tca gca gta atc gta gtg cct gtt acc caa cat tgg atg cac tgt cca cag

        1310            1320            1330            1340            1350            1360
          *               *               *               *               *               *
ttg tgg ctc cat cag aca tta gga taa agt cac gta cgg act tga tta tgt aac gac ttt

        1370            1380            1390            1400            1410            1420
          *               *               *               *               *               *
gtg gtt gta aag ccg tgt caa gcg tcg tgt ccg cgt ctc cct tgt ccc cat act ctt tcg

        1430            1440            1450            1460            1470            1480
          *               *               *               *               *               *
gct gtc ccc tct cct cga cgc gca ttg tct cat tgc cat ggc act gcc cat ttc gta aca

        1490            1500            1510            1520            1530            1540
          *               *               *               *               *               *
gta gaa gtc ctc aat ttc tct gtt atc gca aat tca act cgt gga ccg tgc gca tag aaa
```

EP 0 434 909 A2

# FIG. 39(4)

```
        1550           1560           1570           1580           1590           1600
         *              *              *              *              *              *
ttg tcg cca ctt acc att caa tca tct ctg ctc gta ggg tta agt act gac taa gtt tta

        1610           1620           1630           1640           1650           1660
         *              *              *              *              *              *
aaa cat ttg tta cag CAT ACC GCT GGC AGA ATG ACA GGT GGT CAT GCT GTG AAG ATA ATC
                      H   T   A   G   R   M   T   G   G   H   A   V   K   I   I

        1670           1680           1690           1700           1710           1720
         *              *    Exon 11   *              *              *              *
GGA TGG GGA AAA GAG AAT GGA ACA CCC TAC TGG CTC ATT GCT AAC TCG TGG CAC GAC GAC
 G   W   G   K   E   N   G   T   P   Y   W   L   I   A   N   S   W   H   D   D

        1730           1740           1750           1760           1770           1780
         *              *              *              *              *              *
TGG GGA GAG AAA Ggt gag tct gct gca tgc tgt gaa ctt aaa ggc agg gat cac aat gat
 W   G   E   K   G

        1790           1800           1810           1830
         *              *              *              *
tgg cgt cgg ttt tga tga cgt cac tgg acg tca ttg gtg gaa agc tt
```

EP 0 434 909 A2

# FIG. 40(1)

EP 0 434 909 A2

```
                                    EXON 8                                      *50
                  AA GCT TGG CAA TAT TTC GCA CTA GAA GGT GTT GTG ACC GGT GGA AAT TAC CGC AAG CAG
                     Ala Trp Gln Tyr Phe Ala Leu Glu Gly Val Val Thr Gly Gly Asn Tyr Arg Lys Gln

                        INTRON 8                              *100
                  gtcactaccatcggcatttattgctcataagatcctgatgtagggctgtttttcag GGT TGT TGT CGC CCC TAC
                                                                        Gly Cys Cys Arg Pro Tyr

                              *150                EXON 9
                  GAG TTT CCA CCA TGT GGA AGG CAT GGA AAA GAG CCG TAC TAT GGT GAA TGC TAC GAT ACG
                  Glu Phe Pro Pro Cys Gly Arg His Gly Lys Glu Pro Tyr Tyr Gly Glu Cys Tyr Asp Thr

                        *200                                                      250*
                  GCG AAG ACT CCA AAG TGT CAA AAA ACA TGT CAG AGA GGG TAT CTG AAA GCA TAC AAG GAG
                  Ala Lys Thr Pro Lys Cys Gln Lys Thr Cys Gln Arg Gly Tyr Leu Lys Ala Tyr Lys Glu

                                                                  *300
                  GAT AAG CAT TTC G   gtacacttctttatacataacccataacttacacaaggcagttcaggtgcttcacggagc
                  Asp Lys His Phe G

                              *350        INTRON 9                              *400
                  catcaagcgcggtgcattttttcctatctagttcttttgtcatagaactgtacagtactgaatgattagcacccctctta

                                                  *450
                  ggaatgttttcaatccttcccacttaaatttcgaatatagtcagacaaaacttaaaggcaggtggcacgacaaatagga

                        *500                                          *550
                  cgctgaggaaggggaaaatgcgcccactataagtggagactctactcctcgaaaaacaatggtaaccggttcgcatcgc
```

# FIG. 40(2)

```
                              *600
aggaggtgcccacagcactatagaaaggctttactcaatcctttgtccttcttcccgcccactgactggtcactctagc

       *650                                                  *700
tgtcgtctctcgccgtctgactctagcccttttagcagaaagacagggtttttagcgttgagcgtttagcacctctgtttt

                         *750
cattctcaacgaggaccacctgcgaaaaaacccacgatacctgttctgcacttggttttcctgctttaaagcattgctg

*800                                         *850  GC AAG AGC GCC TAC CGA TTG
gcaatccaaatctgagaaacttttttgagcacttcgcattaaatattgcag    ly Lys Ser Ala Tyr Arg Leu


          EXON 10                    *900
CCG AAT AAT GTG AAA GCA ATC CAG AGA GAT ATC ATG AAG AAC GGA CCT GTA GTG GCT GGG
Pro Asn Asn Val Lys Ala Ile Gln Arg Asp Ile Met Lys Asn Gly Pro Val Val Ala Gly

                   *950
TTC ATC GTC TAT GAA GAC TTC GCG CAC TAC AAA TCA GGA ATA TAT AAG  gtaaagcttttagt
Phe Ile Val Tyr Glu Asp Phe Ala His Tyr Lys Ser Gly Ile Tyr Lys

         *1000                     INTRON 10              *1050
tcccttgtaaaattgcagaatatggtcctaaatgtcaaatcctctcagcagtaatcgtagtgcctgttacccgacattg
```

EP 0 434 909 A2

# FIG. 40(3)

```
                              *1100
gatgcactgtccgcagttgtggctccatcaatcagtgggattgagtcggacgtgggattgagtacggacttgattatgt

*1150                                            *1200
aatgaatgactttgtggttggaaagacgtgtcaagcgtcgtgtccgcgtctctcttgtccccatactctttcgactgtc

            *1250                                              *1300
ccgtctcctcgacgcgcattgtctcattgccgtggcactgcccatccccgtagaagtcctcagtttctctgctatcctg

                              *1350
acatcgactcttggaccgagcgcgtaaaaagccttaccactaaatcatctccgctcctaatgatatgtcctaataaatc

            *1400                    EXON 11                           1450*
ttgaagtacttgtttacag CAT ACC GCT GGT AGG ATG ACA GGT GGA CAT GCT GTG AAG ATA ATT
                     His Thr Ala Gly Arg Met Thr Gly Gly His Ala Val Lys Ile Ile

                                                              *1500
GGA TGG GGA AAA GAG AAA GGA ACA CCT TAT TGG CTC ATT GCT AAC TCG TGG CAT GAC GAC
Gly Trp Gly Lys Glu Lys Gly Thr Pro Tyr Trp Leu Ile Ala Asn Ser Trp His Asp Asp

                              *1550
TGG GGA GAG AAG G   gtgagtctgctgaatgctgtgaacctaaaggcagggatcataatgatcaaagggcgtcag
Trp Gly Glu Lys G
```

EP 0 434 909 A2

# FIG. 40(4)

```
                *1600                    INTRON 11                              *1650
tttcgatgacgtcactggacgtcattggtggaaggctttcgcaaccacttccttcgctcacgcactgcggggctcagtc

                            *1700
caccaatgacgttcaacgacttcatctaaaccgttccgccctacagacatcgtgatcattaccctaaagtgaacgcgta

        *1750                                              *1800
attatagtggattctggatgaacacaaatatttgaacgatataaaatctggatttgaagttccacatagcatcgaacta

            EXON 12                    *1850
ttttatag GC TTC TAC CGT ATG ATC CGT GGA ATC AAC AAC TGC AGA ATA GAA GAG ATG GTG
         ly Phe Tyr Arg Met Ile Arg Gly Ile Asn Asn Cys Arg Ile Glu Glu Met Val

                        *1900                                        1950*
TTC GCC GGA ATT GTT TAG TGA AGACACGAAATTATGGAAAAAAGAATCAACGAAATGAAGATGATTGCAATA
Phe Ala Gly Ile Val --- ---

                                            *2000
TGAGAATGATGAACACAGTGAAGTTTGCAGCGATCAATAAGGCCGATGTTAGACTTTGCGAATCGAGCGATTGGCTAGC

            *2050
GTGCGCGGTGCCGTCGCGGCCACCACACTCTACCGCAAGCAACCGCTGGCGTTATCGAATTC
```

EP 0 434 909 A2

# FIG. 41(1)

```
                                          *50
gagctctgaaatcccatgctcccgaacttccttcgaactgtagaggggccttttttcaatgcatgactagaaattgatgt
```

```
           *100                                                    *150
ggtattggatcaatatcaaggcccacccactgagttaccaaagccgattccctcttgaattcagagggtcccaaccgca
```

```
                              *200
gcactttggtggtacaattcagaagggagggggagatcgaactcaggtgtgtggctcaactcatcttagtaatggggaa
```

```
          *250            INTRON 4                        *300
ataacgccttgaccctcaactcatcttagtaatggggaaataacgccttgacctttttag   C TAC GAT CCT CGA
                                                                  Tyr Asp Pro Arg
```

```
                  EXON 5                           *350
ATT CAA TGG GCC AAC TGC TCT TCG CTC TTT CAC ATC CCC GAC CAA GCG AAC TGC G   gtaa
Ile Gln Trp Ala Asn Cys Ser Ser Leu Phe His Ile Pro Asp Gln Ala Asn Cys G
```

```
      INTRON 5               *400
aacctttcaacagtcccggcctacaacatcctgaaatctaggttagcttcgtctag GC TCA TGT TGG GCA GTC
                                                            ly Ser Cys Trp Ala Val
```

```
      *450                      EXON 6
TCG TCG GCA GCT GCG ATG TCT GAC AGG ATT TGC ATC GCT AGC AAG GGT GCC AAA CAG
Ser Ser Ala Ala Ala Met Ser Asp Arg Ile Cys Ile Ala Ser Lys Gly Ala Lys Gln
```

```
*500            INTRON 6                      *550
gtacagaacaatctacacaatagaattctaaattcacaacctcaggagcacaattcag   GTG CTG ATC TCA GCT
                                                              Val Leu Ile Ser Ala
```

EP 0 434 909 A2

## FIG. 41(2)

```
                EXON 7                          *600
CAA GAC GTT GTG TCG TGC TGC ACA TGG TGT GGT GAT GG  gtacgcttttctcttgactatttgcatt
Gln Asp Val Val Ser Cys Cys Thr Trp Cys Gly Asp Gl

        *650                    INTRON 7                       *700
tctgaaacggttgcggatgatcattctcgcactgggcactgaggtcaccttcctttggaccttgaagaactggtgtatg

                             *750
taccaggcctattttaagctttgacctaagcctaagtctaagcctgaactgagcctaagcccaagcttcggcatgatc

   *800                    .                        *850
atgacctaatgtgtaagcgagtctaagacccaacctaatcgcccaagtctagacataagcctgaacttagggtctaagc

                  *900                                              *950
ttgagcctaagcccgagactgagcctcatagactcaaccctaatgtgtaagcataagcctgctcaagcacaagccgacg

                                          *1000
cccaagctcaattttagccttcgatgatgcgcagctccggtaacctaagaagcaaactgagctcctatgtttcgttcat

          *1050                                    *1100
 gtgtgtaacaactcgcattggtccgcttaaccgaaaccatcgaaacgattacccgacaagttttctacccaagccggat

                  *1150
gattttagtgagatggaatctaaattttggtaagcggcgaattcttgtctaccaaaaaaaacatccactaaaaagatcg

      *1200                                        *1250
acaaatcgtgtttcataaaatggcagaactttctgattcag    A TGT GAA GGT GGC TGG CCG ATC AGT
                                             y Cys Glu Gly Gly Trp Pro Ile Ser
```

# FIG. 4I(3)

```
                              EXON 8                                    *1300
GCT TTC CGA TTT CAC GCA GAT GAA GGC GTT GTG ACT GGT GGA GAC TAC AAC ACA AAG
Ala Phe Arg Phe His Ala Asp Glu Gly Val Val Thr Gly Gly Asp Tyr Asn Thr Lys


                              INTRON 8                *1350
gtgagatcacaacgagctcgagctccttctcgtgatccacgtagggtaagcatttag    GGT AGC TGC CGT CCT
                                                             Gly Ser Cys Arg Pro


                   *1400        .         EXON 9
TAC GAG ATT CAT CCA TGT GGA CAT CAT GGA AAT CAC ACA TAT TAT GGA GAA TGC GTT GGC
Tyr Glu Ile His Pro Cys Gly His His Gly Asn His Thr Tyr Tyr Gly Glu Cys Val Gly

            *1450
ATG GCG GAT ACT CCA CGG TGC AAA AGG AGA TGC CTG CTT GGC TAC CCG AAA TCC TAT CCA
Met Ala Asp Thr Pro Arg Cys Lys Arg Arg Cys Leu Leu Gly Tyr Pro Lys Ser Tyr Pro

*1500                                                    *1550
TCG GAT AGG TAT TAC   ggtacgtctaaaattcgaacttaaaggcaatttagccctgaaactgatcgtgcgaaag
Ser Asp Arg Tyr Tyr

                   *1600           INTRON 9                          1650*
atagccataacgctagatatcaatgtgaacgttcataagttctcgtgcacataataaaccgaaactcagtcaagagggt
```

# FIG. 41(4)

```
                                                 *1700
tgtatagcaccacagggctcagtgcgctttgttcttgtccctttttaaaactcctcgacacgaaactgacagatttctca

           *1750                                                              *1800
gacgctaacgcaagatacggggcactgtgggggaacacatttcgattcgctaacatttctctttgctgaacttgaactt

                                    *1850
aagcgtaacctgagtgggaggagatctcagggtgcgtgagcagctttcatttcacccatagttcctgtcctctcctatg

           *1900                                              *1950
ctcagtcttagctggatgcccgctcccgtatcttgagatagcgccatttcttacacaacctctcttactaggtaagcta

                                *2000
atccgatgaacaccgttctctgaagaggactttaaggaatctttcccgggaaaatcatgattctatctcatccataaat

     *2050                                              *2100
cacgggttcggaacccccacgataggaacgaaaagagcaaagaaattgcgtttgtttcgataaagaaattcgaaaaacc

                *2150                                                   *2200
gaaagggctggtacgcgttaaatttaccacatctttcacaaatgccggcgacgggaacgctcacacacagttcacgaag

                                *2250
agctgtcactagatgtcgctgctgactttaatggcccccacaaactgggcgacagacttttggctcgtccgggttggca

                *2300
cgcggcaaattgcgcggcgagcggcgcggcgatccccgagacgtcgcggaattc
```

EP 0 434 909 A2

# FIG. 41(5)

```
                        EXON 10                                       *50
G AAT TCG GTC AAA GCT ATC CAG AAA GAT ATC ATG AAA AAC GGA CCC GTT GTC GCC ACC TAC
  Asn Ser Val Lys Ala Ile Gln Lys Asp Ile Met Lys Asn Gly Pro Val Val Ala Thr Tyr

                                                  *100
ACT GTT TAT GAA GAT TTC GCA CAC TAC AGA TCA GGA ATT TAT AAG  gtgaatttttcatagggaat
Thr Val Tyr Glu Asp Phe Ala His Tyr Arg Ser Gly Ile Tyr Lys

                    *150                    INTRON 10                          *200
ctcttgctatacctcttatggatctactcctacacctcaagcagtttgccttgaggaaaccctctgagcaaacttgtgaac

                        EXON 11                           *250
catttgtttacag CAC AAG GCT GGA CGC AAA ACA GGT CTG CAT GCT GTG AAG GTG ATT GGA TGG
              His Lys Ala Gly Arg Lys Thr Gly Leu His Ala Val Lys Val Ile Gly Trp

                 *300
GGA GAG GAA AAG GGA ACA CCC TAT TGG ATC GTT GCC AAC TCG TGG CAC GAC GAC TGG GGA
Gly Glu Glu Lys Gly Thr Pro Tyr Trp Ile Val Ala Asn Ser Trp His Asp Asp Trp Gly
```

EP 0 434 909 A2

# FIG. 4I(6)

EP 0 434 909 A2

```
                *350                    INTRON 11                    *400
GAG AAT G   gtgagtgtcggaatagtaaagaaaacatatgtatttgtatgtaaacatagatctgtcagaaatctgaa
Glu Asn G

                                        *450
attctacgcttatctgctttcttgaattacctacgctagaaaatcttaatggaaatgaggtctcaaatatcaacgctac

          *500                          EXON 12
ggatacttttacag  GT TTC TTC CGC ATG CAT CGT GGA AGC AAC GAC TGC GGA TTT GAA GAG
                ly Phe Phe Arg Met His Arg Gly Ser Asn Asp Cys Gly Phe Glu Glu

     *550                                              *600
CGT ATG GCC GCC GGA TCA GTC CAG TGA TGA   GAGTAATGAATAAAGCATCTCAAATATCAAACTATTAGA
Arg Met Ala Ala Gly Ser Val Gln --- ---


AAGTTTGGAC
```

# FIG. 42(1)

|  | EXON 5 | EXON 6 |
|---|---|---|
| ost007 | YDPRIQWANCSSLFHIPDQANC | GSCWAVSSAAAMSDRICIASKGAKQ |
|  | ** * *** * * | * * ** |
| haemAC-1 | YDPRDVWKNCTT FYIRDQANC | GSCWAVSTAAAISDRICIASKAEKQ |

|  | EXON 7 | EXON 8 |
|---|---|---|
| ost003 |  | AWQYFAR EGVVTGGNYRKQ |
|  |  | * |
| ost004 |  | AWQYFAL EGVVTGGNYRKQ |
|  |  | **** ** * * *** |
| ost007 | VLISAQDVVSCCT WCGDG | CEGGWPISAFRFHADDEFVVTGGDYNTK |
|  | * * *** *** | * ****** ** * * * |
| haemAC-1 | VNISATDIMTCCRPQCGDG | CEGGWPIEAWKYFIYD GVVSGGEYLTK |

EP 0 434 909 A2

# FIG. 42(2)

### EXON 9

ost003      GCCRPYEFPPCGRHGKEPYYGECYDTAKTPKCQKTCQRGYLKPYKEDKHF

ost004      GCCRPYEFPPCGRHGKEPYYGECYDTAKTPKCQKTCQRGYLKAYKEDKHF
             *     **   *   ***      *** *     *** **   *  *  ** ***

ost007      GSCRPYEIHPCGHHGNHTYYGECVGMADTPRCKRRCLLGYPKSYPSDRYY
             **     *       *      * * *    *    * ** ** * ** **

haemAC-1   DVCRPYPIHPCGHHGNDTYYGECRGTAPTPPCKRKCRPGVRKMYRIDKRY

### EXON 10

ost003      GKSAYRLPNNVKAIQRDIMRNGPVVAGFLVYEDFAHYKSGIYK
                      *          *

ost004      GKSAYRLPNNVKAIQRDIMKNGPVVAGFIVYEDFAHYKSGIYK
                *      *        ***       *

ost007          NSVKAIQKDIMKNGPVVATYTVYEDFAHYRSGIYK

ost002          NSVKAIQKDIMKNGPVVATYTVYEDFAHYRSGIYK
            *   ****     ** **     ***     *   *

haemAC-1   GKDAYIVKQSVKAIQSEILRNGPVVASFAVYEDFRHYKSGIYK

EP 0 434 909 A2

# FIG. 42(3)

## EXON 11

ost003    HTAGRMTGGHAVKIIGWGKENGTPYWLIANSWHDDWGEK
                                    *

ost004    HTAGRMTGGHAVKIIGWGKEKGTPYWLIANSWHDDWGEK
          *    *   *      *      *       **           *

ost007    HKAGRKTGLHAVKVIGWGEEKGTPYWIVANSWHDDWGEN

ost002    HKAGRKTGLHAVKVIGWGEEKGTPYWIVANSWHDDWGEN
          *  *** *      *        * ** ** **       *         *

haemAC-1  HTAGELRGYHAVKMIGWGNENNTDFWLIANSWHNDWGEK


## EXON 12

ost004    GFYRMIRGINNCRIEEMVFAGIV.
           *   *   * * **   ***   * *

ost007    GFFRMHRGSNDCGFEERMAAGSVQ.
                                     *

ost002    GFFRMHRGSNDCGFEERMAAGLVQ.
           *   **   *      * ***    * *****

haemAC-1  GYFRIIRGTNDCGIEGTIAAGIVDTESL.

(* = MISMATCH)

EP 0 434 909 A2

# FIG. 43

**Phylum** *Nematoda*

**Class** *Phasmidea*

    **Order** *Rabditata*

        *Caenorhabditis elegans*
        *Panagrellus redivivus*
        *Heterorhabditis bacteriophora*
        *Neoaplectana carpocapsae*

    **Order** *Strongylata*

        *Haemonchus contortus*
        *Ostertagia ostertagi*

    **Order** *Ascaridata*

        *Toxocara canis*

    **Order** *Filariata*

        *Dirofilaria immitis*

**Class** *Aphasmidea*

    **Order** *Trichurata*

        *Trichinella spiralis*

# FIG. 44

# FIG. 45

3A3

A) $NH_2$ —————————————— COOH

Peptide

20 aa

B) PKYCAIDGGIFFEDGTRR*

Peptide

EP 0 434 909 A2

# FIG. 46

## A. Adult Worm Proteins

− 55 kDa

## B. Anticoagulant Proteins

− 55 kDa

λ = λgt11

1-17 = phages 84-1 to 84-17

RS = rabbit serum

# FIG. 47

| 84-1 | 84-2 | 84-3 | 84-4 | 84-8 | |
|---|---|---|---|---|---|
| 3 4 A | 3 4 A | 3 4 A | 3 4 A | 3 4 A | MW |

- 200
- 97
- 68
- 43

kDa

- 29

18

- 14

3 = XL3
4 = L4
A = adult

EP 0 434 909 A2

# FIG. 48

EP 0 434 909 A2

84-2

(E)                    E              X        (E)

84-1

(E)   Bs    Xb Hp                 E Hp Bs   (E)

E = EcoR1
X = Xhol
Xb = Xbol
Hp = Hpol
Bs = BstUI

1kb

# FIG. 49(1)

```
                                                                          50*
          Glu Phe Arg Ile Tyr Phe Ser Val Leu Val Glu Trp Gln Gln Leu Val Trp
haem84-1  gaa ttc cGG ATA TAC TTC AGC GTG TTA GTT GAG TGG CAA CAG CTT GTG TGG

                                                                    *100
Thr Ala Phe *** Lys Gly *** Thr Cys Phe Ser Leu Arg Ser Val Thr Asp Glu Gly Ser
ACT GCC TTT TGA AAA GGC TGA ACC TGC TTT TCA CTC CGT TCA GTG ACG GAC GAG GGA AGC

                                              *150
Gly Val Arg Ala Ser Arg Ser Leu Asn Gly Val Lys Ser Arg Phe Ser Leu Phe Lys Arg
GGC GTA CGG GCA TCG CGG TCA CTG AAC GGA GTG AAA AGC AGG TTC AGC CTT TTC AAA AGG

                                    *200
Gln Ser Thr Gln Ala Val Ala Thr Gln Leu Thr Pro Arg Glu Met Asp Ile Leu Gln Arg
CAG TCC ACA CAA GCT GTT GCC ACT CAA CTA ACA CCC AGA GAA ATG GAT ATA CTT CAG CGT

                            *250
Val Arg Leu Gly Arg Pro Pro His Gly Phe Val Pro Asn Leu Asp Thr Val Asp Thr Pro
GTG CGC CTG GGT AGG CCC CCA CAC GGA TTC GTG CCT AAT CTA GAC ACG GTC GAT ACG CCA

                                                                    350*
        *300
Ser Glu Tyr Glu Thr Gly Tyr Phe Thr Gln Pro Val Asp
TCA GAA TAT GAA ACT GGA TAC TTT ACA CAG CCT GTG GAC C-- --- --- --- --- --- ---
                                      haem84-2      gaa ttc cAT TTC AAC AAC CAG AAC CCG
                                                    Glu Phe His Phe Asn Asn Gln Asn Pro
```

EP 0 434 909 A2

# FIG. 49(2)

```
                                                            Asp                                        *400
--- --- --- --- --- --- --- --- --- --- --T --- --- --- --- --- --- --- --- ---
GCC ACA TTT GAT CAG AAA TAC TAC AAA AAC GAA CAA TGG GCA AGA GAA GGC GGT CCA ATA
Ala Thr Phe Asp Gln Lys Tyr Tyr Lys Asn Glu Gln Trp Ala Arg Glu Gly Gly Pro Ile
                                    *50

              Asp              Trp                          *450
--- --- --- --- -AT --- --- T-G --- --- --- --T --- --- --- --- --- --- --- ---
TTC CTT ATG ATC GGC GGC GAA GGA CCA AGT AGC GCG AAA TGG ATT CTC AAC GAA AAT TAT
Phe Leu Met Ile Gly Gly Glu Gly Pro Ser Ser Ala Lys Trp Ile Leu Asn Glu Asn Tyr
                  *100

                                       *500
--- --- --- --- --- --- --- --- --- --- --- --- --- --- --- --- --- --- --- ---
ACT TGG TTG CAA TGG GCA AAA AAA TTC GGA GCA ACA ACA TAC ATG CTA GAA CAC AGA TAC
Thr Trp Leu Gln Trp Ala Lys Lys Phe Gly Ala Thr Thr Tyr Met Leu Glu His Arg Tyr
*150                                                                 *200

                        *550                                     Met Thr
--- --- --- --- --- --- --- --- --- --- --- --- --- --- --- -T- -CC --T --- ---
TAT GGC GAC AGC GAT CTT CAG AGG TTG CTT TTC GAC TCT ACT GAT ACG AAG CTC AAG AGG
Tyr Gly Asp Ser Asp Leu Gln Arg Leu Leu Phe Asp Ser Thr Asp Thr Lys Leu Lys Arg
                                                            *250

        600* Ala                                      650*
--- --- --- G-- --- --A --- --- --G --- --- --- --- --- --- --- --- --- --- ---
ACA TAC ACG ACC TAT CTC TCC TCG CTT CAA ATG CTA TAC GAT ACG GCC AAT TTC ATT CAA
Thr Tyr Thr Thr Tyr Leu Ser Ser Leu Gln Met Leu Tyr Asp Thr Ala Asn Phe Ile Gln
                                  *300
```

EP 0 434 909 A2

# FIG. 49(3)

```
                                                                    *700
--- --- --- --- --- --- --- --- --- --- --- --- --- --T --- --T --- --- --- ---
GCC ATT GAT GCT GAT AAT GGC AAA AAA GGC ACA TGG ATC GTA TTT GGA GGA TCT TAT GCT
Ala Ile Asp Ala Asp Asn Gly Lys Lys Gly Thr Trp Ile Val Phe Gly Gly Ser Tyr Ala
                            *350


                                           *750
--- --- --- --- --- --- --- --- --- --- --- --- --- --- --- --- --- --- --- --C
GGA TCA TTA GCC CTG TGG ATG CGT AAG CTC TTC CCT AAT CTC GTC CAT GGA GCT GTT GGT
Gly Ser Leu Ala Leu Trp Met Arg Lys Leu Phe Pro Asn Leu Val His Gly Ala Val Gly
            *400


                              *800
--- --- --- --- --- --- --- --- --- --- --- --- --- --- --- --G --- --- --- ---
TCA TCG GCA CCT CTT GAA GCA AAG CTG GAC TAC CAT GAA TAT TAC CAA GTG GTC GAA GCA
Ser Ser Ala Pro Leu Glu Ala Lys Leu Asp Tyr His Glu Tyr Tyr Gln Val Val Glu Ala
  *450                                                                *500


            Gly           *850
--- --- --- -G- --- --- --A --- --- --- --- --T --- --- --- --- --- --- --- ---
TCA ATC CGG GAA TAC AGC GAG GAT TGT GCG TAT GCC ATA GGA GAG GGC TTC GAG GAT ATT
Ser Ile Arg Glu Tyr Ser Glu Asp Cys Ala Tyr Ala Ile Gly Glu Gly Phe Glu Asp Ile
                                                        *550


        *900                                                    950*
--- --- --- --- --- --- --- --- --- --- --- --- --- --- --- --- --- --- --- ---
CAT GAG AAA ATG CTC TCG GAA CGT GGA AGG GAA GAA ATA TCA AAA ACA TTC AAG CTC AAC
His Glu Lys Met Leu Ser Glu Arg Gly Arg Glu Glu Ile Ser Lys Thr Phe Lys Leu Asn
                                        *600
```

EP 0 434 909 A2

# FIG. 49(4)

EP 0 434 909 A2

```
                                                               *1000
--- --- --- --- --- --- --- --- --- --- --- --- --- --- --- --- --- --- --- ---
CCT CCA TGG GAT GAT GTC TCC GAT GTT TTC GAA ATA GAC AAG CAG TTC TTC TTC TGG AAC
Pro Pro Trp Asp Asp Val Ser Asp Val Phe Glu Ile Asp Lys Gln Phe Phe Phe Trp Asn
                             *650

                                                  *1050
--- --- --A --- --- --- --- --- --- --- --- --- --- --- --- --- --- --- --T ---
CTT ATG GAG CAG TTT ACG GCC GCC GTC CAG TAT AGT GGA GAT AAC TCA GGG GGA TAC GCT
Leu Met Glu Gln Phe Thr Ala Ala Val Gln Tyr Ser Gly Asp Asn Ser Gly Gly Tyr Ala
            *700

                                      *1100
--- --C --- --- --- --G --- --- --- --- --- --- --- --- --- --- --T --- --- ---
GAT GGA CAC GGA ATT CCT GAT TTG TGC AAA ATC ATG ACC AAC GAA AGA CGG ACG CCC ATG
Asp Gly His Gly Ile Pro Asp Leu Cys Lys Ile Met Thr Asn Glu Arg Arg Thr Pro Met
    *750                                                        *800

                            *1150
--- --- --- --- --- --T --- --- --- --- --- --- --- --- --- --- --- --- --C ---
GCA AGA ATC GCC GAG TTC AAC GAA TAC ATG ACG AGA TTC TTT ACG GGC AAA CCT GCT TTC
Ala Arg Ile Ala Glu Phe Asn Glu Tyr Met Thr Arg Phe Phe Thr Gly Lys Pro Ala Phe
                                                        *850

         *1200                                                      1250*
--- --- --- --- --- --- --- --- --- --- --- --- --- --- --- --- --- --- --- --A
GAA TAC ACA TTC AAT AGT TAC AAA GAG TTC GTC AGC ACC GCG TAC AAG GCT CAA TTC GCG
Glu Tyr Thr Phe Asn Ser Tyr Lys Glu Phe Val Ser Thr Ala Tyr Lys Ala Gln Phe Ala
                             *900
```

# FIG. 49(5)

```
                                                                      *1300
--C --- --- --- --G --- --- --- --- --- --- --- --- --- --- --- --C --A --T ---
ACT GAC AAA AAG GCA GCA GCA GGA ACG CTT TGG CTA TGG CAA ACT TGT ACT GAG TTC GGC
Thr Asp Lys Lys Ala Ala Ala Gly Thr Leu Trp Leu Trp Gln Thr Cys Thr Glu Phe Gly
                            *950

                                             *1350
--- --C --- --- --C --- --- --- --- --- --- --- --- --- --- --- --- --- --- ---
TTC TAT GGA ACA ACT GAC TCC GGA TAC AGT CTT TTC GGC AAC CCA TTA CCA TTG AAC TTC
Phe Tyr Gly Thr Thr Asp Ser Gly Tyr Ser Leu Phe Gly Asn Pro Leu Pro Leu Asn Phe
                *1000

                                    *1400
--- --- --- --- --- --- --- --G --- --- --- --- --- --- --- --- --- --- --- --C
TTC ACA CAA CTA TGC TCG GAT TTA TTC GGC TGG AAG ATA GAT TAC TCA GCG GAA ATG AAT
Phe Thr Gln Leu Cys Ser Asp Leu Phe Gly Trp Lys Ile Asp Tyr Ser Ala Glu Met Asn
   *1050                                                         *1100

                        *1450
--- C-- --- --- --- --- --- --- --- --- --- --- --- --- --- --- --- --- --- ---
CGA AGA GCT ACG CTT AAT GTG AAT AAT CGC TAC GGT GGT CGA TAC AAG TAC GAG AAA ACA
Arg Arg Ala Thr Leu Asn Val Asn Asn Arg Tyr Gly Gly Arg Tyr Lys Tyr Glu Lys Thr
                                                            *1150

           *1500                                                 1550*
--- --- --- --- --T --- --- --- --C --- --- --- --- --- --- --- --- --- --- -gg
AAT GTC GTA ATG ACC TAT GGC ACT CTT GAC CCA TGG ACA GCT CTA GGC CCC GTT GAA TGC
Asn Val Val Met Thr Tyr Gly Thr Leu Asp Pro Trp Thr Ala Leu Gly Pro Val Glu Cys
                                        *1200
```

EP 0 434 909 A2

# FIG. 49(6)

```
aat tcc (end of 84-1)
AAG GAA TCT GAA AAC TGC TTG ATG ATC AAA GGA ACT GCA CAT TGT GCT GAA ATG TAT CCT
Lys Glu Ser Glu Asn Cys Leu Met Ile Lys Gly Thr Ala His Cys Ala Glu Met Tyr Pro
                                  *1250


GCT CGA GAG GCA GAC TTA CCT TCG CTT AAG GAA GCC CGT AGT AAG ATC GAG AAC ATC ATC
Ala Arg Glu Ala Asp Leu Pro Ser Leu Lys Glu Ala Arg Ser Lys Ile Glu Asn Ile Ile
              *1300


GAG GGA TGG GTA CAG GCG AAG AAA GTC AGC CAG GAT CAG CGA CCA GTT GAG AAG AAC TCT
Glu Gly Trp Val Gln Ala Lys Lys Val Ser Gln Asp Gln Arg Pro Val Glu Lys Asn Ser
   *1350                                                            *1400


AAA AAG AGT TTT TTC TCC TTT TTG CAT AGA CAG TGA CGTTAAGAAGAACTCCGCTGGGTCTGTGGCA
Lys Lys Ser Phe Phe Ser Phe Leu His Arg Gln                  *1450


ATCACTTCCTCGATCACACCAATGGTATCCTTCCAAAAAGCACTTTTACTTTAGAAAGAATTGTTCACCTTCGTTTCGA
                        *1500                                              1550*


ACTGTTGTTTTGAGTTAGATGAAGATTGCCCAAACCATAACTCTGTCAAAGTGAAATTGAATGGACTGGATACTAAAAA
                                      *1600


TAAAGTGTATCGAGAAAAAAAAAAAAAAAAAAAAGGAATTC
              *1650
```

EP 0 434 909 A2

# FIG. 50

FIG. 51

# FIG. 52

H = Hind III
E = Eco RI
S = Sal 1
B = BamHI

▨ = F-I HYBRIDIZING REGION

▧ = EXON I-4 HYBRIDIZING REGION

I-kb

EP 0 434 909 A2

# FIG. 53

FIG. 54(I)

GAA TTC nnn TTC CCA ACC ATC TAn nnn nnn TCT ACC TAT TGG GAT GAT CAC CAA ACA CAT

CTC TAT ACC TTG GTT GTC AAA CCA GAT GGA ACA TTC TCG GTT TCA GTC GAT CAA AAA GAA

ATC ATG TCA GGG AAC ATG CTA AAC GAC CTC CAA CCA TCG TTG CAA CCT CCA AAG CAA ATC

GCT GAC CCT ACC GAT AAA AAA CCA GCT GAT TGG GAT GAC AGG GCG GAA ATC GAA GAT GAA

AGC GCT GTC AAG CCA GAT GAT TGG GAT GAG AGC CAG CCT AGC GAA GTC GTT GAT GAA ACG

CGT AAG CCT TCC GAT TGG CTC GAG AAT GAA CCG AGC TGA ATC GTA CCG AAG CAT CGA ACA

GCG ACT GGA TG................approximately 220 bp................

EP 0 434 909 A2

# FIG. 54(2)

```
591   ATA GAA GAG GCT GAG CGT GTT TTC CGA AGG AAG CCC AGT CCA CAT GGA CAC CCA AGG CAG

651   AAA AAG AAG CTG AAA AAA GTT GTA GAG TTG GAG GAT CTT GAG GGC AGT AAC AGC GAA CCA

711   GAT ATT ATA AAC GTT GAA GCA GAC GAA GCT GGA CCA TCG GGT GCT AAG AAA TCG CCA ACT

771   GTC ACG AAG AGG nGT GTT CGA AGG CAA GAC TAA GTT CTA CGG GAT GAG CTC TGG AAn nGT

831   TAG GTT ACC AAC GTC ATT ATT CCG TTA CGT TCC GCT TTC AAG TGA AAT CTT GCT GAT TTT

891   GTC GAT CGT TGC CTT TTC ACC GGC CAT TTC CCG AAG GTT ACT TTG GAC ATC CAA CTG TCT

951   ATT CCT TAC CCT AGC CGC CGT TTT GCG TGC ATG TTA TCA CAT GCT TTG GCC ATG TTC ACG

1011  CAT TCT CTT TTT TTG CTC GCT ATT GTG TTT TTT TGT GTT GTC ATT GGA TCC CAA GTA GTG

1071  TTC TCT CAT TTT GAG ACC GGT TGC GTT GTA GGA GTT GTG GCG TAC TCT GTT TTT GTT TCT

1131  TAT TAG TTG TTC TTT TAC TCG TAA TAA ATC GTT GCC GTT GTA AAA AAA AAA AAA AAA AAA

1191  AGG AAT TC
```

EP 0 434 909 A2

# FIG. 55(1)

```
  1  GAA TTC CGG CAA ATA CTA TGC ATT GAT GCT AAG AGA GAC CAA GGG TTG ACA AAC ATA GTC

 61  AAG ATG CAA AAT TCT ACT CAC TAG CAG CCA AAT TCC CCA AGA AGT TCA CCA ACA AGG GAA

121  AAA CCG TCG TAA TCC AGT ACA CGG TAA AGC ACG AGC AGG GAA TTG ATT GTG GAG GTG GCT

181  ACG TTA AGG TCA TGA GTT CGG ATG TGG ATC TTA AAG ACT TCC ATG GTG AGA CTC CAT ACA

241  ATG TAA TGT TCG GCC CTG ACA TTT GCG GAC CGA CAA AGA AGT TCA CGT TAT TTT CTC GTA

301  CAG GCA AGA ACC ATC TCA TCA AGA AGG ATA TCG TGC AAG ATG ATG AGT TGA CTA CTT GTA

361  CCC ......... approximately 320 bp ..........
```

EP 0 434 909 A2

# FIG. 55(2)

```
683   TGT CAA CTG GGA CGA TCG TTT ACT ATG CAG TCA GTC TGC ACT ATC TCA CAC ATC TGT GAC

743   GAT TCC GTT GAT GAT CTA AAG CTC ATG CTG CTG AGA CAT TCG AAA AGT TGA AGG CnG TCG

803   AGA AGG AGA AGA AGG ATA AGG CTG ATG AAG AAG AGC GAA AGA AGA TAG AGG AGG AAG CCA

863   AGA AGA GGG AAG AGG AGG ACA AGA AGA AAA AAG AGG CGA AGG AGA AGG AAG AGA AGG AAG

923   ACG AGG ATG AGG ACA AGG AAG AGG AAG CTC ATG ACG AAC TCT AAA TTT GCC AAG CTC TGT

983   TGT TTC GGG TCG GTG ACT ACA AGT GCG TTT ATG TAA CTG CTA GTC TCG TTT CTA GCC CCT

1043  TTT TGT TCT TGT TGT TTT TAT TAT TAT TTT GCG TCA CCA ATA CAT GAG GTG ACC CTC TGT

1103  TTT TTC TAG AGG AAT TGT CCT TCC TGC CTC GTA TTG CTT TTT CAG TTG GTT GCG TTA ATA

1163  AAT TAT TTA TTG ATA AAA AAA AAA AAA AAA AAA AGG AAT TCC AC
```

EP 0 434 909 A2

# FIG. 56